# EUROPEAN PATENT APPLICATION

(11) **EP 4 837 907 A1**
(43) Date of publication of application: **11.11.2026**
(21) Application number: 25742315.2
(22) Date of filing: 16.01.2025
(51) Int. Cl.: C07D 403/14, A61K 31/4184, A61P 35/00, A61P 29/00, A61P 37/00, C07D 235/26, C07D 405/14, C07D 401/14, C07D 403/04, C07D 409/14

(54) **NOVEL COMPOUND USED AS CBP/P300 INHIBITOR, AND PHARMACEUTICAL COMPOSITION FOR PREVENTING OR TREATING CANCER, INFLAMMATORY DISORDERS, OR AUTOIMMUNE DISEASES COMPRISING SAME AS ACTIVE INGREDIENT**

(30) Priority: 17.01.2024 KR 20240007542
(71) Applicant: LG Chem, Ltd., Seoul 07336 (KR)
(72) Inventor: KIM, Rira, Daejeon 34122 (KR); PAK, Gyungah, Daejeon 34122 (KR); LEE, Soyun, Daejeon 34122 (KR); CHO, Joongheui, Daejeon 34122 (KR); HAM, Youngjin, Daejeon 34122 (KR); HWANG, Youson, Daejeon 34122 (KR); YOON, Hong Bin, Daejeon 34122 (KR); KIM, Byung Gyu, Daejeon 34122 (KR); KIM, Hak Joong, Daejeon 34122 (KR)
(74) Representative: Hoffmann Eitle
(86) International application number: PCT/KR2025/099056
(87) International publication number: WO 2025/155167

(57) **Abstract**

The present invention provides a novel compound used as a CBP/p300 inhibitor, an isomer thereof, a pharmaceutically acceptable salt thereof, a hydrate thereof, and a solvate thereof, and provides a pharmaceutical composition for preventing or treating cancer, an inflammatory disorder, or an autoimmune disease, the pharmaceutical composition comprising at least one of the compound, isomer, salt, hydrate or solvate as an active ingredient.

## Description

### Technical Field

This application claims the benefit of priority to Korean Patent Application No. 2024-0007542, filed on January 17, 2024, the entire disclosure of which is incorporated herein by reference. This disclosure relates to a novel compound used as a CBP/p300 inhibitor, and a pharmaceutical composition for preventing or treating cancer, inflammatory disorders, or autoimmune diseases containing the same as an active ingredient.

### Background Art

CBP [CREB (cyclic-AMP response element binding protein) binding protein] and the paralog thereof, p300, are transcription coactivators composed of multiple functional domains including a histone acetyl-transferase (HATs) domain and a bromodomain (BRD). CBP and p300 are major epigenetic modulators that recognize and induce histone lysine acetylation at specific loci such as enhancers and promoters on chromatin, thereby regulating the expression of various genes.

In consideration of high structural similarity and functional redundancy, overexpression or mutations of CBP and p300, often referred to as CBP/p300, have been associated with various diseases, especially malignant tumors. For example, CBP and p300 bind to chromatin through BRD and HAT and acetylate histones at enhancer sites of oncogenes, thereby relaxing a chromatin structure and allowing transcriptional functional proteins including RNA polymerase II to assemble. As a result, tumor cells increase the expression of oncogenes such as MYC and IRF4 in tumor cells, thereby forming and growing tumors. Inhibition of the HAT domain causes an overall decrease in acetylation, which is important for transcription, and acts in the same way not only on tumor cells but also on normal cells, especially stem cells. Accordingly, it has been reported that it is difficult to secure a therapeutic window, and toxicity may be induced, so it is expected that the concerns may be overcome through a bromodomain inhibitor strategy.

Accordingly, various studies have been conducted to develop inhibitors that selectively inhibit the bromodomain of CBP/p300 and reduce the transcription of oncogenes activated in tumor cells. For example, CCS1477, a CBP/p300 bromodomain inhibitor developed by CellCentric, is in phase 1b/2a clinical trials for the treatment of hematological malignancies and advanced drug-resistant prostate cancer, and various CBP/p300 activity modulators are also disclosed in Patent Documents 1 to 3 below.

However, the development of effective and selective therapeutic agents targeting CBP/p300 is still a difficult task. Therefore, the development of more chemotypes of CBP/p300 inhibitors is needed.
[Patent Document 1] WO2018073587
[Patent Document 2] WO2019191667
[Patent Document 3] WO2017205538

### Disclosure of Invention

### Technical Goals

The present disclosure has a purpose of providing a novel compound used as a CBP/p300 inhibitor.

The present disclosure also has another purpose of providing an isomer of the compound, a pharmaceutically acceptable salt thereof, a hydrate thereof, or a solvate thereof.

The present disclosure also has another purpose of providing a pharmaceutical composition for preventing or treating cancer, inflammatory disorders, or autoimmune diseases, which includes at least one of the compound, the isomer thereof, the pharmaceutically acceptable salt thereof, the hydrate thereof, and the solvate thereof, as an active ingredient.

### Technical solutions

To solve the task, according to an aspect of the present disclosure, there is provided a compound represented by the following Formula I: 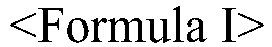 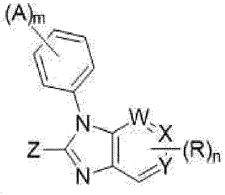 where
W, X and Y are each independently C or N,
n is an integer from 0 to 3, where if n is an integer of 2 or more, Rs are the same or different, and Rs are each independently NR^{a}R^{b}, SO₂NHR^{c}, CONHR^{d}, SO₂R^{e}, CO₂R^{f}, halogen, C₁₋₄ alkyl, C₁₋₄ haloalkyl, or 3- to 9-membered heteroaryl, and R^{a} to R^{f} are each independently selected from the group consisting of hydrogen, C₁₋₆ alkyl, hydroxy, C₁₋₅ alkoxy, and 3- to 10-membered heterocycloalkyl,
m is an integer from 0 to 3, where if m is an integer of 2 or more, As are the same or different, and As are each independently amide, hydroxy, C₁₋₅ alkoxy, 3- to 10-membered heterocycloalkyl, 3- to 9-membered heteroaryl or halogen, or two As are fused together to form with an atom bonded thereto, a 3- to 10-membered heterocycloalkyl, or 3- to 9-membered heteroaryl, and
Z is hydrogen, amide, hydroxy, C₁₋₅ alkoxy, C₁₋₄ alkyl, C₆₋₁₄ aryl, 3- to 10-membered heterocycloalkyl, or 3- to 14-membered heteroaryl,
where R, R^{a} to R^{f}, A and Z are each substituted or unsubstituted with Substituent Group I.

Another aspect of the present disclosure relates to an isomer of the compound above, a pharmaceutically acceptable salt thereof, a hydrate thereof or a solvate thereof.

Another aspect of the present disclosure relates to a pharmaceutical composition for preventing or treating cancer, inflammatory disorders or autoimmune diseases, the composition including at least one among the compound above, an isomer thereof, a pharmaceutically acceptable salt thereof, a hydrate thereof and a solvate thereof, as an active ingredient.

### Effects

According to the present disclosure, it has been found that the compound represented by Formula I has excellent CBP/p300 inhibitory activity. Therefore, the compound represented by Formula I of the present disclosure; an isomer of the compound, a pharmaceutically acceptable salt thereof, a hydrate thereof or a solvate thereof; and a pharmaceutical composition including at least one of them as an active ingredient may be useful for inhibition, such as the prevention or treatment of cancer, inflammatory disorders or autoimmune diseases.

The effects of the present disclosure are not limited to the above-described effects, and are understood to include all effects that may be inferred from the configuration of the invention described in the description or claims of the present disclosure.

### Best Mode for Carrying Out the Invention

Hereinafter, in order to assist the understanding of the present disclosure, the present disclosure will be described in more detail. In this case, the terms or words used in this specification and claims should not be interpreted as limited to usual or dictionary meanings, and should be interpreted as meanings and concepts that conform to the technical idea of the present disclosure based on the principle that the inventor may appropriately define the concept of the term to explain his own invention in the best way.

The present disclosure provides a compound represented by the following Formula I:

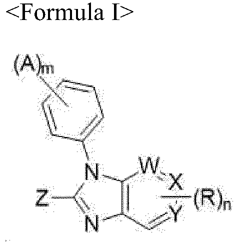

where
W, X and Y are each independently C or N,
n is an integer from 0 to 3, where if n is an integer of 2 or more, Rs are the same or different, and Rs are each independently NR^{a}R^{b}, SO₂NHR^{c}, CONHR^{d}, SO₂R^{e}, CO₂R^{f}, halogen, C₁₋₄ alkyl, C₁₋₄ haloalkyl, or 3- to 9-membered heteroaryl, and R^{a} to R^{f} are each independently selected from the group consisting of hydrogen, C₁₋₆ alkyl, hydroxy, C₁₋₅ alkoxy, and 3- to 10-membered heterocycloalkyl,
m is an integer from 0 to 3, where if m is an integer of 2 or more, As are the same or different, and As are each independently amide, hydroxy, C₁₋₅ alkoxy, 3- to 10-membered heterocycloalkyl, 3- to 9-membered heteroaryl, or halogen, or two As are fused together to form with an atom bonded thereto, a 3- to 10-membered heterocycloalkyl, or 3- to 9-membered heteroaryl, and
Z is hydrogen, amide, hydroxy, C₁₋₅ alkoxy, C₁₋₄ alkyl, C₆₋₁₄ aryl, 3- to 10-membered heterocycloalkyl, or 3- to 14-membered heteroaryl,
where R, R^{a} to R^{f}, A and Z are each substituted or unsubstituted with Substituent Group I:
   Substituent Group I: substituted or unsubstituted C₁₋₁₀ alkyl, substituted or unsubstituted C₂₋₁₀ alkenyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3- to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, aldehyde, carboxy, halogen, substituted or unsubstituted C₁₋₁₀ haloalkyl, hydroxy, substituted or unsubstituted amino, substituted or unsubstituted imine, cyano, substituted or unsubstituted nitro, substituted or unsubstituted amide, thiol, sulfone and phosphoric acid,
   where a C₁₋₁₀ alkyl moiety is interposed by a heteroatom group selected from - O-, -S-, -SO-, -SO₂-, -NR^{g}- (R^{g} is hydrogen or substituted or unsubstituted C₁₋₁₀ alkyl), -N=, =N-, -POR^{g}- and -PO₄R^{g}-, and a heterocycle moiety may include at least one of S, N, P and O as a ring-forming atom.

In the present specification, matters on R may be equally applied to R¹¹², R³¹³, R¹¹⁴, R³¹⁴, R²¹⁵, R³¹⁵, R¹¹⁶, R²¹⁶, R³¹⁶, R³²², R²²³, R³²³, R¹³², R³³³, R¹³⁴, R³³⁴, R¹⁴², R²⁴³, R³⁴⁴, R¹⁴⁵, R³⁴⁵, R¹⁴⁶, R²⁴⁶, R³⁴⁶, R³⁵², R¹⁵³ and R³⁵³.

In defining the compound represented by Formula I throughout this specification, the defined concepts as follows are used. The definitions apply to terms used individually or as part of a larger group throughout this specification, unless otherwise specified. Terms and abbreviations used herein have their original meanings unless otherwise defined.

In this specification, the term "substituted" may mean that a hydrogen atom bonded to the compound or each substituent listed in the substituent group is replaced and bonded with an arbitrary substituent. In this case, the position to be substituted is not limited as long as the position is where a hydrogen atom bonded to the compound or each substituent listed in the substituent group exists, i.e., the position where the hydrogen atom may be substituted with a substituent. If two or more are substituted, the two or more substituents may be the same or different from each other. In contrast, "unsubstituted" may mean that no hydrogen atom bonded to a carbon atom is replaced with an arbitrary substituent.

Arbitrary substituent applicable to the "substitution" may be appropriately selected from the substituents listed in the substituent group as described in this specification, but the scope of the substituents available for the substitution is not limited to the substituent group.

If a substituent is not separately described or mentioned in the chemical formulas described in this specification, it may be considered that hydrogen is bonded, and in some cases, the substituent may be absent depending on the ring-forming elements of a heterocycle.

In this specification, the term "halogen" includes, but is not limited to, for example, fluoro, chloro, bromo, and iodo.

In this specification, the term "alkyl" by itself or as a part of another substituent means a straight or branched monovalent hydrocarbon having an indicated number of carbon atoms (i.e., C₁₋₁₀ means 1 to 10 carbon atoms), unless otherwise referred to.

Examples of the alkyl include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 2-methylpentyl, 4-methylhexyl and 5-methylhexyl, but these are non-limiting examples and the scope of the present disclosure is not limited thereto.

In this specification, the term "aryl" refers to a multiply unsaturated, typically aromatic hydrocarbon ring, where the aryl may be monocycle or polycycle. The case of polycycle includes a form in which only aromatic hydrocarbon rings are fused and a form in which an aliphatic hydrocarbon ring is fused with an aromatic hydrocarbon. Examples of the aryl include monocyclic aryls such as phenyl, biphenyl, terphenyl and quaterphenyl, and polycyclic aryls such as naphthyl, anthracenyl, phenanthrenyl, pyrenyl and benzopyrenyl.

In this specification, the term "heterocycle" refers to a ring including, together with carbon atoms, 1 to 5 heteroatoms other than carbon atoms, i.e., O, N, P and S, as ring-forming atoms. Here, the heterocycle may be aromatic, aliphatic, monocyclic or polycyclic and include, but is not limited to, a thiophenyl group, a furanyl group, a pyrrolyl group, an imidazolyl group, a thiazolyl group, an oxazolyl group, an oxadiazolyl group, a pyridinyl group, a bipyridinyl group, a pyrimidinyl group, a triazinyl group, a triazolyl group, a pyridazinyl group, a pyrazinyl group, a pyrido pyrimidyl group, a pyrido pyrazinyl group, a pyrazino pyrazinyl group, an isoxazolyl group and a thiadiazolyl group, but the scope of the present disclosure is not limited thereto.

In this specification, the term "heterocycloalkyl" means partially or fully saturated hydrocarbon containing one or more heteroatoms selected from O, N, P and S as a ring-forming atom and forming a monocycle or a fused ring. Preferably, the heteroatom may be 1, 1 to 2, or 1 to 3. For example, piperazinyl, piperazinedionyl, piperidinyl, pyrrolidinyl, morpholinyl, imidazolidinyl, imidazolidinonyl, lactamyl, tetrahydropyranyl, tetrahydrofuranyl, diazabicyclooctanyl and diazaspiroctanyl may be included, without limitation.

In this specification, the term "heterocycloalkenyl" means unsaturated hydrocarbon including one or more heteroatoms selected from O, N, P and S as ring-forming atoms and forming a monocycle or a fused ring. Preferably, the heteroatom may be 1, 1 to 2, or 1 to 3. For example, tetrahydropyridinyl may be included, without limitation.

In this specification, the term "heteroaryl" means an aromatic heterocycle and may be a monocycle or polycycle. The polycycle includes not only a form in which multiple aromatic rings are fused, but also a form in which an aromatic ring and an aliphatic ring are fused. Examples of the heteroaryl include pyrrolyl, pyrazolyl, imidazolyl, triazolyl, oxazolyl, isoxazolyl, thiazolyl, tetrazolyl, isothiazolyl, furyl, thienyl, pyridinyl, pyridyl N-oxide, pyrazinyl, pyrimidinyl, pyridazinyl, indolyl, benzothiazolyl, benzoxazolyl, benzothienyl, quinolinyl, quinazolinyl, quinoxalinyl, phthalazinyl, isoquinolinyl, thiophenyl, acridyl, indazolyl, carbazolyl, benzimidazolyl, benzocarbazolyl, benzofuranyl, benzothiophenyl, dibenzothiophenyl, pyrazolopyrimidinyl, indazolyl, pyrrolopyridinyl, pyrazolopyridinyl, or azaindolyl, but the scope of the present disclosure is not limited thereto.

In this specification, the term "amide" is a substituent which may be represented, for example, by R-C(=O)-NR'R", where R, R' and R" are each, but are not limited to, hydrogen, substituted or unsubstituted C₁₋₁₀ alkyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "alkenyl" may be a straight or branched chain. Alkenyl includes, for example, ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl or 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, 1-ethyl-2-methyl-2-propenyl, 1-heptenyl, 2-heptenyl, 3-heptenyl, 1-octenyl, 2-octenyl, 3-octenyl, 4-octenyl, 1-nonenyl, 2-nonenyl, 3-nonenyl, 4-nonenyl, 1-decenyl, 2-decenyl, 3-decenyl, 4-decenyl, and 5-decenyl, but is not limited thereto.

In this specification, the terms "carbocycle" and "carbocyclic" mean a ring in which ring-forming atoms are composed of carbon. The carbocycle may be aliphatic or aromatic, saturated or unsaturated, and monocyclic or polycyclic. The polycyclic ring may be a fused, bridged, or spiro polycyclic ring. A monocyclic carbocycle may have 3 to 17 carbon atoms, specifically 3 to 14 carbon atoms, more specifically 3 to 10 carbon atoms, and particularly specifically 3 to 7 carbon atoms.

In this specification, the term "cycloalkyl" means a monovalent aliphatic carbocycle in which one hydrogen atom is missing from an arbitrary position among the carbon atoms constituting the ring. Examples of cycloalkyl include, but are not limited to, cyclopropyl, cyclobutyl, cyclopentyl, 3-methylcyclopentyl, 2,3-dimethylcyclopentyl, cyclohexylene 3-methylcyclohexyl, 4-methylcyclohexyl, 2,3-dimethylcyclohexyl, 3,4,5-trimethylcyclohexyl, 4-tert-butylcyclohexyl, cycloheptyl, and cyclooctyl.

In this specification, the term "alkoxy" refers to a substituent having oxygen bonded to alkyl and may be a straight, branched, or cyclic chain. Examples of alkoxy include, but are not limited to, methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, and monohalogenated and polyhalogenated variants thereof.

In this specification, the term "oxy" refers to a substituent which may be represented by "-OR^{oxy}", where R^{oxy} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "carbonyl" is a substituent which may be represented by "-C(=O)R^{car}", where R^{car} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl, each of which is defined as described above. "Carbonyl" may be referred to as a "ketone", and may be referred to as "alkylketone" depending on the substituent thereof and bonding site.

In this specification, the term "thio" is a substituent which may be represented by "-SR^{thio}", where R^{thio} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "haloalkyl" is synonymous with "halogenated alkyl" and "halogen-substituted alkyl", and may mean alkyl in which an arbitrary hydrogen atom in the alkyl is substituted with an arbitrary halogen atom. For example, -CF₃, -CF₂H, -CH₂Cl, -CH₂CF₃, and -C(Cl₂)CF₃ are haloalkyl, but the scope of the present disclosure is not limited thereto.

In this specification, the term "amino" is intended to encompass monovalent primary amine, monovalent secondary amine, and monovalent tertiary amine. That is, amino refers to both a monovalent group in which two hydrogen atoms are bonded to a nitrogen atom and a monovalent group in which at least one hydrogen atom is substituted with another substituent. In the above, the group in which two hydrogen atoms are bonded to a nitrogen atom may be an unsubstituted amino, and the group in which at least one hydrogen atom is substituted with another substituent may be a substituted amino. Meanwhile, for dialkylamino, alkyl moieties may be the same or different.

In this specification, the term "imine or imino" is intended to encompass monovalent primary ketimines (-C(=NH)Rⁱ⁻¹), secondary ketimines (-C(N=Rⁱ⁻²)Rⁱ⁻¹), primary aldimines (-C(=NH)H), and secondary aldimines (-C(=NRⁱ⁻¹)H), where Rⁱ⁻¹ and Rⁱ⁻² may be, but are not limited to, each independently, C₁₋₁₀ alkyl, C₂₋₁₀ alkenyl, C₃₋₁₀ carbocycle, C₃₋₁₀ cycloalkyl, C₆₋₃₀ aryl, 3- to 30-membered heterocycle, or C₁₋₁₀ haloalkyl, and each of which may be substituted or unsubstituted, but is not limited thereto.

In this specification, the term "sulfonyl" refers to a substituent which may be represented by "-S(=O)₂R^{s1}", where R^{s1} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "thioamide" refers to a substituent which may be represented by "-C(=S)NR^{ta}₂", "-C(=S)NHR^{ta}" and "-C(=S)NH₂", where R^{ta}s are independent from each other and each may be, but are not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "sulfinyl" refers to a substituent which may be represented by "-S(=O)R^{s2}", where R^{s2} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl, and each of which is defined as described above. Sulfinyl may also be used interchangeably with "thiocarbonyl", and in a narrower sense may also be referred to as alkylthiocarbonyl in a case where R^{s2} is alkyl.

In this specification, the term "phosphoric acid" refers to a substituent which may be represented by "-OP(=O)(OH)₂" or "-OP(=O)(OH)(OR^{P})", where R^{P} may be, but is not limited to, substituted or unsubstituted C₁₋₁₀ alkyl, substituted or unsubstituted C₂₋₁₀ alkenyl, substituted or unsubstituted C₃₋₁₀ carbocycle, substituted or unsubstituted C₃₋₁₀ cycloalkyl, substituted or unsubstituted C₆₋₃₀ aryl, substituted or unsubstituted 3- to 30-membered heterocycle, or substituted or unsubstituted C₁₋₁₀ haloalkyl.

In this specification, the term "ester" is a substituent which may be represented by "-OC(=O)R^{ester}", where R^{ester} may be, but is not limited to, substituted or unsubstituted C₁₋₁₀ alkyl, substituted or unsubstituted C₃₋₁₀ carbocycle, substituted or unsubstituted C₃₋₁₀ cycloalkyl, C₆₋₃₀ aryl, substituted or unsubstituted 3- to 30-membered heterocycle, or substituted or unsubstituted C₁₋₁₀ haloalkyl.

In an embodiment, R, R^{a} to R^{f}, A and Z each may be substituted or unsubstituted with Substituent Group I-1.

Substituent Group I-1: substituted or unsubstituted C₁₋₅ alkyl, substituted or unsubstituted C₃₋₅ cycloalkyl, substituted or unsubstituted amino, substituted or unsubstituted C₆₋₈ aryl, substituted or unsubstituted 3- to 9-membered heterocycloalkyl, substituted or unsubstituted 3- to 11-membered heteroaryl, substituted or unsubstituted C₁₋₃ haloalkyl, substituted or unsubstituted C₁₋₃ alkoxy, hydroxy, halogen, aldehyde, cyano, and substituted or unsubstituted amide.

In an embodiment, the compound of Formula I may be represented by any one of the following Formulas I-1 to I-5:

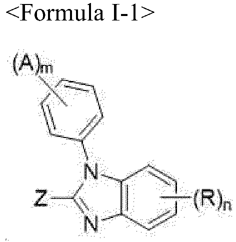

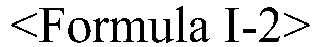

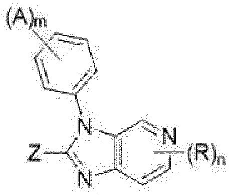

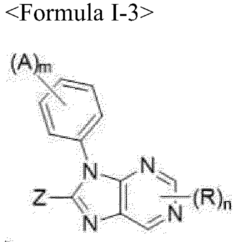

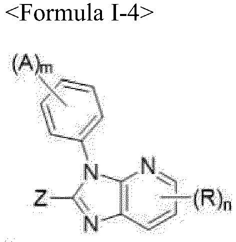

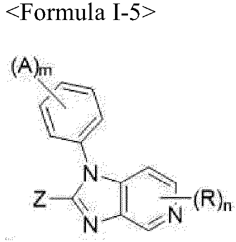

where
R, A, Z, n and m may be the same as defined above.

In an embodiment, Formula I-1 may be represented by any one of the following Formulas I-1-1 to I-1-6: 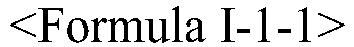 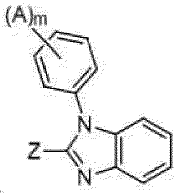 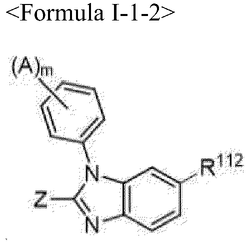 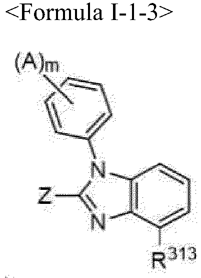 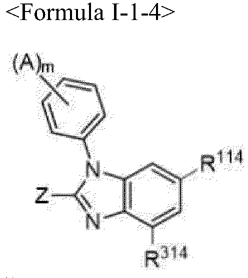 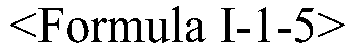 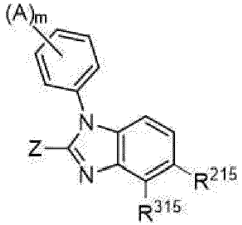 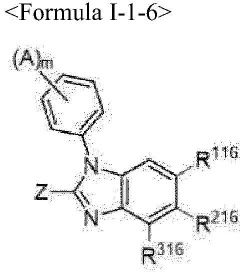 where
R¹¹², R³¹³, R¹¹⁴, R³¹⁴, R²¹⁵, R³¹⁵, R¹¹⁶, R²¹⁶ and R³¹⁶ may be the same or different, may be each independently SO₂NHR^{c}, CONHR^{d}, SO₂R^{e}, CO₂R^{f}, C₁₋₄ haloalkyl, or 3- to 9-membered heteroaryl, and may be each substituted or unsubstituted with Substituent Group I, and
R^{c}, R^{d}, R^{e}, R^{f}, A, Z and m may be the same as defined above.

In an embodiment, Formula I-2 may be represented by any one of the following Formulas I-2-1 to I-2-3:

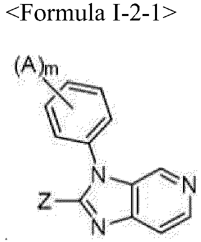

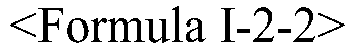

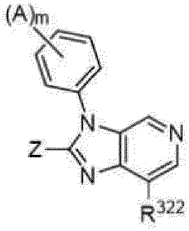

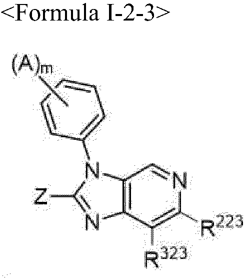

where
R³²², R²²³ and R³²³ may be the same or different, may be each independently NR^{a}R^{b}, SO₂NHR^{c}, CONHR^{d}, C₁₋₄ alkyl, or 3- to 9-membered heteroaryl, and may be each substituted or unsubstituted with Substituent Group I, and
R^{a}, R^{b}, R^{c}, R^{d}, A, Z and m may be the same as defined above.

In an embodiment, Formula I-3 may be represented by any one of the following Formulas I-3-1 to I-3-4:

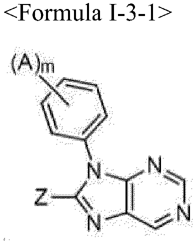

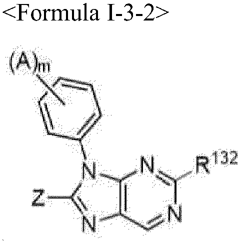

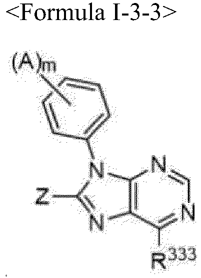

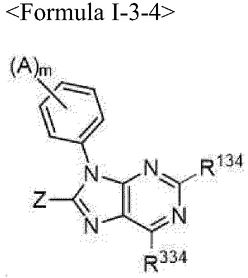

where
R¹³², R³³³, R¹³⁴ and R³³⁴ may be the same or different, may be each independently NR^{a}R^{b}, or halogen, and may be each substituted or unsubstituted with Substituent Group I, and
R^{a}, R^{b}, A, Z and m may be the same as defined above.

In an embodiment, Formula I-4 may be represented by any one of the following Formulas I-4-1 to I-4-6:

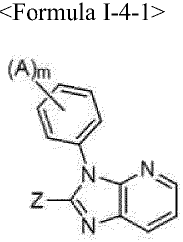

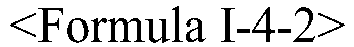

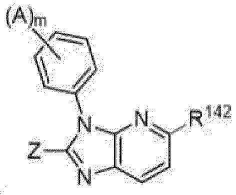

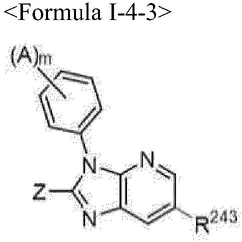

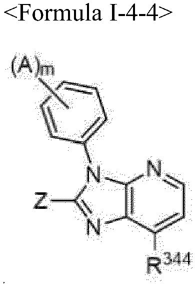

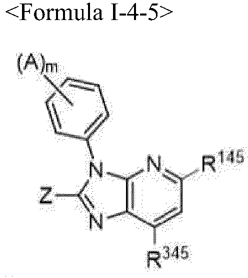

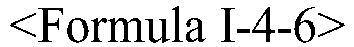

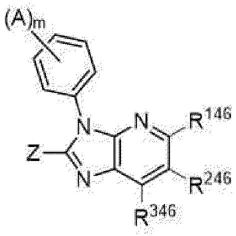

where
R¹⁴², R²⁴³, R³⁴⁴, R¹⁴⁵, R³⁴⁵, R¹⁴⁶, R²⁴⁶ and R³⁴⁶ may be the same or different, may be each independently NR^{a}R^{b} or CONHR^{d}, and may be each substituted or unsubstituted with Substituent Group I, and
R^{a}, R^{b}, R^{d}, A, Z and m may be the same as defined above.

In an embodiment, Formula I-5 may be represented by any one of the following Formulas I-5-1 to I-5-3:

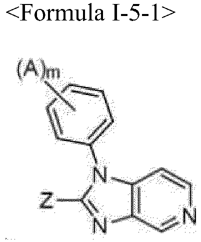

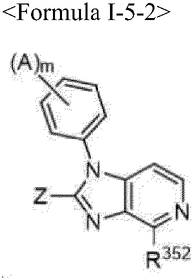

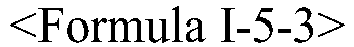

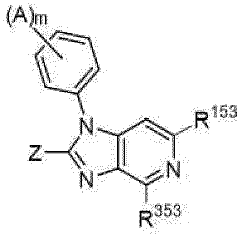

where
R³⁵², R¹⁵³ and R³⁵³ may be the same or different, may be each independently NR^{a}R^{b} or halogen, and may be each substituted or unsubstituted with Substituent Group I, and
R^{a}, R^{b}, A, Z and m may be the same as defined above.

In an embodiment, R may be each independently NR^{a}R^{b}, SO₂NHR^{c}, CONHR^{d}, SO₂R^{e}, CO₂R^{f}, chloro, C₁₋₂ haloalkyl, or pyrazolyl, where R^{a} to R^{f} may be each independently hydrogen, methyl, ethyl, propyl, pyrrolidyl or morpholinyl, and R^{a} to R^{f} may be substituted or unsubstituted with Substituent Group I-1-1, and R may be substituted or unsubstituted with Substituent Group I-1-2.

Substituent Group I-1-1: substituted or unsubstituted C₁₋₃ alkyl, substituted or unsubstituted C₃₋₅ cycloalkyl, substituted or unsubstituted amino, substituted or unsubstituted C₆₋₈ aryl, substituted or unsubstituted 3- to 9-membered heterocycloalkyl, and substituted or unsubstituted 3- to 11-membered heteroaryl; and
Substituent Group I-1-2: substituted or unsubstituted C₁₋₃ alkyl.

In an embodiment, A may be acetamide, carbamide, hydroxy, isoxazolyl, hydropyranyl or tetrahydropyranyl, or imidazolidinonyl, lactamyl, piperazinedionyl or pyrrolyl, which is formed by fusing two As from each other together with an atom bonded thereto, and A may be substituted or unsubstituted with Substituent Group I-1-3.

Substituent Group I-1-3: substituted or unsubstituted C₁₋₄ alkyl, substituted or unsubstituted C₃₋₅ cycloalkyl, and substituted or unsubstituted amide.

In an embodiment, Z may be hydrogen, methyl, hydroxy, amide, phenyl, naphthalenyl, tetrahydrofuranyl, lactamyl, tetrahydropyranyl, pyrrolyl, thiazolyl, imidazolyl, thiophenyl, isoxazolyl, pyrazolyl, pyridinyl, benzimidazolyl, indolyl, azaindolyl, indazolyl, benzofuranyl, benzothiophenyl, pyrazolopyridinyl, pyrazolopyrimidinyl, pyrrolopyridinyl, quinolinyl or isoquinolinyl, and Z may be substituted or unsubstituted with Substituent Group I-1-4.

Substituent Group I-1-4: substituted or unsubstituted C₁₋₅ alkyl, substituted or unsubstituted C₃₋₅ cycloalkyl, C₁₋₃ haloalkyl, substituted or unsubstituted C₆₋₈ aryl, substituted or unsubstituted 3- to 8-membered heteroaryl, substituted or unsubstituted 4- to 8-membered heterocycloalkyl, substituted or unsubstituted 3- to 8-membered heterocycloalkenyl, substituted or unsubstituted C₁₋₃ alkoxy, hydroxy, halogen, aldehyde, cyano, and substituted or unsubstituted amide.

In an embodiment, the compound represented by Formula I may be any one selected from Table 2 below.

In an embodiment, the compound represented by Formula I may be used as a CBP/p300 inhibitor. In this specification, CBP/p300 may mean CBP and/or p300. The CBP refers to CREB (cyclic-AMP response element binding protein) binding protein, and the p300 refers to a paralog of CBP.

The CBP/p300 induces histone H3 lysine 27 acetylation (H3K27ac) at target gene promoters, enhancers and super enhancers, and at the same time, recognizes acetylated histones via BRD to activate gene transcription. According to the overexpression of the CBP/p300 in cancer cells and drug-resistant cancer cells, oncogene transcription is activated and cancer cell proliferation, survival, tumor formation, metastasis, immune evasion or drug resistance is induced. Therefore, it is necessary to inhibit the overexpression of CBP/p300 in cancer cells, or the like. The use of the compound represented by Formula I as a CBP/p300 inhibitor in this specification may mean that the compound has the inhibition activity of the overexpression of CBP/p300 as described above. The compound represented by Formula I of the present disclosure may, for example, reduce the H3K27ac and down-regulate oncogene transcription, thereby inducing cancer cell growth inhibition and apoptosis, activating immune responses, overcoming drug resistance, and inhibiting tumor progression in vivo. The compound represented by Formula I of the present disclosure may also, for example, enhance the anticancer efficacy of chemotherapy, radiotherapy, and epigenetic anticancer agents by inhibiting BRD.

The present disclosure also provides an isomer of the compound represented by Formula I, a pharmaceutically acceptable salt thereof, a hydrate thereof, or a solvate thereof.

In this specification, the term "isomer" may include an enantiomer, a diastereomer thereof, a tautomer thereof, and a geometric isomer thereof. In this specification, enantiomers refer to two stereoisomers of a compound that are non-superimposable mirror images of each other.

In this specification, diastereomers refer to stereoisomers having two or more chiral centers and whose molecules are not mirror images of each other. Diastereomers have different physical properties such as melting points, boiling points, spectral characteristics, and reactivities. Mixtures of diastereomers may be separated under high-resolution analytical procedures such as electrophoresis and chromatography.

In this specification, geometric isomers refer to cis forms, in which the same type of atoms or groups of atoms are on the same side of a double bond, and trans forms, in which the same type of atoms or groups of atoms are on opposite sides of a double bond.

In this specification, tautomers or tautomeric forms refer to structural isomers with different energies that are compatible through a low energy barrier. For example, proton tautomers (also known as protonic tautomers) include interconversions via the transfer of a proton, including keto-enol and imine-enamine isomerization. Atomic tautomers include interconversions by retransfer of some bonding electrons.

In this specification, an arbitrary atom present in the compound of the present disclosure may be present in any of naturally occurring isotopic forms. For example, isotopes of hydrogen, carbon, nitrogen, oxygen, phosphorus, sulfur, fluorine, chlorine and iodine may be ²H ("D"), ³H, ¹¹C, ¹²C, ¹³C, ¹⁴C, ¹³N, ¹⁵N, ¹⁵O, ¹⁷O, ¹⁸O, ³²P, ³³P, ³⁵S, ¹⁸F, ³⁶Cl, ¹²³I and ¹²⁵I. In this specification, the term "acceptable salt" means a salt of an active compound prepared with an acid or a base, which depends on the particular substituent based on the compound described in this specification. If the compound according to the present disclosure contains a relatively acidic functional group, a base addition salt may be obtained by contacting such a compound in a neutral state with a sufficient amount of a desired base in the absence of a solvent or in the presence of a suitable inert solvent.

Acceptable salts include, for example, alkali metal salts, for example, sodium or potassium salt; alkaline earth metal salts, for example, calcium or magnesium salt; ammonium salt; aliphatic amine salts, for example, trimethylamine, triethylamine, dicyclohexylamine, ethanolamine, diethanolamine, triethanolamine or procaine salt; aralkyl amine salts, for example, N,N-dibenzylethylenediamine salt; heterocyclic aromatic amine salts, for example, pyridine salt, picolino salt, quinoline salt or isoquinoline salt; quaternary ammonium salts, for example, tetramethylammonium salt, tetraethylammonium salt, benzyltrimethylammonium salt, benzyltriethylammonium salt, benzyltributylammonium salt, methyltrioctylammonium salt or tetrabutylammonium salt; and basic amino acid salts, for example, arginine salt or lysine salt. The acid salts may be, for example, inorganic acid salts, for example, hydrochloride, sulfate salt, nitrate salt, phosphate salt, carbonate salt, hydrogencarbonate or perchlorate; organic acid salts, for example, acetate, propionate, lactate, malate, fumarate, tartrate, malate, fumarate, citrate, ascorbate, or formic acid; sulfonates, for example, methanesulfonate, isothionate, benzenesulfonate or p-toluenesulfonate; and acidic amino acid salts, for example, aspartate or glutamate.

The term "hydrate" in this specification refers to a compound in which water (H₂O) is bonded to another compound.

In this specification, the term "solvate" refers to an aggregate or composite of one or more solvent molecules with the compound of the present disclosure. Examples of the solvent forming a solvate include, but are not limited to, water, isopropanol, ethanol, methanol, dimethyl sulfoxide, ethyl acetate, acetic acid, and ethanolamine.

The present disclosure also provides a pharmaceutical composition for preventing or treating cancer, inflammatory disorders, or autoimmune diseases, including at least one of the compound represented by Formula I, an isomer thereof, a pharmaceutically acceptable salt thereof, a hydrate thereof, and a solvate thereof, as an active ingredient.

In an embodiment, the cancer may be cancer dependent on oncogene MYC, tumor harboring a loss of function mutation of CBP or p300, or cancer disease with activation of CBP and/or p300 function.

In an embodiment, the cancer disease may be one or more selected from the group consisting of blood cancer, prostate cancer, breast cancer, lung cancer, pancreatic cancer, colorectal cancer, and melanoma.

In an embodiment, the cancer disease may be one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

In an embodiment, the inflammatory disorder or autoimmune disease may be one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.

In an embodiment, the pharmaceutical composition may further include a pharmaceutically acceptable diluent or carrier. Specifically, the pharmaceutical composition of the present disclosure may include a pharmaceutically acceptable carrier, and may be formulated in the form of oral dosage forms such as powders, granules, tablets, capsules, suspensions, emulsions, syrups and aerosols, external preparations, suppositories and sterile injectable solutions, respectively, according to common methods. The pharmaceutically acceptable carrier may include lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinyl pyrrolidone, water, methyl hydroxybenzoate, propyl hydroxybenzoate, talc, magnesium stearates and mineral oil. In addition, the carrier may include diluents or excipients, such as fillers, weighting agents, binders, wetting agents, disintegrating agents and surfactants. An oral solid preparation may include tablets, pills, powders, granules and capsules, and the solid preparation may include one or more excipients, for example, starch, calcium carbonate, sucrose or lactose, and gelatin, and may include a lubricant such as magnesium stearate and talc. An oral liquid preparation may include suspensions, solutions, emulsions and syrups, and may include diluents such as water and liquid paraffin, wetting agents, sweeteners, flavoring agents and preservatives. A parenteral preparation may include sterile aqueous solutions, non-aqueous solvents, suspensions, emulsions, lyophilized preparations and suppositories, and the non-aqueous solvent and suspension may include propylene glycol, polyethylene glycol, vegetable oils such as olive oil, and injectable esters such as ethyl oleate. As a suppository base, witepsol, macrogol, tween 61, cacao butter, laurin butter, glycerogelatin, or the like may be used.

In an embodiment, the dosage of the compound represented by Formula I, the isomer thereof, the pharmaceutically acceptable salt thereof, the hydrate thereof or the solvate thereof, contained in the pharmaceutical composition of the present disclosure varies depending on the patient's condition, weight, the degree of the disease, the drug form, the administration route and period, but may be appropriately selected by a person skilled in the art. For example, the pyrazole derivative compound represented by Formula I, or the pharmaceutically acceptable salt thereof may be administered at a dosage of 0.0001 to 1000 mg/kg, preferably 0.01 to 1000 mg/kg per day, and the administration may be performed once a day or in several divided doses. In addition, the pharmaceutical composition of the present disclosure may contain the pyrazole derivative compound represented by Formula I, or the pharmaceutically acceptable salt thereof, in a weight percentage of 0.001 to 90% based on the total weight of the composition.

In an embodiment, the pharmaceutical composition of the present disclosure may be administered to mammals such as rats, mice, livestock, and humans by various routes, for example, orally, intraperitoneally, rectally, or by intravenous, intramuscular, subcutaneous, intrauterine or intracerebroventricular injection.

Hereinafter, the present disclosure will be described in more detail through preparation examples, examples, and experimental examples. However, the examples and experimental examples below are intended to illustrate the present disclosure, and the scope of the present disclosure is not limited thereto.

The compound having the structure of Formula I may be synthesized by the method below.

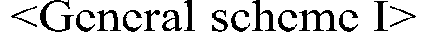

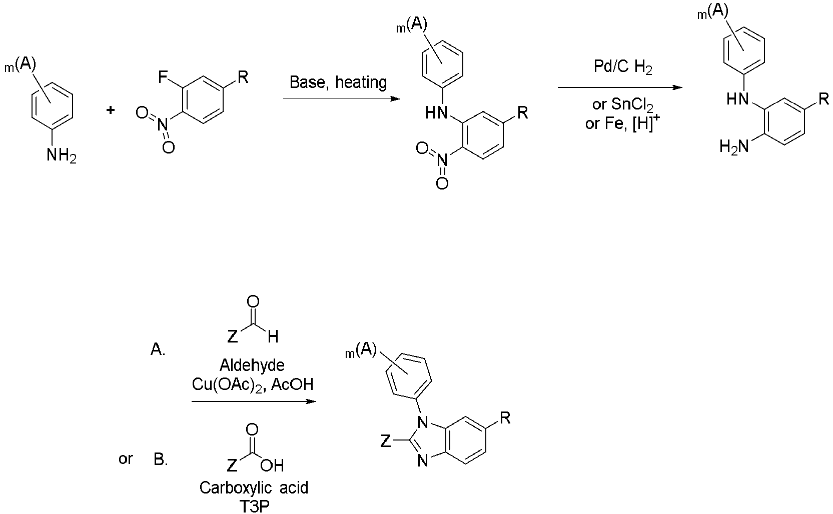

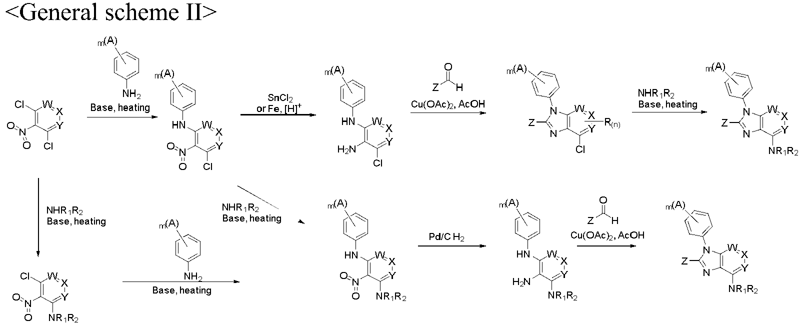

The analyses of the compounds prepared in the Preparation Examples and Examples were performed as follows: Nuclear magnetic resonance (NMR) spectroscopy was performed on a Bruker 500 MHz or Jeol 400 MHz spectrometer. Mass spectrometry was performed using LC/MS (Agilent 1260 infinity II/Agilent infinity lab LC/MSD, API-ES mode, UV detection at 220 nm/254 nm). (Column: ZORBAX RRHT StableBond C18, 2.1 x 50 mm, 1.8 µm, 80 Å, mobile phase: water/acetonitrile/formic acid (30/70/0.1%), isocratic elution for 3 min.)

In addition, medium pressure liquid chromatography (MPLC, CombiFlash Nextgen 300+) or prepHPLC (Thermo Ultimate 3000 equipped with a diode array detector, UV detection: 215 nm, 254 nm, 280 nm) was used for the purification of the compounds. The prepHPLC conditions were as follows. (Column: Agilent Prep-C18, 250 x 21.2 mm, 10 µm, mobile phase A: water/0.1% formic acid, mobile phase B: acetonitrile/0.1% formic acid, gradient elution for 30 min, A:B = 90:10-30:70)

Each starting material is a known compound and was synthesized according to the literature or purchased commercially.

### Intermediate Synthetic Method

### Intermediate I-1 (Synthetic Method I-A): 5-amino-1,3-dimethyl-1,3-dihydro-2H-benzo[d]imidazol-2-one

### Step 1) Preparation of 5-nitro-1,3-dihydro-2H-benzo[d]imidazol-2-one

4-Nitrobenzene-1,2-diamine (10 g, 65.3 mmol) was dissolved in tetrahydrofuran (365 mL), cooled to 0°C, and 1,1-carboimidazole (21 g, 130.6 mmol) was added thereto. The reaction solution was stirred at room temperature for 2 hours. The reaction solution was concentrated under reduced pressure, and the solid obtained was washed with diethyl ether, 1 N hydrochloric acid, and water. The solid was dried under vacuum to obtain the title compound. (9.59 g, 53.5 mmol, 82% yield)

¹H NMR (500 MHz, DMSO-d₆) δ 11.43 (s, 1H), 11.20 (s, 1H), 7.94 (dd, J = 8.6, 2.3 Hz, 1H), 7.71 (d, J = 2.2 Hz, 1H), 7.10 (d, J = 8.6 Hz, 1H).

### Step 2) Preparation of 1,3-dimethyl-5-nitro-1,3-dihydro-2H-benzo[d]imidazol-2-one

5-Nitro-1,3-dihydro-2H-benzo[d]imidazol-2-one (4.9 g, 27.5 mmol) was dissolved in dimethylformamide (70 mL) and cooled to 0°C. Sodium hydride (60% dispersion in mineral oil, 4.4 g, 110 mmol) was added little by little and methyl iodide (11.7 g, 5.13 mL, 83.0 mmol) was added. The reaction solution was stirred at 60°C for 2 hours, and then water was added to terminate the reaction. The reaction solution was diluted with ethyl acetate and washed with 0.1 N hydrochloric acid and brine. An organic layer was concentrated under reduced pressure, and the solid thus obtained was washed with hexane and dried under vacuum to obtain the title compound. (4.95 g, 23.4 mmol, 87% yield)

¹H NMR (500 MHz, Chloroform-d) δ 8.12 (dd, J = 8.6, 2.2 Hz, 1H), 7.89 (d, J = 2.1 Hz, 1H), 7.02 (d, J = 8.6 Hz, 1H), 3.50 (s, 3H), 3.49 (s, 3H).

### Step 3) Preparation of 5-amino-1,3-dimethyl-1,3-dihydro-2H-benzo[d]imidazol-2-one

1,3-Dimethyl-5-nitro-1,3-dihydro-2H-benzo[d]imidazol-2-one (3.5 g, 16.9 mmol) was dissolved in ethyl acetate/methanol (50 ml/50 mL), and palladium/carbon (10 wt%, 0.7 g) was added. The reaction system was replaced with hydrogen using a hydrogen balloon, and the reaction solution was stirred for 3 hours under hydrogen conditions. After the reaction was completed, the reaction solution was filtered through a celite pad to remove a catalyst. The filtrate was concentrated under reduced pressure to obtain the title compound, which was used in a subsequent reaction without further purification (2.79 g, 15.7 mmol, 93% yield).

¹H NMR (500 MHz, DMSO-d₆) δ 6.78 (d, J = 8.2 Hz, 1H), 6.35 (d, J = 2.0 Hz, 1H), 6.31 (dd, J = 8.2, 2.1 Hz, 1H), 4.77 (s, 2H), 3.22 (s, 3H), 3.21 (s, 3H).

### Intermediate I-2 (Synthetic Method I-B): Preparation of 3-fluoro-N-methyl-4-nitrobenzenesulfonamide

### Step 1) Preparation of 3-fluoro-N-methyl-4-nitrobenzenesulfonamide

A solution of 3-fluoro-4-nitrobenzenesulfonyl chloride (10 g, 41.7 mmol) in dichloromethane (100 mL) was cooled to 0°C, and then a methanamine solution (2 M in tetrahydrofuran, 52.2 mL, 104 mmol) was slowly added dropwisely. After stirring at room temperature for 2 hours, when the reaction was completed, a methylamine salt was filtered and washed with dichloromethane. The filtrate was concentrated under reduced pressure and used in a subsequent reaction without further purification (11.4 g, 48.6 mmol).

¹H-NMR (400MHz, Chloroform-d) δ 8.26-8.14 (m, 2H), 7.80 (t, J = 9.3 Hz, 2H), 4.56 (s, 1H), 2.83-2.74 (3H).

### Intermediate I-3: Preparation of 4-amino-3-((1,3-dimethyl-2-oxo-2,3-dihydro-1H-benzo[d]imidazol-5-yl)amino)-N-methylbenzenesulfonamide

### Step 1) Preparation of 3-((1,3-dimethyl-2-oxo-2,3-dihydro-1H-benzo[d]imidazol-5-yl)amino)-N-methyl-4-nitrobenzenesulfonamide

3-Fluoro-N-methyl-4-nitrobenzenesulfonamide (Intermediate I-2, 0.5 g, 2.156 mmol) was added to 1,4-dioxane (5 mL), and 5-amino-1,3-dimethyl-1,3-dihydro-2H-benzo[d]imidazol-2-one (Intermediate I-1, 420 mg, 2.37 mmol) and N,N-diisopropylethylamine (1.13 mL, 6.47 mmol) were added. The solution was heated to 100°C under reflux conditions and stirred for 18 hours. The reaction solution was cooled to 0°C and stirred for 1 hour. The precipitated solid was filtered and an orange filter cake was washed with ethyl acetate (797 mg, 2.04 mmol, 94% yield).

¹H-NMR (400 MHz, Chloroform-d) δ 9.58 (s, 1H), 8.34 (d, J = 9.1 Hz, 1H), 7.52 (t, J = 2.1 Hz, 1H), 7.11 (dd, J = 8.7, 1.8 Hz, 1H), 7.02 (d, J = 1.4 Hz, 2H), 6.89 (s, 1H), 4.31 (d, J = 5.0 Hz, 1H), 3.46 (s, 3H), 3.44-3.35 (m, 3H), 2.73-2.60 (m, 3H).

### Step 2) Preparation of 4-amino-3-((1,3-dimethyl-2-oxo-2,3-dihydro-1H-benzo[d]imidazol-5-yl)amino)-N-methylbenzenesulfonamide

3-((1,3-Dimethyl-2-oxo-2,3-dihydro-1H-benzo[d]imidazol-5-yl)amino)-N-methyl-4-nitrobenzenesulfonamide (1.03 g, 2.64 mmol) was dissolved in ethanol (26 mL), and tin(II) chloride dihydrate (SnCl₂·2H₂O) (0.89 g, 3.96 mmol) was added. The solution was heated to 80°C and stirred under reflux conditions for 4 hours. The reaction solution was cooled to 0°C, and diethyl ether (5 mL) was added thereto, followed by stirring for 30 minutes. The produced solid was filtered and a filter cake was washed with diethyl ether. A red solid was dried with nitrogen (840 mg, 2.32 mmol, 88% yield).

¹H-NMR (400MHz, DMSO-d₆) δ 7.31 (s, 1H), 7.12 (d, J = 8.2 Hz, 1H), 7.05 (s, 1H), 7.02 (d, J = 8.2 Hz, 1H), 6.94 (q, J = 5.1 Hz, 1H), 6.77 (d, J = 8.2 Hz, 1H), 6.69 (s, 1H), 6.64 (d, J = 7.3 Hz, 1H), 5.55 (s, 2H), 3.30 (s, 3H), 3.24 (s, 3H), 2.34 (d, J = 4.9 Hz, 3H).

### Intermediate I-4: Preparation of 5-((6-chloro-5-nitropyrimidin-4-yl)amino)-1,3-dimethyl-1,3-dihydro-2H-benzo[d]imidazol-2-one

To a solution of 5-amino-1,3-dimethyl-1,3-dihydro-2H-benzo[d]imidazol-2-one (Intermediate I-1, 150 mg, 0.77 mmol) dissolved in tetrahydrofuran (10 mL), 4,6-dichloro-5-nitropyrimidine (137.03 mg, 0.773 mmol) was added, and a solution of sodium bicarbonate (129.8 mg, 1.55 mmol) dissolved in water (2 mL) was added at 0°C. The reaction solution was stirred at low temperature for 1 hour. Water and ethyl acetate were added to the reaction solution, and an organic layer was extracted. The collected organic layer was washed with brine, dried over sodium sulfate, filtered, and concentrated under reduced pressure. The title compound was obtained by separation using prepHPLC (200.00 mg, 0.60 mmol, 77.73% yield).

### Intermediate I-5 (Synthetic Method I-C): Preparation of 3-fluoro-N-methyl-4-nitrobenzamide

3-Fluoro-4-nitrobenzoic acid (1 g, 5.4 mmol) and N-ethylcarbodiimide hydrochloride (1.3 g, 7.02 mmol) were dissolved in dichloromethane (15 mL), and methylamine (2 M in tetrahydrofuran, 2.7 mL, 5.4 mmol) was slowly added dropwisely at 0°C. The mixture was stirred for 2 hours while warming to room temperature. After cooling the reaction solution to 0°C, water and 1 N hydrochloric acid were added, and the resultant was extracted with dichloromethane. An organic layer was washed with 1 N sodium hydroxide, concentrated under reduced pressure, and used in a subsequent reaction without further purification.

### Intermediate I-6: Preparation of 4-(difluoromethyl)-2-fluoro-1-nitrobenzene

3-Fluoro-4-nitrobenzaldehyde (1 g, 5.91 mmol) was dissolved in dichloromethane (60 mL) and cooled to 0°C, and then diethylaminosulfur trifluoride (3.34 g, 20.70 mmol) was slowly added. The reaction solution was stirred at room temperature for 12 hours. A saturated sodium bicarbonate solution was added to the reaction solution, and the resultant was extracted with dichloromethane. The collected organic layer was treated with sodium sulfate to remove water and filtered, and the filtrate was concentrated under reduced pressure and purified by MPLC to obtain the title compound (540 mg, 5.91 mmol, 47.8% yield).

### Intermediate I-7: Preparation of 3-(3,5-dimethylisoxazol-4-yl)aniline

3-Bromoaniline (4.8 g, 27.9 mmol) was dissolved in 1,4-dioxane (100 mL), and (3,5-dimethylisoxazol-4-yl)boronic acid (5.90 g, 41.9 mmol) and sodium carbonate (2 M in water) (27.9 mL, 55.8 mmol) were added under nitrogen conditions, followed by bubbling with nitrogen for 5 minutes. Tetrakis(triphenylphosphine)palladium(0) (0.967 g, 0.837 mmol) was added to the reaction solution and the resultant was stirred at 90°C for 24 hours. Water was added to the reaction solution and subjected to extraction with ethyl acetate. An organic layer was washed with brine, concentrated under reduced pressure, and separated by MPLC to obtain the desired material (5.05 g, 26.8 mmol, 96% yield).

¹H-NMR (500 MHz, Chloroform-d) δ 7.21 (t, J = 7.8 Hz, 1H), 6.68 (dd, J = 7.9, 2.1 Hz, 1H), 6.63 (d, J = 7.6 Hz, 1H), 6.55 (d, J = 1.5 Hz, 1H), 3.73 (s, 2H), 2.40 (s, 3H), 2.26 (s, 3H).

### Intermediate I-8: Preparation of 1-(3-aminophenyl)-1,3,3-trimethylurea

### Step 1) Preparation of 1-methyl-3-(3-nitrophenyl)urea

3-Nitroaniline (300 mg, 2.172 mmol) and N-methyl-1H-imidazol-1-carboxamide (1359 mg, 10.86 mmol) were dissolved in dimethylformamide (7.2 mL), and then, N,N-diisopropylethylamine (759 µL, 4.34 mmol) was added. The reaction solution was stirred at 90°C for 18 hours. After the reaction was completed, water was added, and an organic layer was extracted using dichloromethane. After washing with brine, the organic layer was dried over anhydrous magnesium sulfate and concentrated under reduced pressure, and the residue was purified by MPLC to obtain the title compound (193 mg, 0.989 mmol, 45.5% yield).

¹H-NMR (500 MHz, DMSO-d₆) δ 9.08 (d, J = 1.8 Hz, 1H), 8.19 (s, 1H), 8.12 (m, 1H), 8.06-8.03 (m, 1H), 7.62 (d, J = 9.2 Hz, 1H), 2.80 (d, J = 4.3 Hz, 3H).

### Step 2) Preparation of 1,1,3-trimethyl-3-(3-nitrophenyl)urea

1-Methyl-3-(3-nitrophenyl)urea (193.3 mg, 0.990 mmol) was dissolved in dimethylformamide (10 mL), and then sodium hydride (60% dispersed in mineral oil, 158 mg, 3.96 mmol) was slowly added at 0°C, followed by stirring for 30 minutes, and then, iodomethane (0.247 mL, 3.96 mmol) was added. The reaction solution was stirred for 6 hours. After the reaction was completed, water was added at 0°C to terminate the reaction. The reaction solution was filtered, and the filtrate was extracted with dichloromethane. After washing an organic layer with water and brine, the organic layer was dried over anhydrous magnesium sulfate and concentrated under reduced pressure to obtain the title compound, which used directly in a subsequent step without further purification (146 mg, 0.654 mmol, 66.0% yield).

### Step 3) Preparation of 1-(3-aminophenyl)-1,3,3-trimethylurea

1,1,3-Trimethyl-3-(3-nitrophenyl)urea (146.9 mg, 0.658 mmol) was dissolved in methanol (6.6 mL) and palladium/carbon (210 mg, 0.197 mmol) was added, followed by stirring under a hydrogen balloon at room temperature for 18 hours. After the reaction was completed, the reaction product was passed through a celite filter, concentrated, and purified by MPLC to obtain the title compound (140 mg, 0.724 mmol, 110% yield).

¹H-NMR (500 MHz, DMSO-d₆) δ 6.99 (t, J = 7.9 Hz, 1H), 6.36-6.33 (m, 1H), 6.30 (s, 1H), 6.24 (d, J = 7.9 Hz, 1H), 2.98 (s, 3H), 2.63 (s, 6H).

### Intermediate I-9: Preparation of 5-amino-N-methyl-1H-indole-3-carboxamide

### Step 1) Preparation of N-methyl-5-nitro-1H-indole-3-carboxamide

5-Nitro-1H-indole-3-carboxylic acid (310 mg, 1.504 mmol), N,N-diisopropylethylamine (0.524 mL, 3.00 mmol), and 1-(bis(dimethylamino)methylene)-1H-benzo[d][1,2,3]triazol-1-ium 3-oxide tetrafluoroborate (963 mg, 3.00 mmol) were dissolved in dimethylformamide (1.5 mL), and then methaneamine (2 M in tetrahydrofuran, 1.5 mL, 3.00 mmol) was added. The reaction solution was stirred at room temperature for 18 hours. After the reaction was completed, water was added and the reaction solution was alkalized with 1 N sodium hydroxide aqueous solution, and the solid produced was filtered. A filter cake was washed with water, dried under nitrogen conditions, and used directly in a subsequent step without further purification (264 mg, 1.204 mmol, 40.1% yield).

¹H-NMR (500 MHz, DMSO-d₆) δ 9.08 (d, J = 1.8 Hz, 1H), 8.19 (s, 1H), 8.12-8.13 (m, 1H), 8.06-8.03 (m, 1H), 7.62 (d, J = 9.2 Hz, 1H), 2.80 (d, J = 4.3 Hz, 3H).

### Step 2) Preparation of 5-amino-N-methyl-1H-indole-3-carboxamide

N-Methyl-5-nitro-1H-indole-3-carboxamide (310 mg, 1.414 mmol) was dissolved in methanol (7.1 mL) and tetrahydrofuran (7.1 mL), and palladium/carbon (452 mg, 0.424 mmol) was added, followed by stirring under a hydrogen balloon at room temperature for 18 hours. After the reaction was completed, the reaction product was passed through a celite filter, and the filtrate was concentrated to obtain the title compound (264 mg, 1.395 mmol, 99% yield).

¹H-NMR (400 MHz, DMSO-d₆) δ 11.02 (s, 1H), 7.72 (d, J = 3.2 Hz, 1H), 7.59-7.58 (m, 1H), 7.31 (d, J = 2.3 Hz, 1H), 7.07 (d, J = 8.2 Hz, 1H), 6.51 (dd, J = 8.7, 2.3 Hz, 1H), 2.73 (d, J = 4.6 Hz, 3H).

### Intermediate I-10: Preparation of 5-amino-1,4-dimethyl-1,4-dihydroquinoxaline-2,3-dione

### Step 1) Preparation of 5-nitro-1,4-dihydroquinoxaline-2,3-dione

3-Nitrobenzene-1,2-diamine (500 mg, 3.26 mmol) and oxalic acid (588 mg, 6.53 mmol) were dissolved in 4 N hydrochloric acid aqueous solution (0.816 mL, 3.26 mmol), followed by stirring at 120°C for 6 hours. After the reaction was completed, the solid produced was filtered to obtain the title compound as a light green solid (483 mg, 2.332 mmol, 71.4% yield).

### Step 2) Preparation of 1,4-dimethyl-5-nitro-1,4-dihydroquinoxaline-2,3-dione

5-Nitro-1,4-dihydroquinoxaline-2,3-dione (200 mg, 0.966 mmol) was dissolved in dimethylformamide (10 mL), and then sodium hydride (dispersed 60% in mineral oil, 116 mg, 2.90 mmol) was slowly added at 0°C, followed by stirring for 30 minutes, and then iodomethane (0.247 mL, 3.96 mmol) was added. The reaction solution was stirred for 2 hours. After the reaction was completed, water was added at 0°C to terminate the reaction. The reaction solution was filtered, and the filtrate was concentrated under reduced pressure and purified by MPLC to obtain the title compound as a yellow solid (71 mg, 0.302 mmol, 31.3% yield).

¹H-NMR (500 MHz, DMSO-d₆) δ 7.76 (d, J = 2.7 Hz, 1H), 7.74 (d, J = 3.7 Hz, 1H), 7.59 (t, J = 8.2 Hz, 1H), 3.93 (s, 3H), 3.64 (s, 3H).

### Step 3) Preparation of 5-amino-1,4-dimethyl-1,4-dihydroquinoxaline-2,3-dione

1,4-Dimethyl-5-nitro-1,4-dihydroquinoxaline-2,3-dione (71.3 mg, 0.303 mmol) was dissolved in methanol (3 mL) and 1,4-dioxane (3 mL), and palladium/carbon (32.3 mg, 0.030 mmol) was added, followed by stirring under a hydrogen balloon at room temperature for 18 hours. After the reaction was completed, the reaction product was passed through a celite filter, and the filtrate was concentrated to obtain the title compound (50 mg, 0.244 mmol, 80% yield).

¹H-NMR (400 MHz, DMSO-d₆) δ 7.12 (t, J = 8.0 Hz, 1H), 6.59-6.54 (m, 2H), 5.64 (s, 2H), 3.96 (s, 3H), 3.54 (s, 3H), LC/MS 206 (M+H).

### Intermediate I-11: Preparation of N-(3-amino-5-(tetrahydro-2H-pyran-4-yl)phenyl)acetamide

### Step 1) Preparation of N-(3-bromo-5-nitrophenyl)acetamide

3-Bromo-5-nitroaniline (300 mg, 1.382 mmol) was dissolved in dichloromethane (10 mL), and acetyl chloride (0.104 mL, 1.451 mmol) and pyridine (0.223 mL, 2.76 mmol) were added. The reactants were stirred at room temperature for 2 hours. Water was added to the reaction solution and the resultant was extracted with dichloromethane. An organic layer was concentrated under reduced pressure and separated by MPLC to obtain the title compound (385.6 mg, 1.488 mmol, 108% yield).

¹H-NMR (500 MHz, Chloroform-d) δ 8.25 (s, 1H), 8.23 (s, 1H), 8.09 (s, 1H), 2.24 (s, 3H).

### Step 2) Preparation of N-(3-(3,6-dihydro-2H-pyran-4-yl)-5-nitrophenyl)acetamide

N-(3-Bromo-5-nitrophenyl)acetamide (240 mg, 0.926 mmol) was added to 1,4-dioxane (5.8 mL)/water (1.4 mL), and then 2-(3,6-dihydro-2H-pyran-4-yl)-4,4,5,5-tetramethyl-1,3,2-dioxaborolane (214 mg, 1.019 mmol) and sodium carbonate (0.463 mL, 0.926 mmol) (2 M in water) were added. 1,1'-Bis(diphenylphosphino)ferrocene]dichloropalladium(II) (Pd(dppf)Cl₂)·dichloromethane (151 mg, 0.185 mmol) was added and bubbled with nitrogen for 5 minutes. The reaction solution was stirred at 90°C for 18 hours. When the reaction was completed, water was added and the resultant was extracted with ethyl acetate. An organic layer was washed with brine, concentrated under reduced pressure, and separated by MPLC to obtain the title compound (163 mg, 0.62 mmol, 67.1%).

¹H-NMR (500 MHz, Chloroform-d) δ 8.16 (s, 1H), 8.04 (s, 1H), 7.98 (s, 1H), 7.37 (s, 1H), 6.30 (s, 1H), 4.35 (q, J = 2.7 Hz, 2H), 3.95 (t, J = 5.5 Hz, 2H), 2.53 (m, 2H), 2.23 (s, 3H).

### Step 3) Preparation of N-(3-amino-5-(tetrahydro-2H-pyran-4-yl)phenyl)acetamide

N-(3-(3,6-Dihydro-2H-pyran-4-yl)-5-nitrophenyl)acetamide (161.7 mg, 0.617 mmol) was added to methanol (6 mL), and palladium/carbon (10 wt%, 52.5 mg, 0.049 mmol) was added. The reaction solution was stirred under a hydrogen balloon at room temperature for 2 hours. The reaction solution was filtered through a celite pad and washed with methanol. The filtrate was concentrated under reduced pressure and used in a subsequent reaction without further purification. (158.4 mg, 110%).

¹H-NMR (400 MHz, Chloroform-d) δ 7.60 (s, 1H), 7.00 (1H), 6.59 (1H), 6.30 (s, 1H), 4.38-3.95 (m, 2H), 3.72 (s, 2H), 3.48 (s, 3H), 2.75-2.46 (m, 1H), 2.13 (s, 3H), 1.73 (s, 4H).

### Intermediate I-12: Preparation of 3-amino-5-(tetrahydro-2H-pyran-4-yl)phenol

### Step 1) Preparation of 3-(3,6-dihydro-2H-pyran-4-yl)-5-nitrophenol

3-Bromo-5-nitrophenol (60 mg, 0.275 mmol) was dissolved in 1,4-dioxane (1.5 mL), and 2-(3,6-dihydro-2H-pyran-4-yl)-4,4,5,5-tetramethyl-1,3,2-dioxaborolane (69.4 mg, 0.330 mmol) and sodium carbonate (2 M in water) (0.275 mL, 0.550 mmol) were added. The reaction solution was stirred for 5 minutes while bubbling with nitrogen. Tetrakis(triphenylphosphine)palladium(0) (9.54 mg, 8.26 µmol) was added to the reaction solution and stirred under reflux conditions (120°C) for 18 hours. Water was added to the reaction solution, and the resultant was extracted with dichloromethane. An organic layer was concentrated under reduced pressure. The title compound was obtained via separation using MPLC (58 mg, 0.262 mmol, 95% yield).

### Step 2) Preparation of 3-amino-5-(tetrahydro-2H-pyran-4-yl)phenol

3-(3,6-Dihydro-2H-pyran-4-yl)-5-nitrophenol (950 mg, 4.29 mmol) was added to methanol (40 mL), and palladium/carbon (137 mg, 1.288 mmol) was added. The reaction solution was stirred under a hydrogen balloon for 3 hours. The reaction solution was filtered through a celite pad and washed with methanol. The filtrate was concentrated under reduced pressure and separated by MPLC to obtain the title compound (530 mg, 2.74 mmol, 63.9% yield).

¹H-NMR (500 MHz, Chloroform-d) δ 6.19 (s, 1H), 6.13 (s, 1H), 6.05 (s, 1H), 4.68 (s, 1H), 4.06 (d, J = 11.0 Hz, 2H), 3.68 (s, 2H), 3.50 (t, J = 11.3 Hz, 2H), 2.59 (s, 1H), 1.81-1.68 (m, 4H).

In addition, intermediates synthesized in a similar manner as above and the synthetic methods are shown in Table 1.

**[Table 1]**

| **No.** | **Name** | **¹H NMR** | **Synthetic Method** |
|---|---|---|---|
| I-13 | 5-amino-1,3-diethyl-1,3-dihydro-2*H-*benzo[*d*]imidazol-2-one | ¹H NMR (500 MHz, DMSO-d₆) δ 6.83 (d, J = 8.2 Hz, 1H), 6.40 (d, J = 2.0 Hz, 1H), 6.30 (dd, J = 8.2, 2.0 Hz, 1H), 4.75 (s, 2H), 3.74 (qd, J = 7.2, 5.0 Hz, 4H), 1.16 (q, J = 7.1 Hz, 6H). | I-A |
| I-14 | 5-amino-1,3-dicyclopropyl-1,3-dihydro-2*H-*benzo[*d*]imidazol-2-one | ¹H NMR (500 MHz, DMSO-d₆) δ 6.92 (d, J = 8.2 Hz, 1H), 6.64 (s, 1H), 6.47 (d, J = 8.2 Hz, 1H), 6.35 (s, 2H), 2.75 (tq, J = 6.8, 3.5 Hz, 2H), 1.01 - 0.90 (m, 4H), 0.83 - 0.75 (m, 4H). | I-A |
| I-15 | 3-fluoro-*N*-(2-morpholinoethyl)-4-nitrobenzenesulfonamide | N/D | I-B |
| I-16 | 3-fluoro-4-nitro-*N*-(2-(pyrrolidin-1-yl)ethyl)benzenesulfonamide | N/D | I-B |
| I-17 | *N*-(cyclopropylmethyl)-3-fluoro-4-nitrobenzenesulfonamide | N/D | I-B |
| I-18 | 4-((3-fluoro-4-nitrophenyl)sulfonyl)morphol ine | N/D | I-B |
| I-19 | 3-fluoro-4-nitrobenzenesulfonamide | N/D | I-B |
| I-20 | *N*-ethyl-3-fluoro-4-nitrobenzenesulfonamide | N/D | I-B |
| I-21 | *N*-benzyl-3-fluoro-4-nitrobenzenesulfonamide | N/D | I-B |
| I-22 | 3-fluoro-*N*-isobutyl-4-nitrobenzenesulfonamide | N/D | I-B |
| I-23 | 6-chloro-*N*-methyl-5-nitropicolinamide | N/D | I-C |

A synthetic method commonly used in each example is as follows.

### Example 1 (Synthetic Method A): Preparation of N,1',3'-trimethyl-2-(1-methyl-1H-pyrazol-4-yl)-2'-oxo-2',3'-dihydro-1'H-[1,5'-bibenzo[d]imidazole]-6-sulfonamide

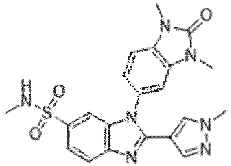

To a solution of 4-amino-3-((1,3-dimethyl-2-oxo-2,3-dihydro-1H-benzo[d]imidazol-5-yl)amino)-N-methylbenzenesulfonamide (Intermediate I-3, 30.00 mg, 83.01 µmol) and 1-methyl-1H-pyrazol-4-carbaldehyde (9.14 mg, 83.01 µmol) dissolved in acetic acid (3 mL), copper acetate (3.02 mg, 16.60 µmol) was added. The solution was heated at 110°C for 1 hour and then cooled to room temperature. Water was added, and an organic layer was extracted with ethyl acetate. Sodium sulfate was added to the collected organic layer to remove water, the resultant was filtered, and the filtrate was concentrated under reduced pressure and separated by MPLC to obtain the title compound (20.00 mg, 44.30 µmol, 53.37% yield).

¹H-NMR (400 MHz, Methanol-d₄) δ 7.86 (d, J =8.5 Hz, 1H), 7.82 (dd, J =8.5, 1.7 Hz, 1H), 7.61 (s, 1H), 7.56 (d, J =1.6 Hz, 1H), 7.53 (s, 1H), 7.44 (d, J =8.2 Hz, 1H), 7.39 (d, J =2.0 Hz, 1H), 7.27 (dd, J =8.2, 2.0 Hz, 1H), 3.84 (s, 3H), 3.55 (s, 3H), 3.44 (s, 3H), 2.48 (s, 3H). LC/MS: 452 (M+H).

### Example 20 (Synthetic Method B): Preparation of 2-(isoquinolin-8-yl)-N,1',3'-trimethyl-2'-oxo-2',3'-dihydro-1'H-[1,5'-bibenzo[d]imidazole]-6-sulfonamide

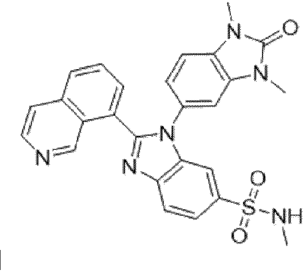

4-Amino-3-((1,3-dimethyl-2-oxo-2,3-dihydro-1H-benzo[d]imidazol-5-yl)amino)-N-methylbenzenesulfonamide (Intermediate I-3, 50 mg, 0.138 mmol) and isoquinoline-8-carboxylic acid (23.9 mg, 0.138 mmol) were dissolved in dichloromethane (1.4 mL), and propanephosphonic anhydride (50 wt% in ethyl acetate, 0.086 mL, 0.14 mmol), N.N-diisopropylethylamine (0.025 mL, 0.144 mmol), and acetic acid (2-5 drops, catalytic amount) were added. The reaction solution was heated to 80°C and stirred for one day. The reaction solution was cooled to room temperature, water was added, and an organic layer was extracted with ethyl acetate. An organic layer obtained by removing water using magnesium sulfate and filtering was concentrated under reduced pressure and separated by MPLC and PTLC to obtain the title compound.

¹H-NMR (500 MHz, Methanol-d₄) δ 9.49-9.37 (m, 1H), 8.57-8.44 (1H), 8.15 (s, 1H), 8.07 (t, J = 8.7 Hz, 2H), 7.92 (dd, J = 8.5, 1.5 Hz, 1H), 7.90 (d, J = 5.8 Hz, 1H), 7.88 (s, 1H), 7.85 (d, J = 7.0 Hz, 1H), 7.78 (t, J = 7.6 Hz, 1H), 7.33 (d, J = 12.2 Hz, 1H), 7.17-7.08 (m, 2H), 3.39 (s, 3H), 3.31 (s, 3H), 2.54 (d, J = 12.8 Hz, 3H).

### Example 21 (Synthetic Method C): Preparation of 5-(6-((3-(azepan-1-yl)propyl)(methyl)amino)-8-(1H-pyrrolo[2,3-b]pyridin-2-yl)-9H-purin-9-yl)-1,3-dimethyl-1,3-dihydro-2H-benzo[d]imidazol-2-one

### Step 1) Preparation of 5-((6-((3-(azepan-1-yl)propyl)(methyl)amino)-5-nitropyrimidin-4-yl)amino)-1,3-dimethyl-1,3-dihydro-2H-benzo[d]imidazol-2-one

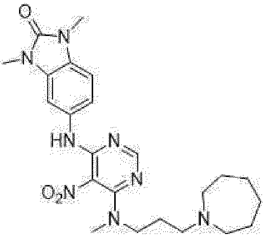

5-((6-Chloro-5-nitropyrimidin-4-yl)amino)-1,3-dimethyl-1,3-dihydro-2H-benzo[d]imidazol-2-one (Intermediate I-4, 200 mg, 601 µmol) and 3-(azepan-1-yl)-N-methylpropan-1-amine (102 mg, 0.60 mmol) were added to dichloromethane (10 mL), and N,N-diisopropylethylamine (155 mg, 1.2 mmol, 209 uL) was added. The reaction solution was heated to 100°C and stirred for 5 hours. The reaction product was concentrated under reduced pressure and separated by MPLC to obtain the title compound (200 mg, 0.42 mmol, 71.3% yield).

### Step 2) Preparation of 5-((5-amino-6-((3-(azepan-1-yl)propyl)(methyl)amino)pyrimidin-4-yl)amino)-1,3-dimethyl-1,3-dihydro-2H-benzo[d]imidazol-2-one

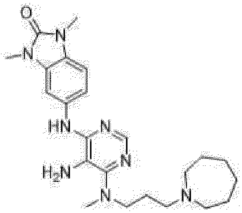

5-((6-((3-(Azepan-1-yl)propyl)(methyl)amino)-5-nitropyrimidin-4-yl)amino)-1,3-dimethyl-1,3-dihydro-2H-benzo[d]imidazol-2-one (200 mg, 0.43 mmol) was added to methanol (10 mL), and Raney Ni (100 mg, 0.43 mmol) was added. The reaction solution was stirred at room temperature for 2 hours under hydrogen conditions. The reaction solution was filtered through a celite pad to remove a catalyst, and the filtrate was concentrated under reduced pressure and used in a subsequent reaction without further purification.

### Step 3) Preparation of 5-(6-((3-(azepan-1-yl)propyl)(methyl)amino)-8-(1H-pyrrolo[2,3-b]pyridin-2-yl)-9H-purin-9-yl)-1,3-dimethyl-1,3-dihydro-2H-benzo[d]imidazol-2-one

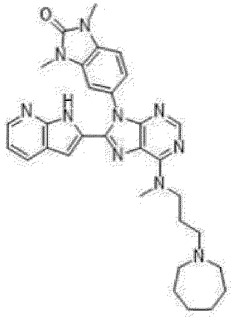

5-((5-Amino-6-((3-(azepan-1-yl)propyl)(methyl)amino)pyrimidin-4-yl)amino)-1,3-dimethyl-1,3-dihydro-2H-benzo[d]imidazol-2-one (30 mg, 0.068 mmol) and 1H-pyrrolo[2,3-b]pyridine-3-carbaldehyde (10.0 mg, 0.068 mmol) were added to ethanol (8 mL), and then acetic acid (0.5 mL) was added. The reaction solution was stirred at 60°C for 1 hour. The reaction solution was concentrated under reduced pressure and separated by MPLC to obtain the title compound. (11.40 mg, 0.020 mmol, 29.3% yield)

¹H-NMR (400 MHz, Methanol-d₄) δ 8.27-8.36 (m, 2 H), 8.10 (d, J = 8.0 Hz, 1 H), 7.42 (q, J = 6.4 Hz, 2 H), 7.24-7.28 (m, 2 H), 5.99 (s, 1 H), 4.41-4.53 (m, 2 H), 3.44-3.53 (m, 6 H), 3.41 (s, 5 H), 3.22 (t, J = 12.0 Hz, 2 H), 2.31 (t, J = 8.0 Hz, 2 H), 1.67-1.88 (m, 8 H).

### Example 34 (Synthetic Method D): Preparation of 5-(8-(1H-indol-5-yl)-6-(methyl(2-(m-tolyloxy)ethyl)amino)-9H-purin-9-yl)-1,3-dimethyl-1,3-dihydro-2H-benzo[d]imidazol-2-one

### Step 1) Preparation of 6-chloro-N-methyl-5-nitro-N-(2-(m-tolyloxy)ethyl)pyrimidin-4-amine

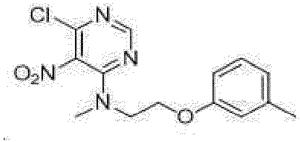

4,6-Dichloro-5-nitropyrimidine (387.95 mg, 2.00 mmol) was dissolved in acetonitrile (10 mL), and N,N-diisopropylethylamine (258.00 mg, 2.00 mmol) and N-methyl-2-(m-tolyloxy)ethan-1-amine (330.46 mg, 2.00 mmol) were added thereto at 0°C. The mixture was stirred for 30 minutes. The reaction solution was concentrated under reduced pressure and used in a subsequent reaction without further purification.

### Step 2) Preparation of 1,3-dimethyl-5-((6-(methyl(2-(m-tolyloxy)ethyl)amino)-5-nitropyrimidin-4-yl)amino)-1,3-dihydro-2H-benzo[d]imidazol-2-one

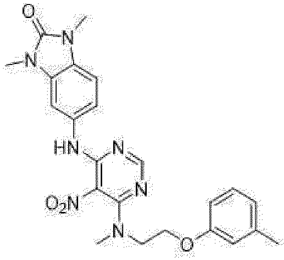

To a solution of 6-chloro-N-methyl-5-nitro-N-(2-(m-tolyloxy)ethyl)pyrimidin-4-amine (appx. 2.00 mmol) and 5-amino-1,3-dimethyl-1,3-dihydro-2H-benzo[d]imidazol-2-one (Intermediate I-1, 354.41 mg, 2.00 mmol), dissolved in dimethylformamide (30 mL), N,N-diisopropylethylamine (516.96 mg, 4.00 mmol, 0.696 mL) was added, followed by stirring at 60°C for 2 hours. The reaction solution was concentrated under reduced pressure and used in a subsequent reaction without further purification.

### Step 3) Preparation of 5-((5-amino-6-(methyl(2-(m-tolyloxy)ethyl)amino)pyrimidin-4-yl)amino)-1,3-dimethyl-1,3-dihydro-2H-benzo[d]imidazol-2-one

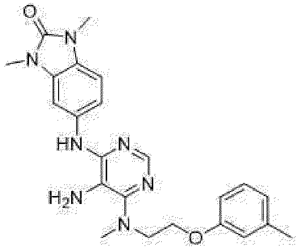

The above material (appx. 2.00 mmol) was added to acetic acid (25 mL), and zinc (780.00 mg, 12.00 mmol) was added. The reaction solution was stirred at room temperature for 30 minutes. The reaction solution was filtered through celite, and the filtrate after filtering was concentrated under reduced pressure and separated by MPLC to obtain the desired material (485.00 mg, 55.94% yield over 3 steps, crude).

### Step 4) Preparation of 5-(8-(1H-indol-5-yl)-6-(methyl(2-(m-tolyloxy)ethyl)amino)-9H-purin-9-yl)-1,3-dimethyl-1,3-dihydro-2H-benzo[d]imidazol-2-one

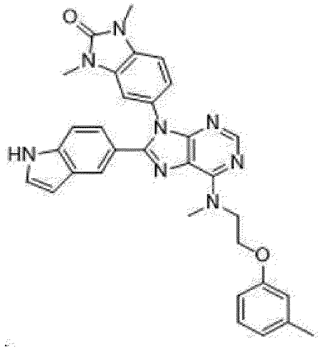

5-((5-Amino-6-(methyl(2-(m-tolyloxy)ethyl)amino)pyrimidin-4-yl)amino)-1,3-dimethyl-1,3-dihydro-2H-benzo[d]imidazol-2-one (385.00 mg, 0.88 mmol) and 1H-indole-5-carbaldehyde (154.70 mg, 1.07 mmol) were added to ethanol (30 mL), and then acetic acid (53.29 mg, 0.88 mmol) was added. The reaction solution was stirred at 60°C for 24 hours. The reaction solution was concentrated under reduced pressure and separated by MPLC to obtain the title compound (23.60 mg, 0.041 mmol, 4.71 % yield).

¹H-NMR (400 MHz, DMSO-d₆) δ 11.25 (s, 1H), 8.18 (s, 1H), 7.78 (s, 1H), 7.35 (d, J = 6.0 Hz, 3H), 7.28 (t, J = 8.4 Hz, 2H), 7.18 (t, J = 8.4 Hz, 1 H), 6.98 (d, J = 8.0 Hz, 1 H), 6.72-6.80 (m, 3 H), 6.38 (s, 1H), 4.34-4.67 (m, 4 H), 3.37 (s, 3H), 3.30 (s, 3H), 3.29 (s, 3H), 2.24 (s, 3H), LC/MS: 559 (M+H).

### Example 44 (Synthetic Method E): Preparation of (S)-2-(1-(3,4-difluorophenyl)-6-oxopiperidin-2-yl)-N,1',3'-trimethyl-2'-oxo-2',3'-dihydro-1'H-[1,5'-bibenzo[d]imidazole]-6-sulfonamide

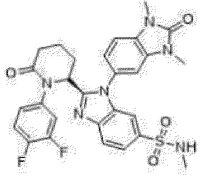

3-(1,3-Dimethyl-2-oxo-benzimidazol-5-yl)-N-methyl-2-(6-oxo-2-piperidyl)benzimidazole-5-sulfonamide (Example 43, 10 mg, 0.021 mmol) was dissolved in dichloromethane (1 mL) and then copper acetate (5.04 mg, 0.028 mmol), pyridine (17 uL, 0.213 mmol) and (3,4-difluorophenyl)boronic acid (11.8 mg, 0.075 mmol) were added. The solution was stirred at room temperature for 4 hours. Water was added to the reaction solution, the resultant was extracted with dichloromethane, and an organic layer was washed with brine. The organic layer was concentrated under reduced pressure and separated by prepHPLC to obtain the title compound (8 mg, 0.021 mmol, 64.6% yield).

¹H-NMR (500 MHz, Chloroform-d) δ 7.95 (d, J = 8.5 Hz, 1H), 7.77 (d, J = 8.2 Hz, 1H), 7.51 (s, 1H), 7.17-7.08 (m, 1H), 7.08-6.93 (m, 3H), 6.93-6.85 (m, 1H), 6.83 (s, 1H), 5.03-4.91 (m, 1H), 4.47-4.34 (m, 1H), 3.62-3.47 (m, 3H), 3.45 (d, J = 6.7 Hz, 1H), 3.32 (s, 2H), 2.86 (dt, J = 17.7, 5.5 Hz, 1H), 2.67-2.59 (m, 3H), 2.50 (m, 1H), 2.29-2.18 (m, 2H), 1.90 (m, 1H), 1.76-1.54 (m, 1H).

### Example 48 (Synthetic Method F): Preparation of 3-(1,3-dimethyl-2-oxo-benzimidazol-5-yl)-N-methyl-2-[6-(1-methylpyrazol-4-yl)-1H-indol-3-yl]benzimidazole-5-sulfonamide

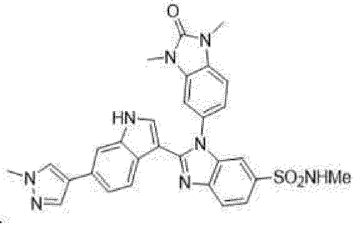

2-(6-Bromo-1H-indol-3-yl)-3-(1,3-dimethyl-2-oxo-benzimidazol-5-yl)-N-methyl-benzimidazole-5-sulfonamide (Example 42, 35 mg, 0.062 mmol) was dissolved in 1,4-dioxane (1.5 mL), and then 2 M sodium carbonate aqueous solution (42.0 mg, 0.396 mmol), 1-methyl-4-(4,4,5,5-tetramethyl-1,3,2-dioxaborolan-2-yl)-1H-pyrazole (25.8 mg, 0.124 mmol) and tetrakis(triphenylphosphine)palladium(0) (2.86 mg, 2.476 µmol) were added, followed by stirring at 100°C for 18 hours under nitrogen conditions. After the reaction was completed, ethyl acetate (10 mL) and water (10 mL) were added, and an organic layer was extracted. The organic layer was washed with water and brine, dried over anhydrous magnesium sulfate, concentrated under reduced pressure, and purified by MPLC and PTLC to obtain the title compound. (7 mg, 0.012 mmol, 19.96% yield).

¹H-NMR (500 MHz, Chloroform-d) δ 8.75-8.69 (m, 1H), 8.63-8.57 (m, 1H), 7.92 (d, J = 8.5 Hz, 1H), 7.74-7.71 (m, 2H), 7.60 (d, J = 7.6 Hz, 2H), 7.48-7.38 (m, 2H), 7.18-7.13 (m, 2H), 6.99 (m, 1H), 6.59 (d, J = 7.9 Hz, 1H), 4.49 (s, 1H), 3.93 (s, 3H), 3.53 (s, 3H), 3.38 (s, 3H), 2.63 (s, 3H)

### Example 49 (Synthetic Method G): Preparation of tert-butyl 4-(3-(1',3'-dimethyl-6-(N-methylsulfamoyl)-2'-oxo-2',3'-dihydro-1'H-[1,5'-bibenzo[d]imidazol]-2-yl)-1H-indol-6-yl)piperidine-1-carboxylate

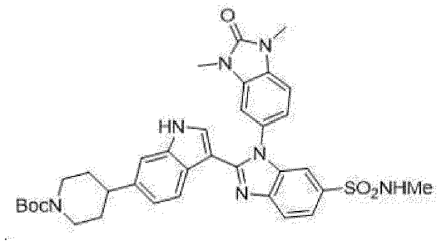

Tert-butyl-4-[3-[1-(1,3-dimethyl-2-oxo-benzimidazol-5-yl)-6-(methylsulfamoyl)benzimidazol-2-yl]-1 H-indol-6-yl]-3,6-dihydro-2H-pyridin-1-carboxylate (Example 47, 30 mg, 0.045 mmol) was added to ethanol (1.5 mL), and then palladium/carbon (9.56 mg, 8.98 µmol) was added, followed by stirring at room temperature for 18 hours under a hydrogen balloon. After the reaction was completed, the reaction product was passed through a celite filter, and the filtrate was concentrated and separated by PTLC to obtain the title compound (15 mg, 0.022 mmol, 49.8% yield).

LC/MS: 670 (M+H)

### Example 50 (Synthetic Method H): Preparation of N,1',3'-trimethyl-2'-oxo-2-(6-(piperidin-4-yl)-1H-indol-3-yl)-2',3'-dihydro-1'H-[1,5'-bibenzo[d]imidazole]-6-sulfonamide

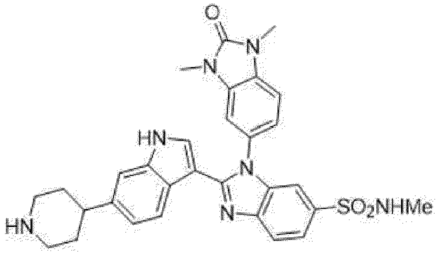

Tert-butyl-4-[3-[1-(1,3-dimethyl-2-oxo-benzimidazol-5-yl)-6-(methylsulfamoyl)benzimidazol-2-yl]-1H-indol-6-yl]piperidine-1-carboxylate (Example 49, 30 mg, 0.045 mmol) was dissolved in dichloromethane (1 mL), and trifluoroacetic acid (0.3 mL, 3.89 mmol) was added. The reaction solution was stirred at room temperature for 2 hours. After the reaction was completed, 1 N sodium hydroxide was added, and an organic layer was extracted with dichloromethane and then washed with water and brine. The organic layer was dried over anhydrous magnesium sulfate, concentrated under reduced pressure, and purified by MPLC to obtain the title compound (13 mg, 0.023 mmol, 50.9% yield).

¹H-NMR (500 MHz, DMSO-d₆) δ 11.40 (s, 1H), 8.57 (d, J = 8.2 Hz, 1H), 7.90 (d, J = 8.5 Hz, 1H), 7.65 (dd, J = 8.5, 1.5 Hz, 1H), 7.51 (d, J = 1.8 Hz, 1H), 7.47 (d, J = 8.2 Hz, 1H), 7.36 (d, J = 1.5 Hz, 1H), 7.33 (d, J = 5.2 Hz, 1H), 7.29 (dd, J = 8.2, 1.8 Hz, 1H), 7.26 (s, 1H), 7.13 (d, J = 8.5 Hz, 1H), 6.59 (d, J = 3.1 Hz, 1H), 3.47 (s, 3H), 3.38-3.36 (m, 2H), 3.34 (s, 3H), 3.17 (d, J = 5.2 Hz, 1H), 3.03-2.94 (m, 3H), 2.36 (d, J = 5.2 Hz, 3H), 1.98-1.95 (m, 2H), 1.91-1.86 (m, 2H), LC/MS: 570 (M+H)

### Example 52 (Synthetic Method I): Preparation of N,1',3'-trimethyl-2-(6-methyl-1H-indol-3-yl)-2'-oxo-2',3'-dihydro-1'H-[1,5'-bibenzo[d]imidazole]-6-carboxamide

### Step 1) Preparation of 4-amino-3-((1,3-dimethyl-2-oxo-2,3-dihydro-1H-benzo[d]imidazol-5-yl)amino)-N-methylbenzamide

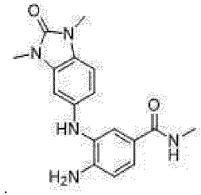

The title compound was synthesized in the same manner as in the synthesis of Intermediate I-3 using 5-amino-1,3-dimethyl-1,3-dihydro-2H-benzo[d]imidazol-2-one (Intermediate I-1) and 3-fluoro-N-methyl-4-nitrobenzamide (Intermediate I-5).

¹H-NMR (500 MHz, DMSO-d₆) δ 7.98-7.90 (m, 1H), 7.52 (s, 1H), 7.32 (d, J = 8.2 Hz, 1H), 6.96 (d, J = 8.2 Hz, 1H), 6.70 (d, J = 7.9 Hz, 1H), 6.61-6.55 (m, 1H), 6.54 (d, J = 3.7 Hz, 1H), 3.28 (s, 3H), 3.25-3.20 (3H), 2.69 (d, J = 4.6 Hz, 3H)

### Step 2) Preparation of N,1',3'-trimethyl-2-(6-methyl-1H-indol-3-yl)-2'-oxo-2',3'-dihydro-1'H-[1,5'-bibenzo[d]imidazole]-6-carboxamide

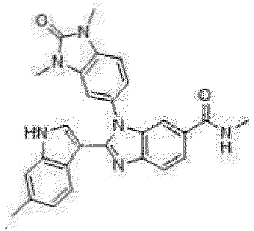

The title compound was synthesized in the same manner as in Synthetic Method A using 4-amino-3-((1,3-dimethyl-2-oxo-2,3-dihydro-1H-benzo[d]imidazol-5-yl)amino)-N-methylbenzamide (50 mg, 0.154 mmol) (8 mg, 0.017 mmol, 11.21% yield).

¹H-NMR (400 MHz, Chloroform-d) δ 8.59 (d, J = 8.7 Hz, 1H), 8.08 (s, 1H), 7.88 (d, J = 8.7 Hz, 1H), 7.59 (dd, J = 8.2, 1.4 Hz, 1H), 7.51 (s, 1H), 7.23 (s, 1H), 7.18-7.12 (2H), 7.02-6.99 (1H), 6.56 (d, J = 3.0 Hz, 1H), 6.15 (d, J = 4.6 Hz, 1H), 3.49 (d, J = 12.3 Hz, 3H), 3.40-3.33 (3H), 3.03-2.95 (3H), 2.49-2.44 (3H), LC/MS: 465 (M+H)

### Examples 55 and 56 (Synthetic Method J): Preparation of 5-(4-chloro-2-(6-methyl-1H-indol-3-yl)-1H-imidazo[4,5-c]pyridin-1-yl)-1,3-dimethyl-1,3-dihydro-2H-benzo[d]imidazol-2-one and 5-[4-(dimethylamino)-2-(6-methyl-1H-indol-3-yl)imidazo[4,5-c]pyridin-1-yl]-1,3-dimethyl-benzimidazol-2-one

### Step 1) Preparation of 5-((2-chloro-3-nitropyridin-4-yl)amino)-1,3-dimethyl-1,3-dihydro-2H-benzo[d]imidazol-2-one

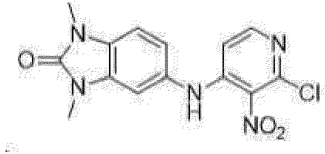

5-Amino-1,3-dimethyl-1,3-dihydro-2H-benzo[d]imidazol-2-one (Intermediate I-1, 2 g, 10.36 mmol) was dissolved in 1,4-dioxane (50 mL), and then 2,4-dichloro-3-nitropyridine (1.84 g, 10.36 mmol) and N,N-diisopropylethylamine (2 g, 15.55 mmol, 2.72 mL) were added. The reaction solution was heated to 80°C and stirred for 5 hours. The reaction product was concentrated under reduced pressure and purified by MPLC to obtain the title compound (1.8 g, 5.39 mmol, 52.0% yield).

### Step 2) Preparation of 5-((3-amino-2-chloropyridin-4-yl)amino)-1,3-dimethyl-1,3-dihydro-2H-benzo[d]imidazol-2-one

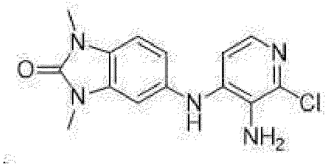

The compound 5-((2-chloro-3-nitropyridin-4-yl)amino)-1,3-dimethyl-1,3-dihydro-2H-benzo[d]imidazol-2-one (1.8 g, 5.39 mmol) obtained in Step 1 was dissolved in methanol/water (20 ml/10 mL), and then iron powder (904 mg, 16.18 mmol) and ammonium chloride (1.44 g, 27.0 mmol) were added. The reaction solution was heated to 100°C and stirred for 2 hours. The reaction solution was filtered to remove a solid, and the filtrate was concentrated under reduced pressure and then purified by MPLC to obtain the title compound (1.2 g, 3.95 mmol, 73.2% yield).

LC/MS: 304 (M+H)

### Step 3) Preparation of 5-(4-chloro-2-(6-methyl-1H-indol-3-yl)-1H-imidazo[4,5-c]pyridin-1-yl)-1,3-dimethyl-1,3-dihydro-2H-benzo[d]imidazol-2-one

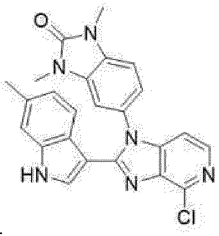

To the compound 5-((3-amino-2-chloropyridin-4-yl)amino)-1,3-dimethyl-1,3-dihydro-2H-benzo[d]imidazol-2-one (300 mg, 0.99 mmol) obtained in Step 2, dissolved in acetic acid (7 mL), 6-methyl-1H-indole-3-carbaldehyde (157 mg, 0.99 mmol) and copper acetate (179 mg, 0.99 mmol) were added. The solution was heated at 110°C for 1 hour and then cooled to room temperature. Water was added and an organic layer was extracted with ethyl acetate. Sodium sulfate was added to the collected organic layer to remove water and filtered. The filtrate was concentrated under reduced pressure and purified by MPLC to obtain the title compound (200 mg, 0.45 mmol, 45.7% yield).

¹H-NMR (400 MHz, DMSO-d₆) δ 11.30 (s, 1H), 8.53 (d, 1H), 8.07 (s, 1H), 7.54 (d, 1H), 7.36 (m, 1H), 7.29 (m, 2H), 7.09 (m, 2H), 6.54 (brs, 1H), 3.46 (s, 3H), 3.35 (s, 3H), 2.43 (s, 3H), LC/MS: 443(M+H)

As a by-product of the reaction, 5-(4-chloro-1H-imidazo[4,5-c]pyridin-1-yl)-1,3-dimethyl-1,3-dihydro-2H-benzo[d]imidazol-2-one was obtained (30 mg, 96.0 µmol, 9.7% yield).

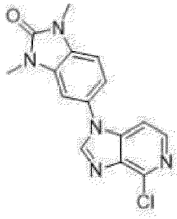

¹H-NMR (400 MHz, DMSO-d₆) δ 11.30 (s, 1H), 8.53 (d, 1H), 8.07 (s, 1H), 7.54 (d, 1H), 7.36 (m, 1H), 7.29 (m, 2H), 7.09 (m, 2H), 6.54 (brs, 1H), 3.46 (s, 3H), 3.35 (s, 3H), 2.43 (s, 3H), LC/MS: 314 (M+H)

### Step 4) Preparation of 5-[4-(dimethylamino)-2-(6-methyl-1H-indol-3-yl)imidazo[4,5-c]pyridin-1-yl]-1,3-dimethyl-benzimidazol-2-one

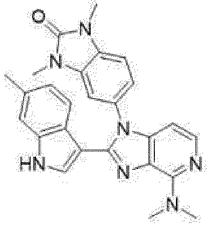

5-(4-Chloro-2-(6-methyl-1H-indol-3-yl)-1H-imidazo[4,5-c]pyridin-1-yl)-1,3-dimethyl-1,3-dihydro-2H-benzo[d]imidazol-2-one (Example 55, 50 mg, 113 µmol) was added to 2 M dimethylamine in methanol (2.8 mL, 5.64 mmol). The reaction solution was heated to 150°C in a microwave reactor and stirred for 2 hours. The reaction product was concentrated under reduced pressure and purified by MPLC to obtain the title compound (24 mg, 53 µmol, 47.1% yield).

¹H-NMR (400 MHz, DMSO-d₆) δ 11.06 (s, 1H), 8.42 (d, 1H), 7.78 (d, 1H), 7.37 (m, 2H), 7.19 (m, 1H), 7.13 (m, 1H), 7.03 (d, 1H), 6.41 (m, 2H), 3.50 (s, 6H), 3.45 (s, 3H), 3.33 (s, 3H), 2.41 (s, 3H), LC/MS: 452 (M + H)

### Example 58 (Synthetic Method K): Preparation of 1,3-dimethyl-5-[2-(6-methyl-1H-indol-3-yl)-6-(1-methylpyrazol-4-yl)benzimidazol-1-yl]benzimidazol-2-one

### Step 1) Preparation of 6-bromo-1',3'-dimethyl-2-(6-methyl-1H-indol-3-yl)-1',3'-dihydro-2'H-[1,5'-bibenzo[d]imidazol]-2'-one

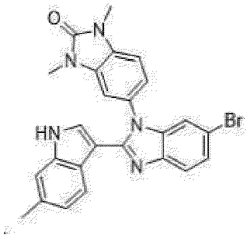

The title compound was synthesized in a similar manner to Synthetic Method I using 5-amino-1,3-dimethyl-1,3-dihydro-2H-benzo[d]imidazol-2-one (Intermediate I-1, 2 g, 10.36 mmol) and 4-bromo-2-fluoro-1-nitrobenzene (1.84 g, 10.36 mmol) (29.4% yield over 3 steps).

LC/MS: 486, 488 (M+H)

### Step 2) Preparation of 1',3'-dimethyl-2-(6-methyl-1H-indol-3-yl)-6-(1-methyl-1H-pyrazol-4-yl)-1',3'-dihydro-2'H-[1,5'-bibenzo[d]imidazole]-2'-one

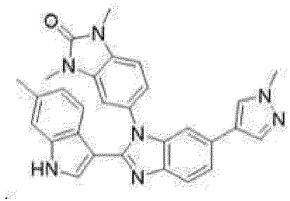

To 6-bromo-1',3'-dimethyl-2-(6-methyl-1H-indol-3-yl)-1',3'-dihydro-2'H-[1,5'-bibenzo[d]imidazole]-2'-one (50 mg, 103 µmol), 1-methyl-4-(4,4,5,5-tetramethyl-1,3,2-dioxaborolan-2-yl)-1H-pyrazole (22 mg, 0.103 mmol), potassium carbonate (22 mg, 0.154 mmol), dissolved in 1,4-dioxane (2 mL) and water (0.5 mL), 1,1'-bis(diphenylphosphino)ferrocene]dichloropalladium(II) (Pd(dppf)Cl₂)·dichloromethane (8.4 mg, 10.3 µmol) was added, followed by stirring at 110°C for 3 hours. After cooling the reaction solution to room temperature, an organic layer was extracted with ethyl acetate. The obtained organic layer was dried over anhydrous magnesium sulfate, filtered, and concentrated under reduced pressure. The title compound was obtained by purification by MPLC (27 mg, 0.055 mmol, 53.9% yield.

¹H-NMR (400 MHz, DMSO-d₆) δ 11.10 (s, 1H), 8.51 (d, 1H), 8.06 (d, 1H), 7.78 (s, 1H), 7.70 (d, 1H), 7.47 (m, 3H), 7.23 (m, 2H), 7.09 (s, 1H), 7.02 (d, 1H), 6.46 (d, 1H), 3.81 (s, 3H), 3.47 (s, 3H), 3.36 (s, 3H), 2.42 (s, 3H), LC/MS: 488 (M+H)

### Example 74 (Synthetic Method L): Preparation of N,1',3'-trimethyl-2,2'-dioxo-2,2',3,3'-tetrahydro-1'H-[1,5'-bibenzo[d]imidazole]-6-sulfonamide

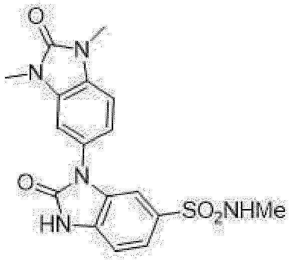

To a solution of 4-amino-3-((1,3-dimethyl-2-oxo-2,3-dihydro-1H-benzo[d]imidazol-5-yl)amino)-N-methylbenzenesulfonamide (Intermediate 1-3, 41.0 mg, 113.0 µmol) and triphosgene (11.0 mg, 39.00 µmol), dissolved in tetrahydrofuran (1 mL), triethylamine (23.0 mg, 227.0 µmol) was added. The solution was stirred at room temperature for 1 hour. Water and ethyl acetate were added, and an organic layer was extracted. Magnesium sulfate was added to the collected organic layer to remove water and filtered. The filtrate was concentrated under reduced pressure and separated by MPLC to obtain the title compound (17.0 mg, 44.0 µmol, 39% yield).

¹H NMR (500 MHz, DMSO-d₆) δ 8.74 (s, 1H), 7.98 (d, 1H), 7.89 (d, 1H), 7.72 (d, 2H), 7.58 (d, 1H), 7.72 (m, 2H), 7.38 (d, 1H), 3.43 (s, 3H), 3.39 (s, 3H), 2.38 (d, 3H), LC/MS: 388 (M+H)

### Example 77 (Synthetic Method M): Preparation of 2-(6-bromo-1H-pyrrolo[3,2-c]pyridin-3-yl)-N,1',3'-trimethyl-2'-oxo-2',3'-dihydro-1'H-[1,5'-bibenzo[d]imidazole]-6-sulfonamide

### Step 1) Preparation of sodium (6-bromo-1H-pyrrolo[3,2-c]pyridin-3-yl)(hydroxy)methanesulfonate

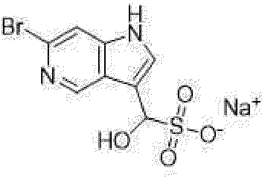

After dissolving sodium metabisulfite (169 mg, 0.889 mmol) in water (0.8 mL), a solution of 6-bromo-1H-pyrrolo[3,2-c]pyridine-3-carbaldehyde (200 mg, 0.889 mmol) dissolved in ethanol (0.8 mL) was slowly added at 0°C. The reaction solution was stirred at 50°C for 18 hours. The produced white solid was filtered to obtain the title compound (130 mg, 0.547 mmol, 95% yield).

### Step 2) Preparation of 2-(6-bromo-1H-pyrrolo[3,2-c]pyridin-3-yl)-N,1',3'-trimethyl-2'-oxo-2',3'-dihydro-1'H-[1,5'-bibenzo[d]imidazole]-6-sulfonamide

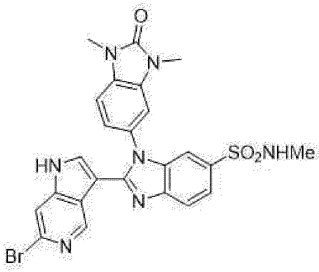

4-Amino-3-((1,3-dimethyl-2-oxo-2,3-dihydro-1H-benzo[d]imidazol-5-yl)amino)-N-methylbenzenesulfonamide (Intermediate 1-3, 89 mg, 0.246 mmol) and sodium (6-bromo-1H-pyrrolo[3,2-c]pyridin-3-yl)(hydroxy)methanesulfonate (89 mg, 0.270 mmol) were added to dimethylformamide (1.2 mL) and stirred at 80°C for 18 hours. After the reaction was completed, water (10 mL) was added, the produced solid was filtered, and the filtrate was concentrated under reduced pressure. The title compound was obtained via purification by PTLC. (80 mg, 0.246 mmol, 57.4% yield)

¹H-NMR (500 MHz, DMSO-d₆) δ 9.48 (s, 1H), 7.94 (d, J = 6.1 Hz, 1H), 7.67-7.63 (m, 2H), 7.54 (s, 1H), 7.47 (d, J = 7.6 Hz, 1H), 7.37-7.30 (m, 3H), 6.74 (s, 1H), 3.46 (s, 3H), 3.34 (s, 3H), 2.36 (d, J = 4.0 Hz, 3H), LC/MS: 566, 568 (M+H)

### Example 80 (Synthetic Method N): Preparation of 1,3-dimethyl-5-[2-(6-methyl-1H-indol-3-yl)imidazo[4,5-c]pyridin-3-yl]benzimidazol-2-one

### Step 1) Preparation of 3-((1,3-dimethyl-2-oxo-2,3-dihydro-1H-benzo[d]imidazol-5-yl)amino)-4-nitropyridine-1-oxide

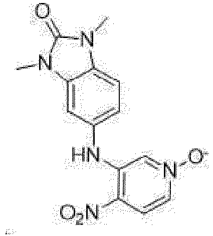

5-Amino-1,3-dimethyl-1,3-dihydro-2H-benzo[d]imidazol-2-one (Intermediate I-1, 1 g, 5.64 mmol) and 3-fluoro-4-nitropyridine 1-oxide (0.98 g, 6.21 mmol) were dissolved in 1,4-dioxane (12 mL), and N,N-diisopropylethylamine (1.97 mL, 11.3 mmol) was added. The solution was stirred at 110°C under reflux conditions for 24 hours. The reaction solution was cooled to 0°C, and the produced solid was filtered. A filter cake was washed with ethyl acetate and dried. The obtained solid was used in a subsequent reaction without further purification. (2.2 g, 124% yield)

¹H-NMR (400 MHz, DMSO-d₆) δ 9.61 (s, 1H), 8.13-8.06 (m, 1H), 7.63-7.53 (m, 2H), 7.27-7.23 (m, 3H), 7.14-7.06 (m, 1H), 3.36 (s, 6H)

### Step 2) Preparation of 5-((4-aminopyridin-3-yl)amino)-1,3-dimethyl-1,3-dihydro-2H-benzo[d]imidazol-2-one

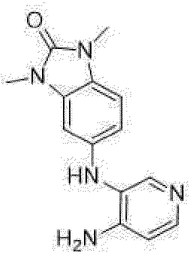

3-((1,3-Dimethyl-2-oxo-2,3-dihydro-1H-benzo[d]imidazol-5-yl)amino)-4-nitropyridine 1-oxide (2.2 g, 6.85 mmol) was added to a solution of ethanol (20 mL) and water (3.5 mL), and iron (1.9 g, 34.3 mmol) and acetic acid (10 mL, 175 mmol) were added. The reaction solution was stirred at 60°C for 5 hours. The reaction solution was filtered through a celite pad and washed with ethanol. The filtrate was concentrated under reduced pressure to remove ethanol, and the residue was diluted in ethyl acetate, and washed with water and 1 N sodium hydroxide. An organic layer was concentrated under reduced pressure and separated by MPLC to obtain the title compound. (1.3 g, 4.8 mmol, 71% yield)

LC/MS: 270 (M+H)

### Step 3) Preparation of 1,3-dimethyl-5-(2-(6-methyl-1H-indol-3-yl)-3H-imidazo[4,5-c]pyridin-3-yl)-1,3-dihydro-2H-benzo[d]imidazol-2-one

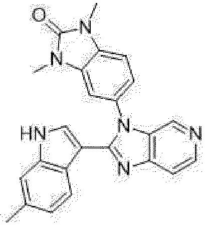

5-((4-Aminopyridin-3-yl)amino)-1,3-dimethyl-1,3-dihydro-2H-benzo[d]imidazol-2-one (300 mg, 1.11 mmol) and 6-methyl-1H-indole-3-carbaldehyde (177 mg, 1.11 mmol) were added to ethanol (6 mL), and copper acetate (101 mg, 0.58 mmol) and acetic acid (0.6 mL) were added. The reaction solution was stirred at 120°C for 4 hours. Water was added to the reaction solution and an organic layer was extracted with dichloromethane. The collected organic layer was concentrated under reduced pressure and separated by prepHPLC to obtain the title compound (72 mg, 0.18 mmol, 15.8% yield).

LC/MS: 409 (M+H)

### Example 81 (Synthetic Method O): Preparation of 2-(4-(difluoromethyl)naphthalen-1-yl)-N,1',3'-trimethyl-2'-oxo-2',3'-dihydro-1'H-[1,5'-bibenzo[d]imidazole]-6-sulfonamide

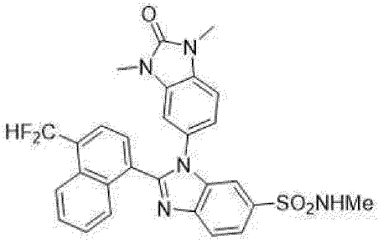

3-(1,3-Dimethyl-2-oxo-benzimidazol-5-yl)-2-(4-formyl-1-naphthyl)-N-methylbenzimidazole-5-sulfonamide (Example 79, 55 mg, 0.105 mmol) was dissolved in dichloroethane (0.55 mL), and diethylaminosulfur trifluoride (0.048 mL, 0.366 mmol) was slowly added at room temperature. The reaction solution was stirred at 80°C for 18 hours. The reaction solution was cooled to 0°C, a saturated sodium bicarbonate aqueous solution was added to terminate the reaction, dichloromethane was added, and an organic layer was extracted. The organic layer was washed with water and brine, dried over anhydrous magnesium sulfate and concentrated under reduced pressure. The residue was purified by MPLC and PTLC to obtain the title compound (5 mg, 9.13 µmol, 8.73% yield).

¹H-NMR (500 MHz, Chloroform-d) δ 8.17 (d, J = 8.2 Hz, 2H), 8.05 (d, J = 8.2 Hz, 1H), 7.91 (s, 1H), 7.87 (d, J = 8.5 Hz, 1H), 7.65-7.57 (m, 3H), 7.49 (d, J = 7.3 Hz, 1H), 7.09 (t, J = 55.5 Hz, 1H), 6.94-6.87 (m, 2H), 6.78 (s, 1H), 4.48-4.46 (m, 1H), 3.39 (s, 3H), 3.24 (s, 3H), 2.68 (d, J = 5.2 Hz, 3H), LC/MS: 548 (M+H)

### Example 83 (Synthetic Method P): Preparation of 1',3'-dimethyl-6-(N-methylsulfamoyl)-2'-oxo-N-(pyridin-3-yl)-2',3'-dihydro-1'H-[1,5'-bibenzo[d]imidazole]-2-carboxamide

### Step 1) Preparation of ethyl 1',3'-dimethyl-6-(N-methylsulfamoyl)-2'-oxo-2',3'-dihydro-1'H-[1,5'-bibenzo[d]imidazole]-2-carboxylate

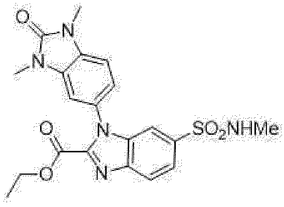

Synthesis was performed in a similar manner to Synthetic Method A using 4-amino-3-((1,3-dimethyl-2-oxo-2,3-dihydro-1H-benzo[d]imidazol-5-yl)amino)-N-methylbenzenesulfonamide (Intermediate I-3, 200 mg, 0.553 mmol) and ethyl 2-oxoacetate (0.113 ml, 0.553 mmol).

¹H-NMR (500 MHz, Chloroform-d) δ 7.23 (s, 1H), 7.13 (d, J = 8.2 Hz, 1H), 6.69 (s, 1H), 6.62 (d, J = 8.2 Hz, 1H), 6.60 (s, 1H), 5.70 (s, 1H), 4.43-4.42 (m, 1H), 4.24-4.20 (m, 2H), 3.37 (s, 3H), 3.36 (s, 3H), 2.63 (d, J = 5.2 Hz, 3H), 1.18 (t, J = 7.0 Hz, 3H)

### Step 2) Preparation of 1',3'-dimethyl-6-(N-methylsulfamoyl)-2'-oxo-2',3'-dihydro-1'H-[1,5'-bibenzo[d]imidazole]-2-carboxylic acid

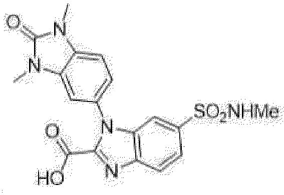

Ethyl 1',3'-dimethyl-6-(N-methylsulfamoyl)-2'-oxo-2',3'-dihydro-1'H-[1,5'-bibenzo[d]imidazole]-2-carboxylate (161 mg, 0.363 mmol) was dissolved in ethanol (0.9 mL), tetrahydrofuran (0.45 mL) and water (0.45 mL), and then lithium hydroxide (38.1 mg, 0.908 mmol) was added. The resultant was stirred at room temperature for 18 hours. After the reaction was completed, the reaction solvent was removed by concentrating under reduced pressure, and water was added to dilute. 1 N hydrochloric acid was added to adjust the pH to about 3-4, and the produced solid was filtered and washed with methanol. The filtrate was concentrated under reduced pressure and used directly in a subsequent step without further purification.

### Step 3) Preparation of 1',3'-dimethyl-6-(N-methylsulfamoyl)-2'-oxo-N-(pyridin-3-yl)-2',3'-dihydro-1'H-[1,5'-bibenzo[d]imidazole]-2-carboxamide

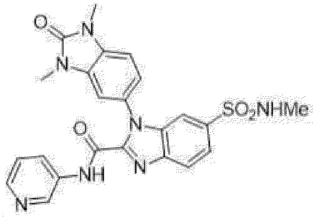

1',3'-Dimethyl-6-(N-methylsulfamoyl)-2'-oxo-2',3'-dihydro-1'H-[1,5'-bibenzo[d]imidazole]-2-carboxylic acid (130 mg, 0.313 mmol) was dissolved in dimethylformamide (3.1 mL), and hydroxybenzotriazole (HOBt) (62.3 mg, 0.407 mmol), pyridin-3-amine (29.5 mg, 0.313 mmol) and HATU (155 mg, 0.407 mmol) were added. The reaction solution was stirred at room temperature for 18 hours. After the reaction was completed, water was added to the reaction solution, and an organic layer was extracted with ethyl acetate. The organic layer was washed with water and brine, dried over anhydrous magnesium sulfate and concentrated under reduced pressure. The residue was purified by MPLC and PTLC to obtain the title compound (3 mg, 6.10 µmol, 1.950% yield).

LC/MS: 492 (M+H).

### Example 90 (Synthetic Method Q): Preparation of 3-(1,3-dimethyl-2-oxo-benzimidazol-5-yl)-2-(6-methyl-1H-indol-3-yl)benzimidazole-5-carboxylic acid

.

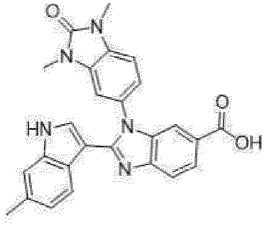

Methyl 1',3'-dimethyl-2-(6-methyl-1H-indol-3-yl)-2'-oxo-2',3'-dihydro-1'H-[1,5'-bibenzo[d]imidazole]-6-carboxylate (Example 89, 30 mg, 0.064 mmol) was added to methanol (0.5 mL)/water (0.5 mL), and lithium hydroxide (4.63 mg, 0.193 mmol) was added. The reaction solution was stirred at 60°C for 18 hours. Water and 1 N hydrochloric acid were added to the reaction solution to adjust the pH to about 2-4, and an organic layer was extracted with ethyl acetate. The organic layer was concentrated under reduced pressure and purified by prepHPLC to obtain the title compound.

¹H-NMR (500MHz, DMSO-d₆) δ 11.22 (s, 1H), 8.51 (d, J = 7.9 Hz, 1H), 7.87 (d, J = 7.9 Hz, 1H), 7.77 (d, J = 8.2 Hz, 1H), 7.53 (s, 1H), 7.49 (s, 1H), 7.45 (d, J = 8.2 Hz, 1H), 7.26 (d, J = 8.2 Hz, 1H), 7.22 (s, 1H), 7.05 (d, J = 8.2 Hz, 1H), 6.54 (d, J = 2.7 Hz, 1H), 3.46 (s, 3H), 3.34 (s, 3H), 2.42 (s, 3H) LC/MS: 452 (M+H)

### Example 101 (Synthetic Method R): Preparation of 1-(3-(3,5-dimethylisoxazol-4-yl)-5-(tetrahydro-2H-pyran-4-yl)phenyl)-N-methyl-2-(6-methyl-1H-indol-3-yl)-1H-benzo[d]imidazole-6-sulfonamide

### Step 1) Preparation of 3-(2-(6-methyl-1H-indol-3-yl)-6-(N-methylsulfamoyl)-1H-benzo[d]imidazol-1-yl)-5-(tetrahydro-2H-pyran-4-yl)phenyl trifluoromethanesulfonate

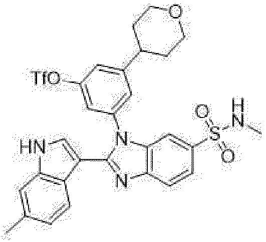

1-(3-Hydroxy-5-(tetrahydro-2H-pyran-4-yl)phenyl)-N-methyl-2-(6-methyl-1H-indol-3-yl)-1H-benzo[d]imidazole-6-sulfonamide (Example 100, 66.2 mg, 0.128 mmol) was added to tetrahydrofuran (1.5 mL), and 4-dimethylaminopyridine (47 mg, 0.384 mmol) and trifluoromethanesulfonic anhydride (0.022 mL, 0.128 mmol) were added at 0°C. The reaction solution was stirred for 24 hours. Water was added to the reaction solution, and an organic layer was extracted with ethyl acetate. The organic layer was concentrated under reduced pressure and separated by MPLC to obtain the title compound (9.2 mg, 0.014 mmol, 11.07% yield).

¹H-NMR (400 MHz, Chloroform-d) δ 8.43 (s, 1H), 8.09 (d, J = 8.2 Hz, 1H), 7.96 (d, J = 8.2 Hz, 1H), 7.80 (dd, J = 8.3, 1.7 Hz, 1H), 7.73-7.67 (1H), 7.35-7.31 (2H), 7.30 (d, J = 1.4 Hz, 1H), 7.17 (s, 1H), 7.06 (d, J = 8.2 Hz, 1H), 6.79 (d, J = 3.2 Hz, 1H), 4.46-4.33 (m, 1H), 4.04 (dd, J = 11.2, 3.4 Hz, 2H), 3.48 (td, J = 11.5, 2.6 Hz, 2H), 3.44-3.35 (m, 2H), 2.93-2.78 (m, 1H), 2.75-2.58 (3H), 2.52-2.42 (3H), 1.83-1.65 (4H)

### Step 2) Preparation of 1-(3-(3,5-dimethylisoxazol-4-yl)-5-(tetrahydro-2H-pyran-4-yl)phenyl)-N-methyl-2-(6-methyl-1H-indol-3-yl)-1H-benzo[d]imidazole-6-sulfonamide

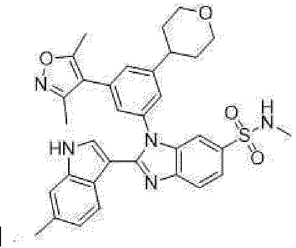

3-(2-(6-Methyl-1H-indol-3-yl)-6-(N-methylsulfamoyl)-1H-benzo[d]imidazol-1-yl)-5-(tetrahydro-2H-pyran-4-yl)phenyltrifluoromethanesulfonate (9.2 mg, 0.014 mmol) was added to 1,4-dioxane (0.5 mL), and (3,5-dimethylisoxazol-4-yl)boronic acid (3.00 mg, 0.021 mmol) and 2 M sodium carbonate in water (0.014 mL, 0.028 mmol) were added. Tetrakis(triphenylphosphine)palladium(0) (0.819 mg, 0.709 µmol) was added to the reaction solution, and nitrogen was bubbled for 5 minutes. The reaction solution was reacted at 90°C for 24 hours. Water was added to the reaction solution, and an organic layer was extracted with ethyl acetate and washed with brine. The organic layer was concentrated under reduced pressure and separated by MPLC to obtain the title compound. (3 mg, 5.04 µmol, 35.5% yield)

LC/MS: 596 (M+H)

### Example 104 (Synthetic Method S): Preparation of N-methyl-2-(3-methylimidazol-4-yl)-3-(2-oxo-1,3-dihydrobenzimidazol-5-yl)benzimidazole-5-sulfonamide

### Step 1) Preparation of N-methyl-4-nitro-3-((2-oxo-2,3-dihydro-1H-benzo[d]imidazole-5-yl)amino)benzenesulfonamide

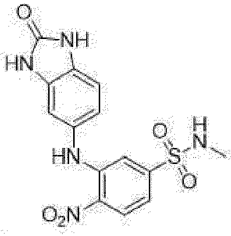

3-Fluoro-N-methyl-4-nitrobenzenesulfonamide (Intermediate I-2, 79 mg, 0.335 mmol) and 5-amino-1,3-dihydro-2H-benzo[d]imidazol-2-one (50 mg, 0.335 mmol) were added to 1,4-dioxane (1.5 mL), and N,N-diisopropylethylamine (0.117 mL, 0.670 mmol) was added. The reaction solution was stirred at 110°C for 18 hours. After the reaction was completed, the reaction solution was cooled to room temperature and the produced solid was filtered. A filter cake was washed with ethyl acetate, dried under nitrogen conditions, and used in a subsequent reaction without further purification. (56.2 mg, 0.155 mmol, 46.1% yield)

¹H-NMR (500 MHz, DMSO-d₆) δ 10.76 (d, J = 11.9 Hz, 2H), 9.54 (s, 1H), 8.29 (d, J = 8.8 Hz, 1H), 7.71 (s, 1H), 7.31 (s, 1H), 7.05 (d, J = 9.2 Hz, 1H), 7.00 (d, J = 8.5 Hz, 1H), 6.92 (s, 2H), 2.39 (s, 3H)

### Step 2) Preparation of N-methyl-2-(1-methyl-1H-imidazol-5-yl)-2'-oxo-2',3'-dihydro-1'H-[1,5'-bibenzo[d]imidazole]-6-sulfonamide

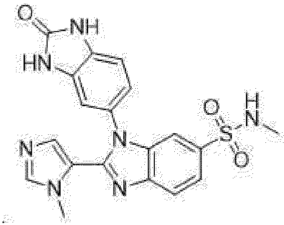

N-Methyl-4-nitro-3-((2-oxo-2,3-dihydro-1H-benzo[d]imidazole-5-yl)amino)benzenesulfonamide (56 mg, 0.154 mmol) was added to dimethyl sulfoxide (1 mL), and 1-methyl-1H-imidazole-5-carbaldehyde (25.5 mg, 0.231 mmol) and sodium hydrosulfite (95 mg, 0.462 mmol) were added. The reaction solution was stirred at 90°C for 3 hours. The reaction solution was cooled to 0°C and diluted with water and ethyl acetate, and then 25% ammonium hydroxide was added to adjust the pH to about 9-11, and an organic layer was extracted with ethyl acetate. The organic layer was concentrated under reduced pressure and separated by PTLC to obtain the title compound. (20 mg, 0.047 mmol, 30.6% yield)

¹H-NMR (500MHz, Methanol-d₄) δ 7.93 (d, J = 8.5 Hz, 1H), 7.88 (s, 1H), 7.83-7.78 (1H), 7.63 (s, 1H), 7.28 (d, J = 7.9 Hz, 1H), 7.15 (d, J = 7.9 Hz, 2H), 6.62-6.55 (1H), 4.10 (s, 3H), 2.50-2.45 (3H)

### Example 113 (Synthetic Method T): Preparation of 2-(1,6-dimethyl-1H-indol-3-yl)-1',3'-dimethyl-1',3'-dihydro-2'H-[1,5'-bibenzo[d]imidazol]-2'-one

.

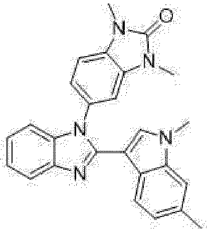

1',3'-Dimethyl-2-(6-methyl-1H-indol-3-yl)-1',3'-dihydro-2'H-[1,5'-bibenzo[d]imidazol]-2'-one (Example 111, 21.3 mg, 0.05 mmol) was dissolved in N,N-dimethylformamide (1 mL), and potassium carbonate (21.7 mg, 0.16 mmol) and methyl iodide (0.004 mL, 0.06 mmol) were added, followed by heating to 80°C and stirring for 2 hours. Water and dichloromethane were added to the reaction solution, an organic layer was extracted and washed with brine, and moisture was removed using magnesium sulfate. The organic layer was concentrated under reduced pressure and separated by MPLC to obtain the title compound (15 mg, 0.04 mmol, 68% yield).

¹H NMR (500 MHz, Chloroform-d) δ 8.43 (d, J = 8.2 Hz, 1H), 7.88 (d, J = 8.0 Hz, 1H), 7.31 - 7.27 (m, 1H), 7.21 - 7.16 (m, 2H), 7.10 (dd, J = 15.5, 7.7 Hz, 3H), 7.04 (d, J = 7.9 Hz, 1H), 7.00 (d, J = 1.8 Hz, 1H), 6.44 (s, 1H), 3.59 (s, 3H), 3.52 (s, 3H), 3.38 (s, 3H), 2.50 (s, 3H).

### Examples 2-19, 22-33, 35-43, 45-47, 51, 53, 54, 57, 59-73, 75-76, 78-79, 82, 84-89, 91-100, 102, 103, 105-112 and 114-165

Syntheses were performed similarly using the same synthetic methods as the representative examples above.

Specifically, Examples 2-19, 28-29, 38, 40-42, 51, 60-64, 67-71, 76, 79, 88, 94, 115-116, 118, 123-124, 128-135 and 153-165 were similarly prepared according to Synthetic Method A, Examples 43, 45-46 and 75 were similarly prepared according to Synthetic Method B, Examples 22-27, 30-33, 39 and 136-140 were similarly prepared according to Synthetic Method C, Examples 35-37 were similarly prepared according to Synthetic Method D, Examples 47 and 72-73 were similarly prepared according to Synthetic Method F, Examples 53-54, 65-66, 82, 85-87, 89, 91-93, 95-100, 102, 106-112, 114 and 141-152 were similarly prepared according to Synthetic Method I, Examples 57, 59 and 119-122 were similarly prepared according to Synthetic Method J, Example 78 was similarly prepared according to Synthetic Method M, Example 84 was similarly prepared according to Synthetic Method P, Example 103 was similarly prepared according to Synthetic Method Q, Example 105 was similarly prepared according to Synthetic Method S, and Examples 117 and 125-127 were similarly prepared according to Synthetic Method T.

The structures, names, and NMR/MS of the compounds synthesized from each of the examples are listed in Table 2 below.

**[Table 2]**

| Example | Compound structure | Compound name | NMR / MS |
|---|---|---|---|
| 1 | 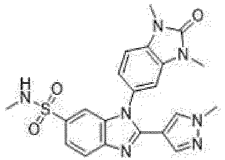 | N,1',3'-trimethyl-2-(1-methyl-1H-pyrazol-4-yl)-2'-oxo-2',3'-dihydro-1'H-[1,5'-bibenzo[d]imidazole ]-6-sulfonamide | ¹H-NMR (400 MHz, Methanol-d₄) δ 7.86 (d, J =8.5 Hz, 1H), 7.82 (dd, J =8.5, 1.7 Hz, 1H), 7.61 (s, 1H), 7.56 (d, J =1.6 Hz, 1H), 7.53 (s, 1H), 7.44 (d, J =8.2 Hz, 1H), 7.39 (d, J =2.0 Hz, 1H), 7.27 (dd, J =8.2, 2.0 Hz, 1H), 3.84 (s, 3H), 3.55 (s, 3H), 3.44 (s, 3H), 2.48 (s, 3H); m/z 452 |
| 2 | 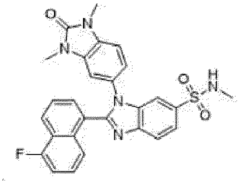 | 2-(5-fluoronaphthalen-1-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 8.14 (d, J= 8.5 Hz, 1H), 8.07 (d, J= 8.6 Hz, 1H), 7.87 (d, J= 8.6 Hz, 1H), 7.83-7.75 (m, 2H), 7.66 (d, J= 1.7 Hz, 1H), 7.62 (dd, J= 8.5, 7.2 Hz, 1H), 7.56-7.48 (m, 1H), 7.48-7.42 (m, 2H), 7.39 (dd, J= 10.8, 7.7 Hz, 1H), 7.14 (d, J= 8.2 Hz, 1H), 7.09 (dd, J = 8.2, 2.0 Hz, 1H), 3.28 (s, 3H), 3.21 (s, 3H), 2.41 (d, J= 5.0 Hz, 3H); m/z 516 |
| 3 | 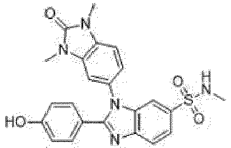 | 2-(4-hydroxyphenyl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 9.98 (s, 1H), 7.91 (d, J= 8.5 Hz, 1H), 7.67 (dd, J= 8.5, 1.8 Hz, 1H), 7.49-7.46 (m, 2H), 7.45-7.43 (m, 2H), 7.37 (s, 1H), 7.37-7.34 (m, 1H), 7.17 (dd, J= 8.1, 2.0 Hz, 1H), 6.77-6.69 (m, 2H), 3.42 (s, 3H), 3.31 (s, 4H), 2.35 (d, J= 5.1 Hz, 3H); m/z 464 |
| 4 | 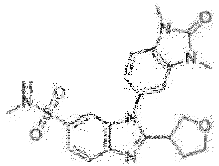 | N,1',3'-trimethyl-2'-oxo-2-(tetrahydrofuran-3-yl)-2',3'-dihydro-1'H-[1,5'-bibenzo[d]imidazole ]-6-sulfonamide | ¹H-NMR (400 MHz, DMSO-d₆) δ 7.89 (d, J= 8.5 Hz, 1H), 7.66 (dd, J= 8.4, 1.8 Hz, 1H), 7.50 (s, 1H), 7.46-7.39 (m, 2H), 7.35 (d, J= 5.1 Hz, 1H), 7.32-7.26 (m, 1H), 3.90 (s, 3H), 3.73 (q, J= 7.4 Hz, 1H), 3.57 (q, J= 7.5 Hz, 1H), 3.43 (s, 3H), 3.36 (s, 4H),2.34 (d, J= 4.9 Hz, 4H); m/z 442 |
| 5 | 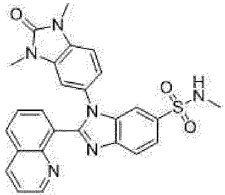 | N,1',3'-trimethyl-2'-oxo-2-(quinolin-8-yl)-2',3'-dihydro-1'H-[1,5'-bibenzo[d]imidazole ]-6-sulfonamide | ¹H-NMR (400 MHz, DMSO-d₆) δ 11.10 (d, J =2.4 Hz, 1 H), 8.28 (d, J = 8.4 Hz, 1 H), 8.12 (s, 1 H), 7.51 (q, J = 2.8 Hz, 1 H), 7.38 (q, J = 3.6 Hz, 2 H), 7.31 (d, J = 1.6 Hz, 1 H), 7.17 (t, J = 5.6 Hz, 3 H), 6.87 (d, J = 5.2 Hz, 1 H), 6.41 (d, J = 2.8 Hz, 1 H), 3.45 (s, 3 H), 3.34 (s, 6 H), 3.30 (s, 2 H), 3.11 (s, 2 H), 2.39 (s, 3 H); m/z 499 |
| 6 | 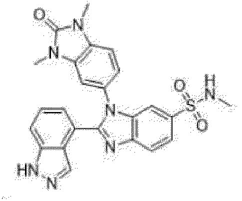 | 2-(1H-indazol-4-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 13.30 (s, 1H), 8.48 (s, 1H), 8.07 (d, J = 8.5 Hz, 1H), 7.75 (dd, J= 8.5, 1.8 Hz, 1H), 7.62 (d, J= 8.4 Hz, 1H), 7.57 (d, J= 1.7 Hz, 1H), 7.49 (d, J= 2.0 Hz, 1H), 7.46-7.40 (m, 1H), 7.32 (d, J = 8.2 Hz, 1H), 7.29-7.18 (m, 2H), 7.12-7.01 (m, 1H), 3.39 (s, 3H), 3.27 (s, 3H), 2.38 (d, J= 5.0 Hz, 3H); m/z 488 |
| 7 | 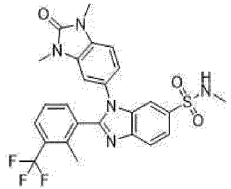 | N,1',3'-trimethyl-2-(2-methyl-3-(trifluoromethyl)phe nyl)-2'-oxo-2',3'-dihydro-1'H-[1,5'-bibenzo[d]imidazole ]-6-sulfonamide | ¹H-NMR (400 MHz, DMSO-d₆) δ 8.02 (d, J =8.5 Hz, 1H), 7.80-7.72 (d, 3H), 7.63 (d, J =1.7 Hz, 1H), 7.49-7.37 (m, 3H), 7.24 (d, J =8.2 Hz, 1H), 7.16 (dd, J =8.2, 2.0 Hz, 1H), 3.34 (s, 3H), 3.26 (s, 3H), 2.39 (d, J =5.1 Hz, 3H), 2.34 (s, J =1.8 Hz, 3H); m/z 531 |
| 8 | 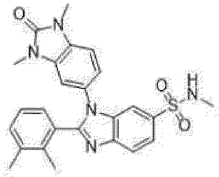 | 2-(2,3-dimethylphenyl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 7.98 (d, J= 8.5 Hz, 1H), 7.73 (dd, J= 8.5, 1.8 Hz, 1H), 7.60 (d, J= 1.7 Hz, 1H), 7.45-7.38 (m, 1H), 7.36 (d, J= 2.0 Hz, 1H), 7.26 (d, J= 7.6 Hz, 1H), 7.21 (dd, J= 8.0, 2.4 Hz, 2H), 7.11-7.05 (m, 2H), 3.33 (s, 3H), 3.26 (s, 3H), 2.39 (d, J = 5.1 Hz, 4H), 2.22 (s, 3H), 2.09 (s, 3H); m/z 476 |
| 9 | 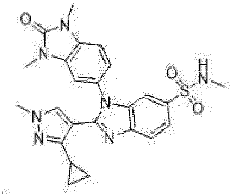 | 2-(3-cyclopropyl-1-methyl-1H-pyrazol-4-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 7.88 (d, J =8.5 Hz, 1H), 7.65 (dd, J =8.4, 1.8 Hz, 1H), 7.46 (d, J =2.0 Hz, 1H), 7.40 (d, J =8.2 Hz, 1H), 7.38-7.32 (m, 2H), 7.18 (dd, J =8.3, 2.0 Hz, 1H), 6.93 (s, 1H), 3.60 (s, 3H), 3.44 (s, 3H), 3.34 (s, 3H), 2.35 (d, J =5.1 Hz, 3H), 0.91 (dd, J =8.4, 2.8 Hz, 2H), 0.85-0.78 (m, 2H); m/z 492 |
| 10 | 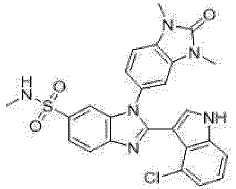 | 2-(4-chloro-1H-indol-3-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 11.84 (s, 1H), 7.97 (d, J =8.5 Hz, 1H), 7.78 (d, J =2.8 Hz, 1H), 7.72 (dd, J =8.4, 1.8 Hz, 1H), 7.59 (d, J =1.7 Hz, 1H), 7.40 (d, J =7.2 Hz, 2H), 7.35 (d, J =2.0 Hz, 1H), 7.19 (d, J =8.3 Hz, 1H), 7.15-7.07 (m, 2H), 7.04 (d, J =7.4 Hz, 1H), 3.29 (s, 3H), 3.23 (s, 3H), 3.17 (s, 3H), 2.39 (d, J =5.0 Hz, 3H); m/z 521 |
| 11 | 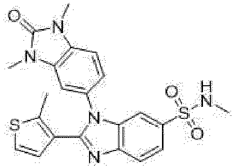 | N,1',3'-trimethyl-2-(2-methylthiophen-3-yl)-2'-oxo-2',3'-dihydro-1'H-[1,5'-bibenzo[d]imidazole ]-6-sulfonamide | ¹H-NMR (400 MHz, DMSO-d₆) δ 7.97 (d, J =8.4 Hz, 1H), 7.70 (dd, J =8.5, 1.8 Hz, 1H), 7.54 (d, J =1.7 Hz, 1H), 7.45-7.35 (m, 2H), 7.33 (d, J =8.2 Hz, 1H), 7.21 (d, J =5.4 Hz, 1H), 7.16 (dd, J =8.2, 2.0 Hz, 1H), 6.66 (d, J =5.4 Hz, 1H), 3.40 (s, 3H), 3.30 (s, 3H), 2.66 (s, 3H), 2.36 (d, J =5.0 Hz, 3H); m/z 468 |
| 12 | 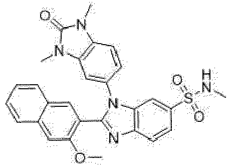 | 2-(3-methoxynaphthalen-2-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 8.21 (s, 1H), 8.00 (d, J =8.4 Hz, 1H), 7.96-7.89 (m, 1H), 7.81 (d, J =8.3 Hz, 1H), 7.75 (dd, J =8.5, 1.7 Hz, 1H), 7.65 (d, J =1.7 Hz, 1H), 7.57-7.48 (m, 1H), 7.44-7.38 (m, 2H), 7.36 (d, J =2.0 Hz, 1H), 7.28 (s, 1H), 7.15 (d, J =8.3 Hz, 1H), 6.99 (dd, J =8.2, 2.0 Hz, 1H), 3.58 (s, 3H), 3.30 (s, 3H), 3.26 (s, 3H), 2.40 (d, J =5.1 Hz, 3H); m/z 528 |
| 13 | 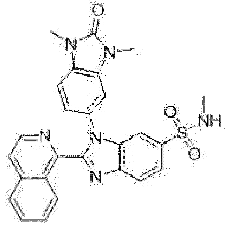 | 2-(isoquinolin-1-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 8.46 (dd, J= 16.8, 7.1 Hz, 2H), 8.09 (dd, J= 23.8, 8.4 Hz, 2H), 7.92 (d, J= 5.6 Hz, 1H), 7.89-7.77 (m, 2H), 7.78-7.69 (m, 2H), 7.52-7.44 (m, 1H), 7.40 (d, J = 2.0 Hz, 1H), 7.15 (d, J= 8.2 Hz, 1H), 7.03 (dd, J= 8.2, 2.0 Hz, 1H), 3.31 (s, 3H), 3.23 (s, 3H), 2.42 (d, J= 5.1 Hz, 3H); m/z 499 |
| 14 | 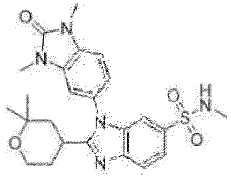 | 2-(2,2-dimethyltetrahydro-2H-pyran-4-yl)-N,1',3'-trimethyl-2'-oxo-2',3'-dihydro-1'H-[1,5'-bibenzo[d]imidazole ]-6-sulfonamide | ¹H-NMR (400 MHz, Methanol-d₄) δ 7.96-7.84 (m, 2H), 7.63 (s, 1H), 7.45 (d, J =7.9 Hz, 2H), 7.33 (t, J =8.7 Hz, 1H), 3.76 (dd, J =12.3, 4.8 Hz, 1H), 3.66 (t, J =12.0 Hz, 1H), 3.54 (s, 3H), 3.47 (s, 3H), 2.47 (s, 3H), 1.98 (dd, J =18.5, 7.0 Hz, 1H), 1.91-1.83 (m, 3H), 1.23 (d, J =3.7 Hz, 3H), 1.06 (d, J =5.1 Hz, 3H); m/z 484 |
| 15 | 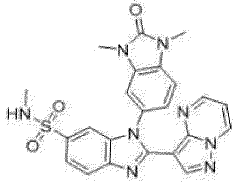 | N,1',3'-trimethyl-2'-oxo-2-(pyrazolo[1,5-a]pyrimidin-3-yl)-2',3'-dihydro-1'H-[1,5'-bibenzo[d]imidazole ]-6-sulfonamide | ¹H-NMR (400 MHz, Methanol-d₄) δ 9.21 (s, 1H), 9.03 (s, 1H), 8.10 (d, J =31.0 Hz, 2H), 7.74 (s, 1H), 7.57-7.44(m, 18.9 Hz, 5H), 3.58 (s, 3H), 3.44 (s, 3H), 2.51 (s, 3H); m/z 489 |
| 16 | 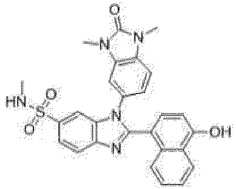 | 2-(4-hydroxynaphthalen-1-yl)-N,1',3'-trimethyl-2'-oxo-2',3'-dihydro-1'H-[1,5'-bibenzo[d]imidazole]-6-sulfonamide | ¹H-NMR (400 MHz, DMSO-d₆) δ 10.62 (s, 1H), 8.16 (d, J= 7.7 Hz, 1H), 8.07-7.98 (m, 2H), 7.75 (dd, J = 8.6, 1.8 Hz, 1H), 7.61 (d, J= 1.7 Hz, 1H), 7.53-7.36 (m, 5H), 7.14 (d, J= 8.2 Hz, 1H), 7.07-7.00 (m, 1H), 6.79 (d, J= 7.9 Hz, 1H), 3.29 (s, 3H), 3.22 (s, 3H), 2.40 (d, J= 5.0 Hz, 3H); m/z 514 |
| 17 | 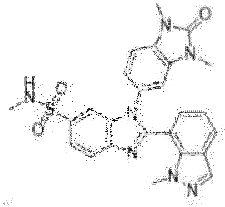 | N,1',3'-trimethyl-2-(1-methyl-1H-indazol-7-yl)-2'-oxo-2',3'-dihydro-1'H-[1,5'-bibenzo[d]imidazole ]-6-sulfonamide | ¹H-NMR (400 MHz, DMSO-d₆)δ 8.15 (s, 1H), 8.06 (d, J= 8.6 Hz, 1H), 7.86 (d, J= 8.1 Hz, 1H), 7.78 (dd, J= 8.4, 1.8 Hz, 1H), 7.65 (d, J= 1.7 Hz, 1H), 7.55-7.42 (m, 3H), 7.21 (d, J= 2.4 Hz, 2H), 7.15-7.06 (m, 1H), 3.81 (s, 3H), 3.32 (s, 3H), 3.25 (s, 3H), 2.41 (d, J= 4.9 Hz, 3H); m/z 502 |
| 18 | 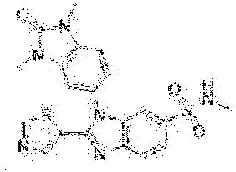 | N,1',3'-trimethyl-2'-oxo-2-(thiazol-5-yl)-2',3'-dihydro-1'H-[1,5'-bibenzo[d]imidazole ]-6-sulfonamide | ¹H-NMR (400 MHz, DMSO-d₆) δ 9.19 (s, 1H), 7.97 (d, J =8.5 Hz, 1H), 7.80 (s, 1H), 7.72 (d, J =8.7 Hz, 1H), 7.59 (d, J =2.0 Hz, 1H), 7.52-7.39 (m, 3H), 7.39-7.31 (m, 1H), 3.46 (s, 3H), 3.34 (s, 3H), 2.37 (d, J =4.9 Hz, 3H); m/z 455 |
| 19 | 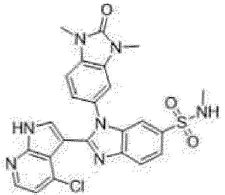 | 2-(4-chloro-1H-pyrrolo[2,3-b]pyridin-3-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 8.19 (d, J =5.2 Hz, 1H), 7.98 (d, J =8.6 Hz, 1H), 7.90 (d, J =2.8 Hz, 1H), 7.73 (dd, J =8.6, 1.8 Hz, 1H), 7.60 (d, J =1.7 Hz, 1H), 7.46-7.35 (m, 2H), 7.24-7.16 (m, 2H), 7.13 (dd, J =8.3, 1.9 Hz, 1H), 3.31 (s, 3H), 3.25 (s, 3H), 2.39 (d, J =5.0 Hz, 3H); m/z 522, 523 |
| 20 | 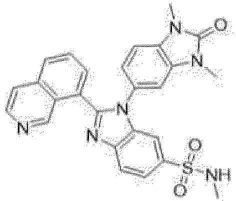 | 2-(isoquinolin-8-yl)-N,1',3'-trimethyl-2'-oxo-2',3'-dihydro-1'H-[1,5'-bibenzo[d]imidazole ]-6-sulfonamide | ¹H-NMR (500MHz, Methanol-d₄) δ 9.49-9.37 (m, 1H), 8.57-8.44 (1H), 8.15 (s, 1H), 8.07 (t, J = 8.7 Hz, 2H), 7.92 (dd, J = 8.5, 1.5 Hz, 1H), 7.90 (d, J = 5.8 Hz, 1H), 7.88 (s, 1H), 7.85 (d, J = 7.0 Hz, 1H), 7.78 (t, J = 7.6 Hz, 1H), 7.33 (d, J = 12.2 Hz, 1H), 7.17-7.08 (m, 2H), 3.39 (s, 3H), 3.31 (s, 3H), 2.54 (d, J = 12.8 Hz, 3H); m/z 499 |
| 21 | 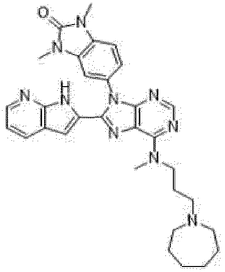 | 5-(6-((3-(azepan-1-yl)propyl)(methyl)a mino)-8-(1H-pyrrolo[2,3-b]pyridin-2-yl)-9H-purin-9-yl)-1,3-dimethyl-1,3-dihydro-2H-benzo[d]imidazol-2-one | ¹H-NMR (400 MHz, Methanol-d₄) δ 8.27-8.36 (m, 2 H), 8.10 (d, J = 8.0 Hz, 1 H), 7.42 (q, J = 6.4 Hz, 2 H), 7.24-7.28 (m, 2 H), 5.99 (s, 1 H), 4.41-4.53 (m, 2 H), 3.44-3.53 (m, 6 H), 3.41 (s, 5 H), 3.22 (t, J = 12.0 Hz, 2 H), 2.31 (t, J = 8.0 Hz, 2 H), 1.67-1.88 (m, 8 H); m/z 565 |
| 22 | 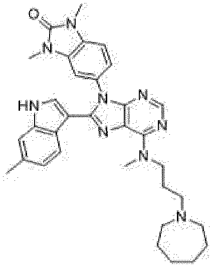 | 5-(6-((3-azepan-1-yl)propyl)(methyl)a mino)-8-(6-methyl-1H-indol-3-yl)-9H-purin-9-yl)-1,3-dimethyl-1,3-dihydro-2H-benzo[d]imidazol-2-one | ¹H-NMR (400 MHz, Methanol-d₄) δ 7.36 (d, J = 8.0 Hz, 2 H), 7.22 (q, J = 4.0 Hz, 2 H), 7.09-7.21 (m, 3 H), 6.61 (s, 1 H), 4.45 (s, 2 H), 3.60 (d, J = 12.4 Hz, 2 H), 3.52 (s, 3 H), 3.41 (s, 3 H), 3.35 (t, J = 8.0 Hz, 3 H), 3.08 (t, J = 8.0 Hz, 2 H), 2.46 (s, 3 H), 2.33 (t, J = 8.0 Hz, 2 H), 1.51-1.67 (m, 8 H); m/z 578 |
| 23 | 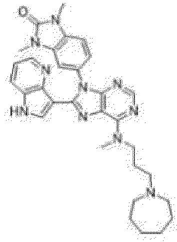 | 5-(6-((3-(azepan-1-yl)propyl)(methyl)a mino)-8-(1H-pyrrolo[3,2-b]pyridin-3-yl)-9H-purin-9-yl)-1,3-dimethyl-1H-benzo[d]imidazol-2(3H)-one | ¹H-NMR (400 MHz, Methanol-d₄) δ 8.42 (d, J = 8.0 Hz, 1 H), 8.21 (s, 1 H), 7.88 (d, J = 8.0 Hz, 1 H), 7.41 (s, 1 H), 7.32 (d, J = 1.6 Hz, 1 H), 7.27 (q, J = 4.0 Hz, 2 H), 7.19 (d, J = 8.0 Hz, 1 H), 7.10 (q, J = 8.0 Hz, 1 H), 4.31 (s, 2 H), 3.47-3.50 (m, 3 H), 3.44 (s, 3 H), 3.38 (s, 3 H), 3.12-3.13 (m, 2 H), 3.00 (s, 4 H), 2.17 (d, J = 8.0 Hz, 2 H), 1.28-1.51 (m, 8 H); m/z 565 |
| 24 | 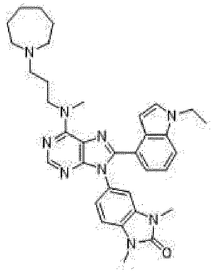 | 5-(6-((3-(azepan-1-yl)propyl)(methyl)a mino)-8-(1-ethyl-1H-indol-4-yl)-9H-purin-9-yl)-1,3-dimethyl-1,3-dihydro-2H-benzo[d]imidazol-2-one | ¹H-NMR (400 MHz, Methanol-d₄) δ 8.32 (s, 1 H), 7.55 (d, J = 8.0 Hz, 1 H), 7.41 (d, J = 3.2 Hz, 1 H), 7.27 (d, J = 2.0 Hz, 1 H), 7.16 (d, J = 8.0 Hz, 1 H), 6.96-7.06 (m, 3 H), 6.78 (d, J = 4.0 Hz, 1 H), 4.24-4.34 (m, 4 H), 3.50 (s, 2 H), 3.27 (s, 3 H), 3.26 (s, 3 H), 3.19 (t, J = 8.0 Hz, 3 H), 3.15 (t, J = 1.2 Hz, 2 H), 2.96 (t, J = 12.0 Hz, 2 H), 2.26-2.29 (m, 2 H), 1.45 (s, 1 H), 1.31 (t, J = 20.0 Hz, 4 H), 1.25 (s, 6 H); m/z 592 |
| 25 | 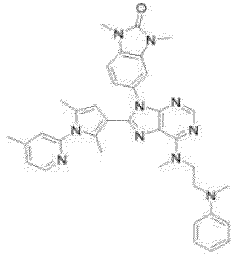 | 5-(8-(2,5-dimethyl-1-(4-methylpyridin-2-yl)-1H-pyrrol-3-yl)-6-(methyl(2-(methyl(phenyl)amin o)ethyl)amino)-9H-purin-9-yl)-1,3-dimethyl-1,3-dihydro-2H-benzo[d]imidazol-2-one | ¹H-NMR (400 MHz, Methanol-d₄) δ 8.47 (d, J = 5.2 Hz, 1 H), 8.22 (s, 1 H), 7.42 (1 H), 7.35-7.39 (m, 2 H), 7.28 (s, 1 H), 7.23 (q, J = 2.0 Hz, 1 H), 7.14 (t, J = 7.6 Hz, 2 H), 6.87 (d, J = 7.6 Hz, 2 H), 6.73 (d, J = 8.0 Hz, 1 H), 5.39 (s, 1 H), 4.57-4.64 (m, 2 H), 3.90 (t, J = 8.0 Hz, 2 H), 3.05 (s, 3 H), 2.51 (s, 3 H), 2.37 (s, 3 H), 1.87 (s, 3 H); m/z 627 |
| 26 | 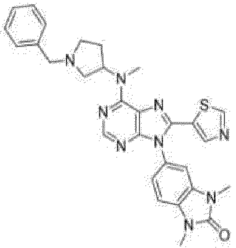 | 5-(6-((1-benzylpyrrolidin-3-yl)(methyl)amino)-8-(thiazol-5-yl)-9H-purin-9-yl)-1,3-dimethyl-1,3-dihydro-2H-benzo[d]imidazol-2-one | ¹H-NMR (400 MHz, DMSO-d₆) δ 9.07 (s, 1 H), 8.15 (s, 1 H), 7.64 (s, 1 H), 7.34 (q, J = 20.0 Hz, 3 H), 7.27 (3 H), 7.16 (d, J = 8.4 Hz, 3 H), 3.59 (d, J = 3.6 Hz, 2 H), 3.38 (d, J = 43.2 Hz, 6 H), 2.90 (d, J= 4.0 Hz, 1 H), 2.83 (t, J = 20.0 Hz, 2 H), 2.39-2.52 (m, 3 H), 2.09 (s, 1 H), 1.94-2.06 (m, 2 H), 1.64 - 1.65 (m, 1 H); m/z 552 |
| 27 | 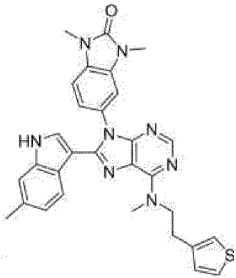 | 1,3-dimethyl-5-(6-(methyl(2-(thiophen-3-yl)ethyl)amino)-8-(6-methyl-1H-indol-3-yl)-9H-purin-9-yl)-1,3-dihydro-2H-benzo[d]imidazol-2-one | ¹H-NMR (400 MHz, Methanol-d₄) δ 7.86 (d, J =8.5 Hz, 1H), 7.82 (dd, J =8.5, 1.7 Hz, 1H), 7.61 (s, 1H), 7.56 (d, J =1.6 Hz, 1H), 7.53 (s, 1H), 7.44 (d, J =8.2 Hz, 1H), 7.39 (d, J =2.0 Hz, 1H), 7.27 (dd, J =8.2, 2.0 Hz, 1H), 3.84 (s, 3H), 3.55 (s, 3H), 3.44 (s, 3H), 2.48 (s, 3H); m/z 549 |
| 28 | 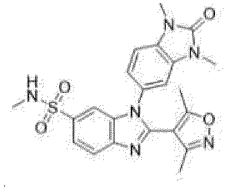 | 2-(3,5-dimethylisoxazol-4-yl)-N,1',3'-trimethyl-2'-oxo-2',3'-dihydro-1'H-[1,5'-bibenzo[d]imidazole ]-6-sulfonamide | ¹H-NMR (400 MHz, DMSO-d₆) δ 8.02 (d, J =8.5 Hz, 1H), 7.74 (dd, J =8.4, 1.8 Hz, 1H), 7.71 (d, J =1.6 Hz, 1H), 7.49-7.40 (m, 2H), 7.34 (d, J =8.2 Hz, 1H), 7.22 (dd, J =8.3, 2.0 Hz, 1H), 3.39 (s, 3H), 3.30 (s, 3H), 2.37 (d, J =5.0 Hz, 3H), 2.18 (s, 3H), 2.08 (s, 3H); m/z 467 |
| 29 | 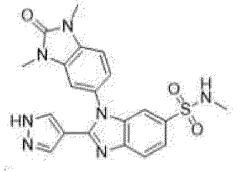 | N,1',3'-trimethyl-2'-oxo-2-(1H-pyrazol-4-yl)-2',3'-dihydro-1'H-[1,5'-bibenzo[d]imidazole ]-6-sulfonamide | ¹H-NMR (400 MHz, DMSO-d₆) δ 13.20 (s, 1H), 7.86 (d, J = 8.4 Hz, 1H), 7.65 (dd, J = 8.4, 1.8 Hz, 1H), 7.58-7.48 (m, 3H), 7.44 (d, J = 8.2 Hz, 1H), 7.39 (d, J = 1.8 Hz, 1H), 7.37-7.30 (m, 1H), 7.25 (dd, J = 8.2, 2.0 Hz, 1H), 3.45 (s, 3H), 3.34 (s, 3H), 2.35 (d, J = 4.9 Hz, 3H); m/z 438 |
| 30 | 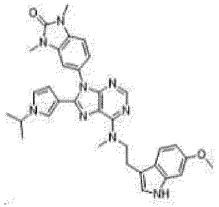 | 5-(8-(1-isopropyl-1H-pyrrol-3-yl)-6-((2-(6-methoxy-1H-indol-3-yl)ethyl)(methyl)ami no)-9H-purin-9-yl)-1,3-dimethyl-1,3-dihydro-2H-benzo[d]imidazol-2-one | ¹H-NMR (400 MHz, Methanol-d₄) δ 8.05 (s, 1 H), 7.50 (d, J = 8.4 Hz, 1 H), 7.36 (d, J = 8.0 Hz, 1 H), 7.23 (d, J = 1.6 Hz, 1 H), 7.14 (q, J = 3.2 Hz, 1 H), 7.00 (s, 1 H), 6.81 (q, J = 2.0 Hz, 2 H), 6.71 (t, J = 2.4 Hz, 1 H), 6.51 (q, J = 3.6 Hz, 1 H), 5.98 (q, J = 1.6 Hz, 1 H), 4.78 (t, J = 4.8 Hz, 2 H), 3.78-4.76 (m, 1 H), 3.75 (s, 3 H), 3.47-3.74 (m, 9 H), 3.24 (t, J = 6.8 Hz, 2 H), 1.33 (d, J = 6.8 Hz, 6 H); m/z 590 |
| 31 | 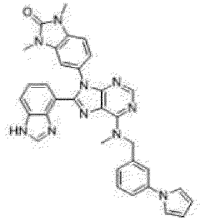 | 5-(6-((3-(1H-pyrrol-1-yl)benzyl)(methyl)a mino)-8-(1H-benzo[d]imidazol-4-yl)-9H-purin-9-yl)-1,3-dimethyl-1,3-dihydro-2H-benzo[d]imidazol-2-one | ¹H-NMR (400 MHz, Methanol-d₄) δ 8.32 (s, 1 H), 7.80 (d, J = 8.4 Hz, 1 H), 7.50 (s, 1 H), 7.34-7.42 (m, 4 H), 7.24 (q, J = 8.0 Hz, 3 H), 7.08-7.13 (q, J = 7.6 Hz, 3 H), 6.24 (t, J = 2.4 Hz, 2 H), 3.68-3.85 (m, 2 H), 3.65 (s, 3 H,), 3.48 (s, 3 H), 3.40 (s, 3 H); m/z 581 |
| 32 | 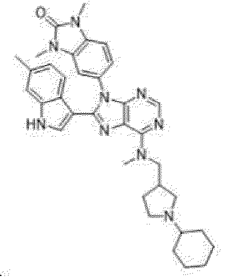 | 5-(6-(((1-cyclohexylpyrrolidin -3-yl)methyl)(methyl)a mino)-8-(6-methyl-1H-indol-3-yl)-9H-purin-9-yl)-1,3-dimethyl-1,3-dihydro-2H-benzo[d]imidazol-2-one | ¹H-NMR (400 MHz, Methanol-d₄) δ 8.24 (d, J = 8.0 Hz, 2 H), 8.15 (s, 1 H), 7.33 (q, J = 8.4 Hz, 2 H), 7.16-7.22 (m, 2 H), 7.07 (d, J = 8.0 Hz, 1 H), 6.61 (s, 1 H), 3.51 (s, 3 H), 3.47 (s, 2 H), 3.40 (s, 3 H), 3.17 (d, J = 8.0 Hz, 2 H), 3.05 (s, 2 H), 2.55 (s, 1 H), 2.45 (s, 3 H), 2.23 (q, J = 7.2 Hz, 1 H), 1.91 (t, J = 5.6 Hz, 1 H), 1.78 (s, 2 H), 1.59 (s, 2 H), 1.49 (d, J = 12.0 Hz, 1 H), 1.28 (s, 2 H), 0.97-1.10 (m, 4 H); m/z 604 |
| 33 | 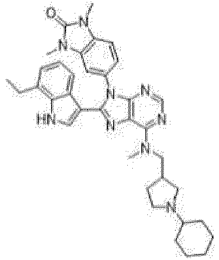 | 5-(6-(((1-cyclohexylpyrrolidin -3-yl)methyl)(methyl)a mino)-8-(7-ethyl-1H-indol-3-yl)-9H-purin-9-yl)-1,3-dimethyl-1,3-dihydro-2H-benzo[d]imidazol-2-one | ¹H-NMR (400 MHz, Methanol-d₄) δ 7.97-8.28 (m, 2 H), 7.34-7.41 (m, 2 H), 7.20-7.25 (m, 2 H), 7.12 (t, J = 7.6 Hz, 1 H), 6.76 (d, J = 104.8 Hz, 1 H), 3.61-3.75 (m, 2 H), 3.53 (s, 3 H), 3.51 (s, 2 H), 3.29 (d, J = 4.0 Hz, 3 H), 3.13-3.20 (m, 2 H), 2.88 (q, J = 11.6 Hz, 2 H), 2.84 (s, 1 H), 2.01-2.46 (m, 2 H), 1.97 (q, J = 9.6 Hz, 2 H), 1.38-1.61 (m, 4 H), 1.27 -1.31 (m, 3 H), 0.95-1.26 (m, 5 H); m/z 618 |
| 34 | 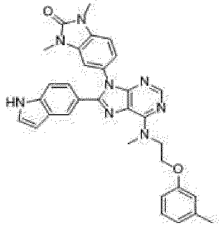 | 5-(8-(1H-indol-5-yl)-6-(methyl(2-(m-tolyloxy)ethyl)amino )-9H-purin-9-yl)-1,3-dimethyl-1,3-dihydro-2H-benzo[d]imidazol-2-one | ¹H-NMR (400 MHz, DMSO-d₆) δ 11.25 (s, 1H), 8.18 (s, 1H), 7.78 (s, 1H), 7.35 (d, J = 6.0 Hz, 3H), 7.28 (t, J = 8.4 Hz, 2H), 7.18 (t, J = 8.4 Hz, 1 H), 6.98 (d, J = 8.0 Hz, 1 H), 6.72-6.80 (m, 3 H), 6.38 (s, 1H), 4.34-4.67 (m, 4 H), 3.37 (s, 3H), 3.30 (s, 3H), 3.29 (s, 3H), 2.24 (s, 3H); m/z 559 |
| 35 | 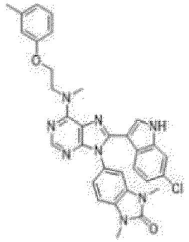 | 5-(8-(6-chloro-1H-indol-3-yl)-6-(methyl(2-(m-tolyloxy)ethyl)amino )-9H-purin-9-yl)-1,3-dimethyl-1,3-dihydro-2H-benzo[d]imidazol-2-one | ¹H-NMR (400 MHz, DMSO-d₆) δ 11.35 (s, 1 H), 8.43 (d, J = 8.4 Hz, 1 H), 8.17 (s, 1 H), 7.47 (s, 1 H,), 7.37-7.40 (m, 2 H), 7.12-7.24 (m, 2 H), 6.99 (s, 1 H), 6.80-6.84 (m, 2 H), 6.75 (d, J = 7.6 Hz, 1 H), 6.54 (d, J = 2.8 Hz, 1 H), 4.41-4.69 (m, 4 H), 3.86 (s, 3 H), 3.44 (s, 3 H), 3.33 (s, 3 H), 2.26 (s, 3 H); m/z 593 |
| 36 | 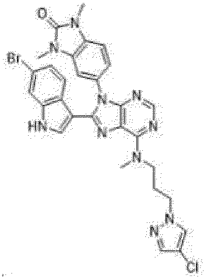 | 5-(8-(6-bromo-1H-indol-3-yl)-6-((3-(4-chloro-1H-pyrazol-1-yl)propyl)(methyl)a mino)-9H-purin-9-yl)-1,3-dimethyl-1H-benzo[d]imidazol-2(3H)-one | ¹H-NMR (400 MHz, DMSO-d₆) δ 8.42 (d, J = 8.4 Hz, 1 H), 8.11 (s, 1 H), 7.97 (s, 1 H), 7.66 (d, J = 1.6 Hz, 1 H), 7.52 (s, 1 H), 7.33-7.39 (m, 3 H), 7.15 (q, J = 2.4 Hz, 1 H), 6.58 (s, 1 H), 4.25 (t, J = 7.2 Hz, 2 H), 3.78 (s, 5 H), 3.45 (s, 3 H), 3.34 (s, 3 H), 2.31 (s, 2 H); m/z 647 |
| 37 | 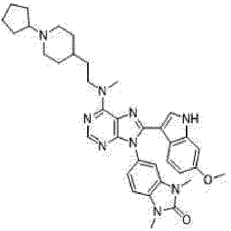 | 5-(6-((2-(1-cyclopentylpiperidin -4-yl)ethyl)(methyl)ami no)-8-(6-methoxy-1H-indol-3-yl)-9H-purin-9-yl)-1,3-dimethyl-1,3-dihydro-2H-benzo[d]imidazol-2-one | ¹H-NMR (400 MHz, DMSO-d₆) δ 11.03 (d, J = 2.4 Hz, 1 H), 8.40 (d, J = 8.8 Hz, 1 H), 8.08 (s, 1 H), 7.38 (d, J = 8.0 Hz, 2 H), 7.16 (dd, J = 1.6, 6.4 Hz, 1 H), 6.91 (d, J = 2.4 Hz, 1 H), 6.79 (d, J = 2.0 Hz, 1 H), 6.34 (d, J = 2.8 Hz, 1 H), 3.78 (s, 3 H), 3.44 (s, 3 H), 3.33 (s, 7 H), 3.21 (d, J = 3.6 Hz, 1 H), 2.93 (d, J = 11.2 Hz, 2 H), 2.49 (d, J = 3.6, 2 H), 1.81 (t, J = 12.8 Hz, 2 H), 1.60-1.75 (m, 6 H), 1.46-1.59 (m, 4 H), 1.23-1.34 (m, 4 H); m/z 634 |
| 38 | 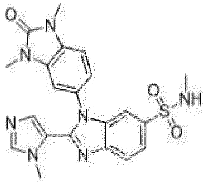 | N,1',3'-trimethyl-2-(1-methyl-1H-imidazol-5-yl)-2'-oxo-2',3'-dihydro-1'H-[1,5'-bibenzo[d]imidazole ]-6-sulfonamide | ¹H-NMR (500 MHz, DMSO-d₆) δ 7.96 (d, J = 8.5 Hz, 1H), 7.87 (s, 1H), 7.69 (dd, J = 8.5, 1.8 Hz, 1H), 7.50 (d, J = 1.9 Hz, 1H), 7.41 (d, J = 7.9 Hz, 3H), 7.27 (dd, J = 8.2, 2.0 Hz, 1H), 6.39 (d, J = 1.0 Hz, 1H), 4.07 (s, 3H), 3.44 (s, 6H), 2.35 (s, 3H); m/z N.D. |
| 39 | 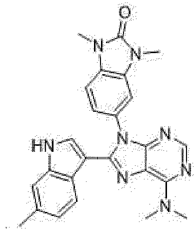 | 5-(6-(dimethylamino)-8-(6-methyl-1H-indol-3-yl)-9H-purin-9-yl)-1,3-dimethyl-1,3-dihydro-2H-benzo[d]imidazol-2-one | ¹H-NMR (500 MHz, Methanol-d₄) δ 8.35 (d, J = 8.2 Hz, 1H), 8.08 (s, 1H), 7.35 (d, J = 8.2 Hz, 1H), 7.31 (d, J = 1.9 Hz, 1H), 7.19 (dd, J = 8.2, 1.9 Hz, 1H), 7.16 (s, 1H), 7.02 (dd, J = 8.3, 1.2 Hz, 1H), 6.47 (s, 1H), 3.68 (s, 6H), 3.52 (s, 3H), 3.41 (s, 3H), 2.44 (s, 3H); m/z N.D. |
| 40 | 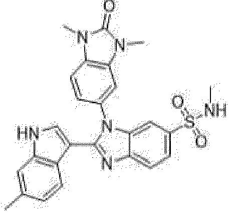 | 3-(1,3-dimethyl-2-oxo-benzimidazol-5-yl)-N-methyl-2-(6-methyl-1H-indol-3-yl)benzimidazole-5-sulfonamide | ¹H-NMR (500 MHz, Methanol-d₄) δ 8.20 (d, J = 7.9 Hz, 1H), 8.04 (s, 1H), 7.92 (d, J = 8.5 Hz, 1H), 7.79 (d, J = 8.2 Hz, 1H), 7.57 (s, 1H), 7.43 (d, J = 8.2 Hz, 1H), 7.37 (s, 1H), 7.30 (d, J = 7.9 Hz, 1H), 7.22 (s, 1H), 7.06 (d, J = 7.9 Hz, 1H), 6.81 (s, 1H), 3.55 (s, 3H), 3.41 (s, 3H), 2.51 (s, 3H), 2.46 (s, 3H); m/z 501 |
| 41 | 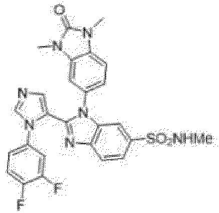 | 2-[3-(3,4-difluorophenyl)imid azol-4-yl]-3-(1,3-dimethyl-2-oxo-benzimidazol-5-yl)-N-methyl-benzimidazole-5-sulfonamide | ¹H-NMR (500 MHz, Chloroform-d) δ 7.81 (d, J = 8.5 Hz, 1H), 7.74-7.72 (m, 1H), 7.69 (d, J = 1.5 Hz, 1H), 7.63 (s, 1H), 7.22 (s, 1H), 7.16-7.10 (m, 1H), 6.97 (d, J = 7.9 Hz, 1H), 6.87-6.81 (m, 2H), 6.76 (dd, J = 8.1, 2.0 Hz, 1H), 6.61 (d, J = 1.8 Hz, 1H), 4.69 (q, J = 5.4 Hz, 1H), 3.48 (s, 3H), 3.34 (s, 3H), 2.59 (d, J = 5.2 Hz, 3H); m/z N.D. |
| 42 | 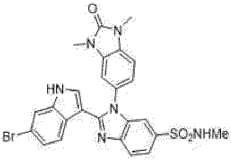 | 2-(6-bromo-1H-indol-3-yl)-3-(1,3-dimethyl-2-oxo-benzimidazol-5-yl)-N-methyl-benzimidazole-5-sulfonamide | ¹H-NMR (400 MHz, Chloroform-d) δ 8.61 (d, J = 8.7 Hz, 1H), 8.25 (s, 1H), 7.96 (d, J = 8.2 Hz, 1H), 7.77 (dd, J = 8.5, 1.6 Hz, 1H), 7.60 (d, J = 1.8 Hz, 1H), 7.55-7.52 (m, 1H), 7.43 (dd, J = 8.7, 1.8 Hz, 1H), 7.19-7.18 (m, 2H), 7.00 (s, 1H), 6.60 (d, J = 3.2 Hz, 1H), 4.26 (q, J = 5.2 Hz, 1H), 3.55 (s, 3H), 3.41 (s, 3H), 2.65 (d, J = 5.5 Hz, 3H); m/z 565, 567 |
| 43 | 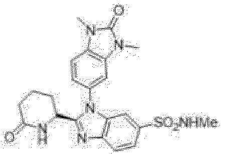 | 3-(1,3-dimethyl-2-oxo-benzimidazol-5-yl)-N-methyl-2-(6-oxo-2-piperidyl)benzimida zole-5-sulfonamide | ¹H-NMR (500 MHz, Chloroform-d) δ 8.07-7.98 (1H), 7.89-7.77 (m, 1H), 7.77-7.68 (m, 1H), 7.63 (d, J = 15.3 Hz, 1H), 7.14 (d, J = 8.2 Hz, 1H), 7.11-7.03 (m, 1H), 6.99 (d, J = 25.6 Hz, 1H), 4.77 (s, 1H), 4.24 (td, J = 7.0, 5.2 Hz, 1H), 3.52 (s, 3H), 3.45 (s, 3H), 2.60 (s, 3H), 2.53-2.27 (m, 2H), 2.21 (m, 1H), 2.12-1.95 (1H), 1.99-1.57 (m, 2H); m/z 469 |
| 44 | 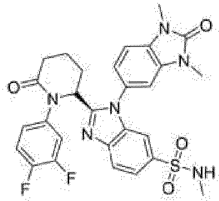 | (S)-2-(1-(3,4-difluorophenyl)-6-oxopiperidin-2-yl)-N,1',3'-trimethyl-2'-oxo-2',3'-dihydro-1'H-[1,5'-bibenzo[d]imidazole ]-6-sulfonamide | ¹H-NMR (500MHz, Chloroform-d) δ 7.95 (d, J = 8.5 Hz, 1H), 7.77 (d, J = 8.2 Hz, 1H), 7.51 (s, 1H), 7.17-7.08 (m, 1H), 7.08-6.93 (m, 3H), 6.93-6.85 (m, 1H), 6.83 (s, 1H), 5.03-4.91 (m, 1H), 4.47-4.34 (m, 1H), 3.62-3.47 (m, 3H), 3.45 (d, J = 6.7 Hz, 1H), 3.32 (s, 2H), 2.86 (dt, J = 17.7, 5.5 Hz, 1H), 2.67-2.59 (m, 3H), 2.50 (m, 1H), 2.29-2.18 (m, 2H), 1.90 (m, 1H), 1.76-1.54 (m, 1H); m/z N.D. |
| 45 | 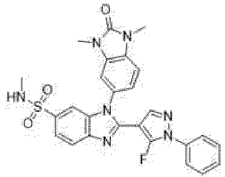 | 2-(5-fluoro-1-phenyl-1H-pyrazol-4-yl)-N,1',3'-trimethyl-2'-oxo-2',3'-dihydro-1'H-[1,5'-bibenzo[d]imidazole ]-6-sulfonamide | ¹H-NMR (500 MHz, Chloroform-d) δ 8.00 (d, J = 8.5 Hz, 1H), 7.82 (d, J = 8.5 Hz, 1H), 7.70 (s, 1H), 7.61 (d, J = 7.9 Hz, 2H), 7.50 (t, J = 7.9 Hz, 2H), 7.41 (t, J = 7.5 Hz, 1H), 7.18 (s, 2H), 7.11 (d, J = 2.1 Hz, 1H), 7.05-6.99 (1H), 4.36 (s, 1H), 3.55 (s, 3H), 3.45 (s, 3H), 2.65 (s, 3H); m/z 532 |
| 46 | 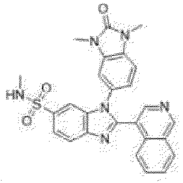 | 3-(1,3-dimethyl-2-oxo-benzimidazol-5-yl)-2-(4-isoquinolyl)-N-methyl-benzimidazole-5-sulfonamide | ¹H-NMR (500 MHz, Chloroform-d) δ 9.40 (s, 1H), 8.54 (s, 1H), 8.43 (d, J = 8.5 Hz, 1H), 8.18 (d, J = 8.2 Hz, 1H), 8.16-8.08 (1H), 8.02 (d, J = 15.3 Hz, 1H), 7.96 (t, J = 7.6 Hz, 1H), 7.91 (s, 1H), 7.89 (s, 1H), 7.85-7.79 (1H), 6.95-6.92 (2H), 6.90 (s, 1H), 3.53-3.37 (3H), 3.37-3.24 (3H), 2.69 (s, 3H); m/z 499 |
| 47 | 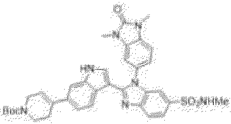 | tert-butyl 4-[3-[1-(1,3-dimethyl-2-oxo-benzimidazol-5-yl)-6-(methylsulfamoyl)be nzimidazol-2-yl]-1H-indol-6-yl]-3,6-dihydro-2H-pyridine-1-carboxylate | ¹H-NMR (500 MHz, Chloroform-d) δ 8.51 (d, J = 8.2 Hz, 1H), 7.81 (s, 1H), 7.67-7.64 (m, 2H), 7.58 (s, 1H), 7.46-7.44 (m, 1H), 7.12-7.09 (m, 2H), 6.97 (s, 1H), 6.65 (s, 1H), 5.97 (s, 1H), 4.97 (s, 1H), 4.03 (s, 2H), 3.59 (m, 2H), 3.47 (s, 3H), 3.32 (s, 3H), 2.58 (d, J = 4.9 Hz, 3H), 2.45 (s, 2H), 1.22 (s, 9H); m/z 668 |
| 48 | 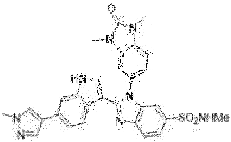 | 3-(1,3-dimethyl-2-oxo-benzimidazol-5-yl)-N-methyl-2-[6-(1-methylpyrazol-4-yl)-1H-indol-3-yl]benzimidazole-5-sulfonamide | ¹H-NMR (500 MHz, Chloroform-d) δ 8.75-8.69 (m, 1H), 8.63-8.57 (m, 1H), 7.92 (d, J = 8.5 Hz, 1H), 7.74-7.71 (m, 2H), 7.60 (d, J = 7.6 Hz, 2H), 7.48-7.38 (m, 2H), 7.18-7.13 (m, 2H), 6.99-6.99 (m, 1H), 6.59 (d, J = 7.9 Hz, 1H), 4.49 (s, 1H), 3.93 (s, 3H), 3.53 (s, 3H), 3.38 (s, 3H), 2.63 (s, 3H); m/z N.D. |
| 49 | 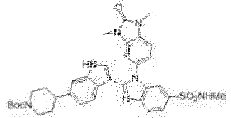 | tert-butyl 4-(3-(1',3'-dimethyl-6-(N-methylsulfamoyl)-2'-oxo-2',3'-dihydro-1'H-[1,5'-bibenzo[d]imidazol]-2-yl)-1H-indol-6-yl)piperidine-1-carboxylate | N.D. m/z 670 |
| 50 | 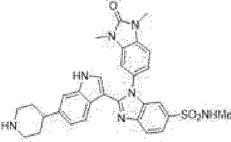 | N,1',3'-trimethyl-2'-oxo-2-(6-(piperidin-4-yl)-1H-indol-3-yl)-2',3'-dihydro-1'H-[1,5'-bibenzo[d]imidazole ]-6-sulfonamide | ¹H-NMR (500 MHz, DMSO-d₆) δ 11.40 (s, 1H), 8.57 (d, J = 8.2 Hz, 1H), 7.90 (d, J = 8.5 Hz, 1H), 7.65 (dd, J = 8.5, 1.5 Hz, 1H), 7.51 (d, J = 1.8 Hz, 1H), 7.47 (d, J = 8.2 Hz, 1H), 7.36 (d, J = 1.5 Hz, 1H), 7.33 (d, J = 5.2 Hz, 1H), 7.29 (dd, J = 8.2, 1.8 Hz, 1H), 7.26 (s, 1H), 7.13 (d, J = 8.5 Hz, 1H), 6.59 (d, J = 3.1 Hz, 1H), 3.47 (s, 3H), 3.38-3.36 (m, 2H), 3.34 (s, 3H), 3.17 (d, J = 5.2 Hz, 1H), 3.03-2.94 (m, 3H), 2.36 (d, J = 5.2 Hz, 3H), 1.98-1.95 (m, 2H), 1.91-1.86 (m, 2H); m/z 570 |
| 51 | 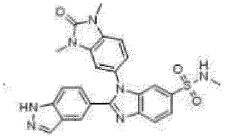 | 3-(1,3-dimethyl-2-oxo-benzimidazol-5-yl)-2-(1H-indazol-5-yl)-N-methyl-benzimidazole-5-sulfonamide | ¹H-NMR (400 MHz, DMSO-d₆) δ 8.07 (s, 1H), 8.03-7.99 (1H), 7.93 (d, J = 8.2 Hz, 1H), 7.67 (dd, J = 8.5, 1.6 Hz, 1H), 7.61 (dd, J = 8.7, 1.4 Hz, 1H), 7.49 (d, J = 8.9 Hz, 1H), 7.47-7.44 (2H), 7.31 (d, J = 8.2 Hz, 1H), 7.17 (dd, J = 8.2, 1.8 Hz, 1H), 3.37 (s, 3H), 3.26 (s, 3H), 2.33 (d, J = 5.0 Hz, 3H); m/z 488 |
| 52 | 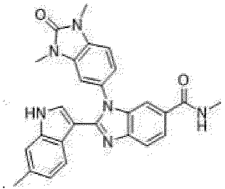 | N,1',3'-trimethyl-2-(6-methyl-1H-indol-3-yl)-2'-oxo-2',3'-dihydro-1'H-[1,5'-bibenzo[d]imidazole ]-6-carboxamide | ¹H-NMR (400 MHz, Chloroform-d) δ 8.59 (d, J = 8.7 Hz, 1H), 8.08 (s, 1H), 7.88 (d, J = 8.7 Hz, 1H), 7.59 (dd, J = 8.2, 1.4 Hz, 1H), 7.51 (s, 1H), 7.23 (s, 1H), 7.18-7.12 (2H), 7.02-6.99 (1H), 6.56 (d, J = 3.0 Hz, 1H), 6.15 (d, J = 4.6 Hz, 1H), 3.49 (d, J = 12.3 Hz, 3H), 3.40-3.33 (3H), 3.03-2.95 (3H), 2.49-2.44 (3H); m/z 465 |
| 53 | 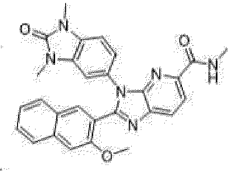 | 3-(1,3-dimethyl-2-oxo-2,3-dihydro-1H-benzo[d]imidazol-5-yl)-2-(3-methoxynaphthalen-2-yl)-N-methyl-3H-imidazo[4,5-b]pyridine-5-carboxamide | ¹H-NMR (400 MHz, DMSO-d₆) δ 8.35 (d, J = 8.2 Hz, 1H), 8.25 (s, 1H), 8.23-8.14(m, 1H), 8.10 (d, J = 8.3 Hz, 1H), 7.94 (d, J = 8.1 Hz, 1H), 7.82 (d, J = 8.1 Hz, 1H), 7.58-7.49 (m, 1H), 7.46-7.35 (m, 2H), 7.28 (s, 1H), 7.07 (d, J = 8.2 Hz, 1H), 6.91 (dd, J = 8.2, 2.0 Hz, 1H), 3.53 (s, 3H), 3.29 (s, 6H), 2.81 (d, J = 4.7 Hz, 3H); m/z 493 |
| 54 | 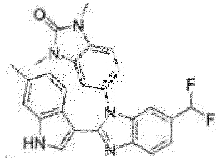 | 5-[6-(difluoromethyl)-2-(6-methyl-1H-indol-3-yl)benzimidazol-1-yl]-1,3-dimethyl-benzimidazol-2-one | ¹H-NMR (500 MHz, Chloroform-d) δ 8.50 (d, 1H), 7.93 (d, 1H), 7.72 (d, 1H), 7.12 (m, 3H), 6.82 (m, 3H), 6.67 (m, 1H), 3.54 (s, 3H), 3.39 (s, 3H), 2.15 (s, 3H); m/z 458 |
| 55 | 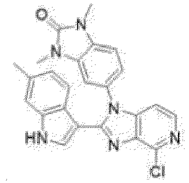 | 5-(4-chloro-2-(6-methyl-1H-indol-3-yl)-1H-imidazo[4,5-c]pyridin-1-yl)-1,3-dimethyl-1,3-dihydro-2H-benzo[d]imidazol-2-one | ¹H-NMR (400MHz, DMSO-d₆) δ 11.30 (s, 1H), 8.53 (d, 1H), 8.07 (s, 1H), 7.54 (d, 1H), 7.36 (m, 1H), 7.29 (m, 2H), 7.09 (m, 2H), 6.54 (brs, 1H), 3.46 (s, 3H), 3.35 (s, 3H), 2.43 (s, 3H); m/z 443 |
| 56 | 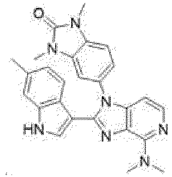 | 5-[4-(dimethylamino)-2-(6-methyl-1H-indol-3-yl)imidazo[4,5-c]pyridin-1-yl]-1,3-dimethyl-benzimidazol-2-one | ¹H-NMR (400MHz, DMSO-d₆) δ 11.06 (s, 1H), 8.42 (d, 1H), 7.78 (d, 1H), 7.37 (m, 2H), 7.19 (m, 1H), 7.13 (m, 1H), 7.03 (d, 1H), 6.41 (m, 2H), 3.50 (s, 6H), 3.45 (s, 3H), 3.33 (s, 3H), 2.41 (s, 3H); m/z 452 |
| 57 | 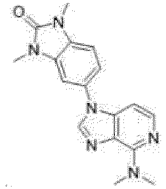 | 5-[4-(dimethylamino)imi dazo[4,5-c]pyridin-1-yl]-1,3-dimethyl-benzimidazol-2-one | ¹H-NMR (500 MHz, Chloroform-d) δ 8.00 (d, 1H), 7.93 (s, 1H), 7.28 (s, 1H), 7.17 (m, 1H), 7.13 (m, 1H), 7.06 (s, 1H), 6.70 (d, 1H), 3.58 (s, 6H), 3.51 (s, 3H), 3.48 (s, 3H); m/z 323 |
| 58 | 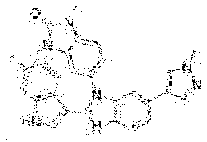 | 1,3-dimethyl-5-[2-(6-methyl-1H-indol-3-yl)-6-(1-methylpyrazol-4-yl)benzimidazol-1-yl]benzimidazol-2-one | ¹H-NMR (400MHz, DMSO-d₆) δ 11.10 (s, 1H), 8.51 (d, 1H), 8.06 (d, 1H), 7.78 (s, 1H), 7.70 (d, 1H), 7.47 (m, 3H), 7.23 (m, 2H), 7.09 (s, 1H), 7.02 (d, 1H), 6.46 (d, 1H), 3.81 (s, 3H), 3.47 (s, 3H), 3.36 (s, 3H), 2.42 (s, 3H); m/z 488 |
| 59 | 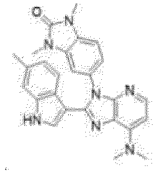 | 5-[7-(dimethylamino)-2-(6-methyl-1H-indol-3-yl)imidazo[4,5-b]pyridin-3-yl]-1,3-dimethyl-benzimidazol-2-one | ¹H-NMR (500 MHz, DMSO-d₆) δ 11.06 (s, 1H), 8.42 (d, 1H), 7.78 (d, 1H), 7.37 (m, 2H), 7.19 (m, 1H), 7.13 (m, 1H), 7.03 (d, 1H), 6.41 (m, 2H), 3.50 (s, 6H), 3.45 (s, 3H), 3.33 (s, 3H), 2.41 (s, 3H); m/z 452 |
| 60 | 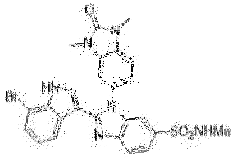 | 2-(7-bromo-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.64 (s, 1H), 8.66 (d, J = 7.9 Hz, 1H), 7.95 (d, J = 8.2 Hz, 1H), 7.67 (dd, J = 8.4, 1.7 Hz, 1H), 7.55 (d, J = 1.8 Hz, 1H), 7.50 (d, J = 8.2 Hz, 1H), 7.47 (d, J = 7.6 Hz, 1H), 7.40 (d, J = 1.5 Hz, 1H), 7.35-7.33 (m, 2H), 7.21-7.18 (m, 1H), 6.61 (s, 1H), 3.47 (s, 3H), 3.34 (s, 3H), 2.37 (d, J = 4.9 Hz, 3H); m/z N.D. |
| 61 | 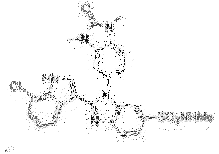 | 2-(7-chloro-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.81 (s, 1H), 8.62 (d, J = 7.9 Hz, 1H), 7.95 (d, J = 8.2 Hz, 1H), 7.68-7.66 (m, 1H), 7.55 (d, J = 1.5 Hz, 1H), 7.49 (d, J = 8.2 Hz, 1H), 7.40 (s, 1H), 7.35-7.32 (m, 3H), 7.27-7.24 (m, 1H), 6.62 (s, 1H), 3.47 (s, 3H), 3.34 (s, 3H), 2.37 (d, J = 4.9 Hz, 3H); m/z N.D. |
| 62 | 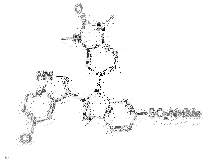 | 2-(5-chloro-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.56 (s, 1H), 8.68-8.68 (m, 1H), 7.97 (d, J = 8.5 Hz, 1H), 7.67-7.65 (m, 1H), 7.54 (d, J = 2.1 Hz, 1H), 7.49 (d, J = 7.0 Hz, 1H), 7.47 (d, J = 6.4 Hz, 1H), 7.37 (d, J = 1.5 Hz, 1H), 7.32-7.30 (m, 2H), 7.24 (dd, J = 8.9, 2.1 Hz, 1H), 6.68 (s, 1H), 3.47 (s, 3H), 3.34 (s, 3H), 2.37-2.36 (m, 3H); m/z N.D. |
| 63 | 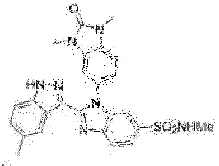 | 3-(1,3-dimethyl-2-oxo-benzimidazol-5-yl)-N-methyl-2-(5-methyl-1H-indazol-3-yl)benzimidazole-5-sulfonamide | ¹H-NMR (500 MHz, Chloroform-d) δ 8.41 (s, 1H), 8.06 (d, J = 8.5 Hz, 1H), 7.82 (d, J = 8.5 Hz, 1H), 7.71 (s, 1H), 7.39-7.37 (m, 2H), 7.32-7.30 (m, 2H), 7.17-7.15 (m, 1H), 7.11-7.09 (m, 1H), 7.02 (s, 1H), 4.29 (q, J = 5.5 Hz, 1H), 3.52 (s, 3H), 3.40 (s, 3H), 2.66 (d, J = 5.2 Hz, 3H), 2.56 (s, 3H); m/z N.D. |
| 64 | 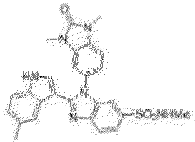 | 3-(1,3-dimethyl-2-oxo-benzimidazol-5-yl)-N-methyl-2-(5-methyl-1H-indol-3-yl)benzimidazole-5-sulfonamide | ¹H-NMR (500 MHz, Chloroform-d) δ 8.50 (s, 1H), 8.16 (s, 1H), 7.99 (d, J = 8.7 Hz, 1H), 7.77 (d, J = 8.2 Hz, 1H), 7.60 (s, 1H), 7.28 (s, 1H), 7.21-7.13 (m, 3H), 6.99 (s, 1H), 6.60 (d, J = 2.7 Hz, 1H), 4.24 (q, J = 5.3 Hz, 1H), 3.55 (s, 3H), 3.40 (s, 3H), 2.65 (d, J = 5.2 Hz, 3H), 2.55 (s, 3H); m/z N.D. |
| 65 | 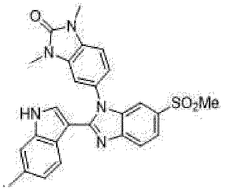 | 1,3-dimethyl-5-[2-(6-methyl-1H-indol-3-yl)-6-methylsulfonyl-benzimidazol-1-yl]benzimidazol-2-one | ¹H-NMR (400 MHz, DMSO-d₆) δ 11.28 (s, 1H), 8.53 (d, J = 8.7 Hz, 1H), 7.98-7.93 (1H), 7.78 (d, J = 8.7 Hz, 1H), 7.54-7.50 (1H), 7.48 (d, J = 8.2 Hz, 1H), 7.45-7.42 (1H), 7.29 (d, J = 6.4 Hz, 1H), 7.23 (s, 1H), 7.09-7.04 (1H), 6.54 (d, J = 2.7 Hz, 1H), 3.34 (s, 6H), 3.29 (s, 3H), 2.44-2.41 (3H); m/z N.D. |
| 66 | 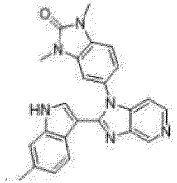 | 1,3-dimethyl-5-[2-(6-methyl-1H-indol-3-yl)imidazo[4,5-c]pyridin-1-yl]benzimidazol-2-one | ¹H-NMR (500 MHz, Chloroform-d) δ 9.06 (s, 1H), 8.83 (s, 1H), 8.51 (d, J = 8.5 Hz, 1H), 8.23 (d, J = 4.9 Hz, 1H), 7.19-7.10 (m, 4H), 7.07 (s, 1H), 6.88 (d, J = 5.2 Hz, 1H), 6.46 (s, 1H), 3.53 (s, 3H), 3.44 (s, 3H), 2.47 (s, 3H); m/z 409 |
| 67 | 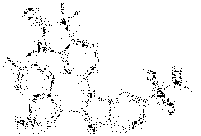 | N-methyl-2-(6-methyl-1H-indol-3-yl)-1-(1,3,3-trimethyl-2-oxoindolin-6-yl)-1H-benzo[d]imidazole-6-sulfonamide | ¹H-NMR (500 MHz, DMSO-d₆) δ 11.37 (s, 1H), 8.48 (d, 1H), 7.93 (d, 1H), 7.71 (m, 2H), 7.44 (s, 1H), 7.38 (m, 2H), 7.28 (m, 1H), 7.23 (d, 1H), 7.07 (d, 1H), 6.62 (brs, 1H), 3.13 (s, 3H), 2.43 (s, 3H), 2.34 (d, 3H), 1.43 (s, 6H); m/z 514 |
| 68 | 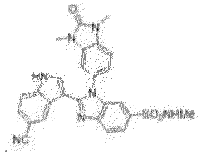 | 2-(5-cyano-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.85 (s, 1H), 9.07 (s, 1H), 8.01 (d, J = 8.5 Hz, 1H), 7.69-7.65 (m, 2H), 7.61-7.59 (m, 1H), 7.56 (d, J = 2.1 Hz, 1H), 7.48 (d, J = 8.2 Hz, 1H), 7.39 (d, J = 1.2 Hz, 1H), 7.37-7.35 (m, 1H), 7.33 (dd, J = 8.2, 1.8 Hz, 1H), 6.82 (s, 1H), 3.47 (s, 3H), 3.34 (s, 3H), 2.37 (d, J = 4.9 Hz, 3H); m/z N.D. |
| 69 | 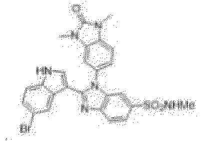 | 2-(5-bromo-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.59 (s, 1H), 8.81 (d, J = 1.8 Hz, 1H), 7.98 (d, J = 8.5 Hz, 1H), 7.66 (dd, J = 8.2, 1.5 Hz, 1H), 7.54 (d, J = 1.8 Hz, 1H), 7.48-7.43 (m, 2H), 7.37-7.30 (m, 4H), 6.67 (s, 1H), 3.47 (s, 3H), 3.34 (s, 3H), 2.37 (s, 3H); m/z N.D. |
| 70 | 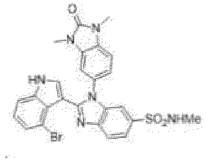 | 2-(4-bromo-1H-indol-3-yl)-3-(1,3-dimethyl-2-oxo-benzimidazol-5-yl)-N-methyl-benzimidazole-5-sulfonamide | ¹H-NMR (500 MHz, DMSO-d₆) δ 11.83 (s, 1H), 7.96 (d, J = 8.5 Hz, 1H), 7.83 (s, 1H), 7.71 (dd, J = 8.4, 1.7 Hz, 1H), 7.59 (d, J = 1.5 Hz, 1H), 7.44 (d, J = 7.9 Hz, 1H), 7.38 (s, 1H), 7.35 (d, J = 1.5 Hz, 1H), 7.20-7.14 (m, 3H), 7.03 (t, J = 7.9 Hz, 1H), 3.28 (s, 3H), 3.22 (s, 3H), 2.39 (s, 3H); m/z N.D. |
| 71 | 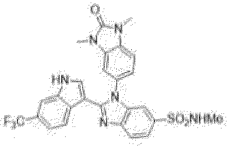 | 3-(1,3-dimethyl-2-oxo-benzimidazol-5-yl)-N-methyl-2-[6-(trifluoromethyl)-1H-indol-3-yl]benzimidazole-5-sulfonamide | ¹H-NMR (500 MHz, DMSO-d₆) δ 11.72 (s, 1H), 8.84 (d, J = 8.5 Hz, 1H), 7.96 (d, J = 8.5 Hz, 1H), 7.84 (s, 1H), 7.67 (dd, J = 8.4, 1.7 Hz, 1H), 7.56-7.54 (m, 2H), 7.49-7.47 (m, 1H), 7.39 (d, J = 1.5 Hz, 1H), 7.36-7.31 (m, 2H), 6.86 (s, 1H), 3.47 (s, 3H), 3.34 (s, 3H), 2.37 (d, J = 4.9 Hz, 3H); m/z N.D |
| 72 | 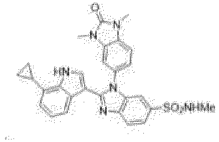 | 2-(7-cyclopropyl-1H-indol-3-yl)-3-(1,3-dimethyl-2-oxo-benzimidazol-5-yl)-N-methyl-benzimidazole-5-sulfonamide | ¹H-NMR (400 MHz, Chloroform-d) δ 8.54 (d, J = 8.2 Hz, 1H), 8.48 (s, 1H), 7.96 (d, J = 8.7 Hz, 1H), 7.75 (dd, J = 8.5, 1.6 Hz, 1H), 7.59 (d, J = 1.8 Hz, 1H), 7.20 (d, J = 1.8 Hz, 1H), 7.16 (s, 1H), 7.05 (d, J = 7.3 Hz, 1H), 6.99-6.99 (m, 1H), 6.64 (d, J = 2.7 Hz, 1H), 4.24 (q, J = 5.0 Hz, 1H), 3.54 (s, 3H), 3.40 (s, 3H), 2.64 (d, J = 5.5 Hz, 3H), 2.01-1.96 (m, 1H), 0.96-0.92 (m, 2H), 0.76-0.72 (m, 2H); m/z 527 |
| 73 | 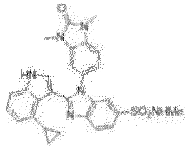 | 2-(4-cyclopropyl-1H-indol-3-yl)-3-(1,3-dimethyl-2-oxo-benzimidazol-5-yl)-N-methyl-benzimidazole-5-sulfonamide | ¹H-NMR (500 MHz, Chloroform-d) δ 8.32 (s, 1H), 7.95 (d, J = 8.2 Hz, 1H), 7.80-7.79 (m, 2H), 7.21 (d, J = 7.6 Hz, 1H), 7.18-7.15 (m, 1H), 7.10-7.07 (m, 1H), 6.98-6.97 (m, 1H), 6.90 (s, 1H), 6.81 (d, J = 7.0 Hz, 1H), 4.28 (q, J = 5.5 Hz, 1H), 3.43 (s, 3H), 3.27 (s, 3H), 2.68 (d, J = 5.5 Hz, 3H), 2.41-2.38 (m, 1H), 0.89-0.87 (m, 2H), 0.66-0.64 (m, 2H); m/z 527 |
| 74 | 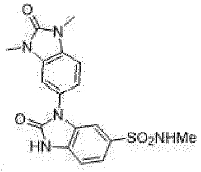 | N,1',3'-trimethyl-2,2'-dioxo-2,2',3,3'-tetrahydro-1'H-[1,5'-bibenzo[d]imidazole ]-6-sulfonamide | ¹H-NMR (500 MHz, DMSO-d₆) δ 8.74 (s, 1H), 7.98 (d, 1H), 7.89 (d, 1H), 7.72 (d, 2H), 7.58 (d, 1H), 7.72 (m, 2H), 7.38 (d, 1H), 3.43 (s, 3H), 3.39 (s, 3H), 2.38 (d, 3H); m/z 388 |
| 75 | 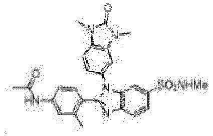 | N-[4-[1-(1,3-dimethyl-2-oxo-benzimidazol-5-yl)-6-(methylsulfamoyl)be nzimidazol-2-yl]-3-methyl-phenyl]acetamide | ¹H-NMR (400 MHz, Chloroform-d) δ 7.99 (d, 1H), 7.84 (m, 2H), 7.48 (s, 1H), 7.34 (m, 1H), 7.23 (m, 2H), 6.99 (m 2H), 6.77 (s, 1H), 3.47 (s, 3H), 3.35 (s, 3H), 2.29 (s, 3H), 2.19 (s, 3H), 1.28 (s, 3H); m/z 519 |
| 76 | 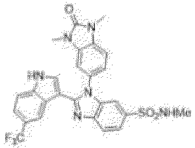 | 3-(1,3-dimethyl-2-oxo-benzimidazol-5-yl)-N-methyl-2-[5-(trifluoromethyl)-1H-indol-3-yl]benzimidazole-5-sulfonamide | ¹H-NMR (500 MHz, DMSO-d₆) δ 11.80 (s, 1H), 8.97 (s, 1H), 8.00 (d, J = 8.2 Hz, 1H), 7.68 (s, 1H), 7.66 (s, 1H), 7.56 (s, 1H), 7.53 (d, J = 7.0 Hz, 1H), 7.47 (d, J = 7.9 Hz, 1H), 7.40 (s, 1H), 7.36-7.34 (m, 1H), 7.32 (dd, J = 8.2, 1.8 Hz, 1H), 6.84 (s, 1H), 3.46 (s, 3H), 3.34 (s, 3H), 2.37 (d, J = 4.3 Hz, 3H); m/z N.D. |
| 77 | 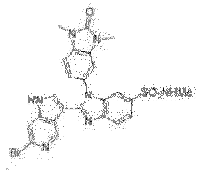 | 2-(6-bromo-1H-pyrrolo[3,2-c]pyridin-3-yl)-N,1',3'-trimethyl-2'-oxo-2',3'-dihydro-1'H-[1,5'-bibenzo[d]imidazole ]-6-sulfonamide | ¹H-NMR (500 MHz, DMSO-d₆) δ 9.48 (s, 1H), 7.94 (d, J = 6.1 Hz, 1H), 7.67-7.63 (m, 2H), 7.54 (s, 1H), 7.47 (d, J = 7.6 Hz, 1H), 7.37-7.30 (m, 3H), 6.74 (s, 1H), 3.46 (s, 3H), 3.34 (s, 3H), 2.36 (d, J = 4.0 Hz, 3H); m/z 566, 568 |
| 78 | 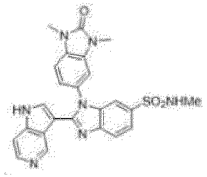 | 3-(1,3-dimethyl-2-oxo-benzimidazol-5-yl)-N-methyl-2-(1H-pyrrolo[3,2-c]pyridin-3-yl)benzimidazole-5-sulfonamide | ¹H-NMR (400 MHz, Methanol-d₄) δ 9.53 (s, 1H), 8.26 (d, J = 5.9 Hz, 1H), 7.96 (d, J = 8.7 Hz, 1H), 7.81-7.79 (m, 1H), 7.60 (d, J = 1.4 Hz, 1H), 7.48 (d, J = 5.5 Hz, 1H), 7.44-7.42 (m, 2H), 7.32-7.29 (m, 1H), 7.00 (s, 1H), 3.54 (s, 3H), 3.41 (s, 3H), 2.50 (s, 3H); m/z N.D. |
| 79 | 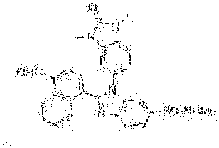 | 3-(1,3-dimethyl-2-oxo-benzimidazol-5-yl)-2-(4-formyl-1-naphthyl)-N-methyl-benzimidazole-5-sulfonamide | ¹H-NMR (500 MHz, Chloroform-d) δ 10.41 (s, 1H), 9.26 (d, J = 8.5 Hz, 1H), 8.18 (d, J = 8.5 Hz, 1H), 8.05 (d, J = 8.2 Hz, 1H), 7.93-7.87 (m, 3H), 7.74-7.71 (m, 1H), 7.64-7.61 (m, 2H), 6.92-6.85 (m, 2H), 6.82 (s, 1H), 4.53-4.51 (m, 1H), 3.37 (s, 3H), 3.25 (s, 3H), 2.68 (d, J = 5.2 Hz, 3H); m/z 526 |
| 80 | 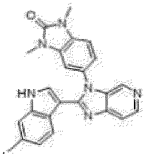 | 1,3-dimethyl-5-[2-(6-methyl-1H-indol-3-yl)imidazo[4,5-c]pyridin-3-yl]benzimidazol-2-one | N.D. m/z 409 |
| 81 | 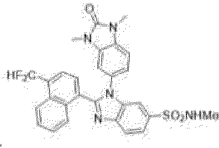 | 2-(4-(difluoromethyl)nap hthalen-1-yl)-N,1',3'-trimethyl-2'-oxo-2',3'-dihydro-1'H-[1,5'-bibenzo[d]imidazole ]-6-sulfonamide | ¹H-NMR (500 MHz, Chloroform-d) δ 8.17 (d, J = 8.2 Hz, 2H), 8.05 (d, J = 8.2 Hz, 1H), 7.91 (s, 1H), 7.87 (d, J = 8.5 Hz, 1H), 7.65-7.57 (m, 3H), 7.49 (d, J = 7.3 Hz, 1H), 7.09 (t, J = 55.5 Hz, 1H), 6.94-6.87 (m, 2H), 6.78 (s, 1H), 4.48-4.46 (m, 1H), 3.39 (s, 3H), 3.24 (s, 3H), 2.68 (d, J = 5.2 Hz, 3H); m/z 548 |
| 82 | 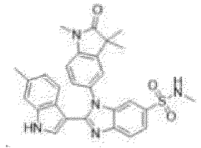 | N-methyl-2-(6-methyl-1H-indol-3-yl)-3-(1,3,3-trimethyl-2-oxo-indolin-5-yl)benzimidazole-5-sulfonamide | ¹H-NMR (500 MHz, DMSO-d₆) δ 11.33 (s, 1H), 8.47 (d, 1H), 7.92 (d, 1H), 7.67 (m, 2H), 7.55 (d, 1H), 7.40 (m, 3H), 7.24 (m, 1H), 7.06 (d, 1H), 6.60 (d, 1H), 3.27 (s, 3H), 3.43 (s, 3H), 2.39 (d, 3H), 1.32 (s, 6H); m/z 514 |
| 83 | 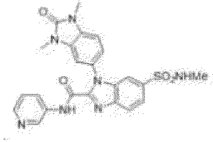 | 1',3'-dimethyl-6-(N-methylsulfamoyl)-2'-oxo-N-(pyridin-3-yl)-2',3'-dihydro-1'H-[1,5'-bibenzo[d]imidazole ]-2-carboxamide | N.D; m/z 492 |
| 84 | 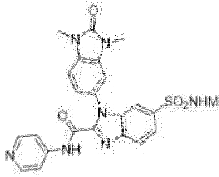 | 1-(1,3-dimethyl-2-oxo-benzimidazol-5-yl)-6-(methylsulfamoyl)-N-(4-pyridyl)benzimidazo le-2-carboxamide | N.D; m/z 492 |
| 85 | 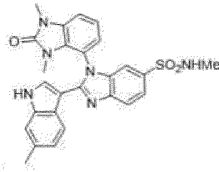 | 3-(1,3-dimethyl-2-oxo-benzimidazol-4-yl)-N-methyl-2-(6-methyl-1H-indol-3-yl)benzimidazole-5-sulfonamide | ¹H-NMR (400 MHz, Chloroform-d) δ 8.61 (d, J = 8.2 Hz, 1H), 8.41 (s, 1H), 7.97 (d, J = 8.2 Hz, 1H), 7.78 (dd, J = 8.2, 1.8 Hz, 1H), 7.50 (d, J = 1.4 Hz, 1H), 7.31-7.27 (m, 1H), 7.21-7.11 (m, 4H), 6.51 (d, J = 3.2 Hz, 1H), 4.46 (q, J = 5.3 Hz, 1H), 3.48 (s, 3H), 2.61 (d, J = 5.5 Hz, 3H), 2.60 (s, 3H), 2.46 (s, 3H); m/z N.D. |
| 86 | 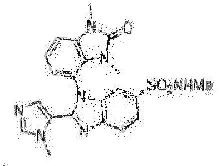 | 3-(1,3-dimethyl-2-oxo-benzimidazol-4-yl)-N-methyl-2-(3-methylimidazol-4-yl)benzimidazole-5-sulfonamide | ¹H-NMR (400 MHz, Chloroform-d) δ 7.94 (d, J = 8.2 Hz, 1H), 7.81 (dd, J = 8.2, 1.8 Hz, 1H), 7.56-7.55 (m, 2H), 7.31-7.27 (m, 1H), 7.22 (dd, J = 8.0, 1.1 Hz, 1H), 7.04 (dd, J = 8.0, 1.1 Hz, 1H), 6.48 (s, 1H), 4.38 (q, J = 5.5 Hz, 1H), 4.21 (s, 3H), 3.51 (s, 3H), 2.63 (d, J = 5.5 Hz, 3H), 2.61 (s, 3H); m/z N.D. |
| 87 | 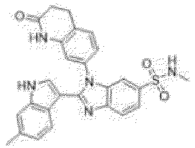 | N-methyl-2-(6-methyl-1H-indol-3-yl)-3-(2-oxo-3,4-dihydro-1H-quinolin-7-yl)benzimidazole-5-sulfonamide | ¹H-NMR (500 MHz, Chloroform-d) δ 8.54 (s, 1H), 8.40 (d, J = 8.2 Hz, 1H), 8.28 (s, 1H), 8.01-7.93 (1H), 7.74 (d, J = 8.5 Hz, 1H), 7.56 (s, 1H), 7.29 (d, J = 7.9 Hz, 1H), 7.11 (d, J = 8.2 Hz, 1H), 7.05 (s, 1H), 6.93 (d, J = 7.9 Hz, 1H), 6.67 (s, 1H), 6.48 (s, 1H), 4.59-4.47 (1H), 3.08 (t, J = 7.5 Hz, 2H), 2.74 (t, J = 7.5 Hz, 2H), 2.62 (d, J = 5.5 Hz, 3H), 2.43 (s, 3H); m/z 486 |
| 88 | 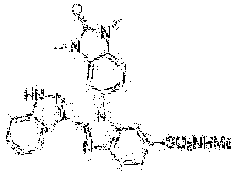 | 3-(1,3-dimethyl-2-oxo-benzimidazol-5-yl)-2-(1H-indazol-3-yl)-N-methyl-benzimidazole-5-sulfonamide | ¹H-NMR (500 MHz, DMSO-d₆) δ 13.53 (s, 1H), 8.57 (d, J = 8.2 Hz, 1H), 8.05 (d, J = 8.7 Hz, 1H), 7.73 (d, J = 8.5 Hz, 1H), 7.57 (d, J = 8.2 Hz, 1H), 7.50-7.44 (m, 3H), 7.42-7.39 (m, 1H), 7.37-7.32 (m, 2H), 7.26 (dd, J = 8.1, 1.7 Hz, 1H), 3.44 (s, 3H), 2.38 (d, J = 4.0 Hz, 3H); m/z N.D. |
| 89 | 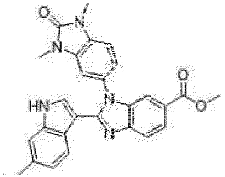 | methyl 3-(1,3-dimethyl-2-oxo-benzimidazol-5-yl)-2-(6-methyl-1H-indol-3-yl)benzimidazole-5-carboxylate | ¹H-NMR (500 MHz, DMSO-d₆) δ 11.24 (s, 1H), 8.53 (d, 1H), 7.90 (m, 1H), 7.84 (d, 1H), 7.53 (s, 1H), 7.47 (d, 2H), 7.28 (d, 1H), 7.23 (d, 1H), 7.07 (d, 1H), 6.54 (s, 1H), 3.93 (s, 3H), 3.81 (s, 3H), 3.48 (s, 3H), 2.43 (s, 3H); m/z 466 |
| 90 | 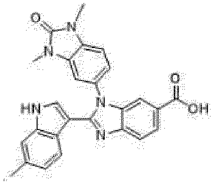 | 3-(1,3-dimethyl-2-oxo-benzimidazol-5-yl)-2-(6-methyl-1H-indol-3-yl)benzimidazole-5-carboxylic acid | ¹H-NMR (500MHz, DMSO-d₆) δ 11.22 (s, 1H), 8.51 (d, J = 7.9 Hz, 1H), 7.87 (d, J = 7.9 Hz, 1H), 7.77 (d, J = 8.2 Hz, 1H), 7.53 (s, 1H), 7.49 (s, 1H), 7.45 (d, J = 8.2 Hz, 1H), 7.26 (d, J = 8.2 Hz, 1H), 7.22 (s, 1H), 7.05 (d, J = 8.2 Hz, 1H), 6.54 (d, J = 2.7 Hz, 1H), 3.46 (s, 3H), 3.34 (s, 3H), 2.42 (s, 3H); m/z 452 |
| 91 | 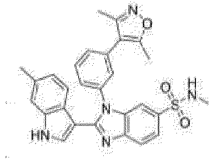 | 3-[3-(3,5-dimethylisoxazol-4-yl)phenyl]-N-methyl-2-(6-methyl-1H-indol-3-yl)benzimidazole-5-sulfonamide | ¹H-NMR (Chloroform-d) δ 8.73 (s, 1H), 8.01-7.95 (1H), 7.93 (d, J = 8.5 Hz, 1H), 7.78 (s, 1H), 7.77-7.73 (1H), 7.69 (t, J = 7.9 Hz, 1H), 7.53 (d, J = 7.9 Hz, 1H), 7.43 (d, J = 7.9 Hz, 1H), 7.19 (s, 1H), 7.12 (s, 1H), 7.00 (d, J = 8.2 Hz, 1H), 6.92 (d, J = 2.7 Hz, 1H), 4.54 (q, J = 5.4 Hz, 1H), 2.65 (d, J = 5.2 Hz, 3H), 2.42 (s, 3H), 2.23 (s, 3H), 2.05 (s, 3H); m/z 512 |
| 92 | 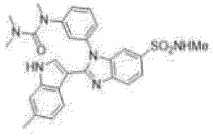 | 1,1,3-trimethyl-3-[3-[2-(6-methyl-1H-indol-3-yl)-6-(methylsulfamoyl)be nzimidazol-1-yl]phenyl]urea | ¹H-NMR (500 MHz, Methanol-d₄) δ 7.88 (d, J = 8.2 Hz, 1H), 7.81-7.78 (m, 2H), 7.67-7.63 (m, 2H), 7.37 (d, J = 7.9 Hz, 1H), 7.28 (dd, J = 8.2, 1.8 Hz, 1H), 7.21 (s, 1H), 7.12 (s, 1H), 7.03 (t, J = 1.8 Hz, 1H), 6.94 (d, J = 8.2 Hz, 1H), 3.08 (s, 3H), 2.54 (s, 6H), 2.50 (s, 3H), 2.41 (s, 3H); m/z 517 |
| 93 | 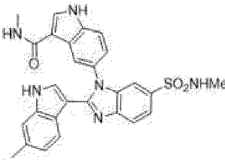 | N-methyl-5-[2-(6-methyl-1H-indol-3-yl)-6-(methylsulfamoyl)be nzimidazol-1-yl]-1H-indole-3-carboxamide | ¹H-NMR (500 MHz, Methanol-d₄) δ 8.29 (d, J = 1.8 Hz, 1H), 8.22 (d, J = 8.2 Hz, 1H), 8.05 (s, 1H), 7.90 (d, J = 8.2 Hz, 1H), 7.77 (dd, J = 8.5, 1.5 Hz, 1H), 7.70 (d, J = 8.5 Hz, 1H), 7.55 (d, J = 1.2 Hz, 1H), 7.24 (dd, J = 8.5, 2.1 Hz, 1H), 7.17 (s, 1H), 7.04 (d, J = 8.2 Hz, 1H), 6.70 (s, 1H), 2.87 (s, 3H), 2.49 (s, 3H), 2.44 (s, 3H); m/z 513 |
| 94 | 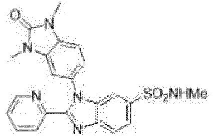 | 3-(1,3-dimethyl-2-oxo-benzimidazol-5-yl)-N-methyl-2-(2-pyridyl)benzimidazo le-5-sulfonamide | ¹H-NMR (500 MHz, DMSO-d₆) δ 8.38 (d, J = 4.9 Hz, 1H), 8.21 (d, J = 7.9 Hz, 1H), 8.03 (d, J = 8.2 Hz, 1H), 7.96 (td, J = 7.7, 1.6 Hz, 1H), 7.74 (dd, J = 8.5, 1.5 Hz, 1H), 7.55 (d, J = 1.5 Hz, 1H), 7.42-7.39 (m, 3H), 7.29 (d, J = 8.2 Hz, 1H), 7.13 (dd, J = 8.2, 1.8 Hz, 1H), 3.40 (s, 3H), 3.29 (s, 3H), 2.37 (d, J = 2.7 Hz, 3H); m/z 449 |
| 95 | 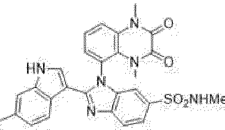 | 3-(1,4-dimethyl-2,3-dioxo-quinoxalin-5-yl)-N-methyl-2-(6-methyl-1H-indol-3-yl)benzimidazole-5-sulfonamide | ¹H-NMR (500 MHz, Methanol-d₄) δ 8.11 (d, J = 8.2 Hz, 1H), 7.91 (d, J = 8.2 Hz, 1H), 7.78-7.71 (m, 3H), 7.66 (dd, J = 7.3, 1.5 Hz, 1H), 7.45 (d, J = 1.5 Hz, 1H), 7.17 (s, 1H), 7.02 (d, J = 8.2 Hz, 1H), 6.81 (s, 1H), 3.77 (s, 3H), 3.40 (s, 3H), 2.46 (s, 3H), 2.43 (s, 3H); m/z N.D. |
| 96 | 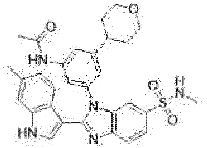 | N-[3-[2-(6-methyl-1H-indol-3-yl)-6-(methylsulfamoyl)be nzimidazol-1-yl]-5-tetrahydropyran-4-yl-phenyl]acetamide | ¹H-NMR (500 MHz, Chloroform-d) δ 8.58 (s, 1H), 8.35 (d, J = 8.2 Hz, 1H), 8.32 (s, 1H), 7.87 (d, J = 8.5 Hz, 1H), 7.77-7.71 (2H), 7.57 (s, 1H), 7.16 (s, 1H), 7.10 (d, J = 8.5 Hz, 1H), 7.07-7.02 (1H), 6.81 (s, 1H), 6.37 (d, J = 2.4 Hz, 1H), 4.63-4.54 (1H), 4.04-3.90 (m, 2H), 3.42 (td, J = 11.4, 2.7 Hz, 2H), 2.77-2.64 (m, 1H), 2.65-2.61 (3H), 2.44 (s, 3H), 2.28 (s, 3H), 1.69-1.61 (m, 4H); m/z 558 |
| 97 | 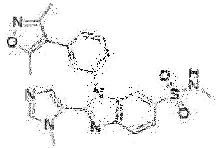 | 3-[3-(3,5-dimethylisoxazol-4-yl)phenyl]-N-methyl-2-(3-methylimidazol-4-yl)benzimidazole-5-sulfonamide | ¹H-NMR (500 MHz, Chloroform-d) δ 7.93 (d, J = 8.5 Hz, 1H), 7.80 (dd, J = 8.5, 1.5 Hz, 1H), 7.76-7.71 (m, 2H), 7.60 (s, 1H), 7.53 (d, J = 7.9 Hz, 1H), 7.42 (d, J = 7.9 Hz, 1H), 7.22 (s, 1H), 6.57 (s, 1H) 4.37 (q, J = 5.3 Hz, 1H), 4.20 (d, J = 12.2 Hz, 3H), 2.66 (d, J = 5.5 Hz, 3H), 2.41 (s, 3H), 2.26 (s, 3H); m/z 463 |
| 98 | 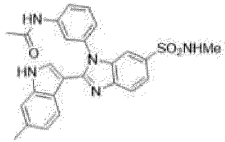 | N-[3-[2-(6-methyl-1H-indol-3-yl)-6-(methylsulfamoyl)be nzimidazol-1-yl]phenyl]acetamide | ¹H-NMR (500 MHz, Methanol-d₄) δ 8.08 (d, J = 8.2 Hz, 1H), 7.89 (d, J = 8.2 Hz, 1H), 7.81-7.77 (m, 3H), 7.62 (s, 1H), 7.59 (t, J = 7.9 Hz, 1H), 7.22-7.21 (m, 2H), 7.02 (d, J = 8.2 Hz, 1H), 6.91 (s, 1H), 2.50 (s, 3H), 2.44 (s, 3H), 2.12 (s, 3H); m/z N.D. |
| 99 | 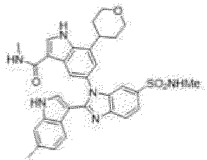 | N-methyl-5-[2-(6-methyl-1H-indol-3-yl)-6-(methylsulfamoyl)be nzimidazol-1-yl]-7-tetrahydropyran-4-yl-1H-indole-3-carboxamide | ¹H-NMR (500 MHz, Methanol-d₄) δ 8.22 (d, J = 1.8 Hz, 1H), 8.13 (d, J = 8.2 Hz, 1H), 8.07 (s, 1H), 7.92 (d, J = 8.5 Hz, 1H), 7.79 (dd, J = 8.5, 1.5 Hz, 1H), 7.63 (d, J = 1.2 Hz, 1H), 7.19 (s, 1H), 7.10 (s, 1H), 7.02 (d, J = 8.2 Hz, 1H), 6.75 (s, 1H), 4.02 (d, J = 10.7 Hz, 2H), 3.65 (td, J = 11.7, 2.2 Hz, 2H), 3.36 (m, 1H), 2.90 (s, 3H), 2.52 (s, 3H), 2.45 (s, 3H), 1.81 (s, 4H); m/z N.D. |
| 100 | 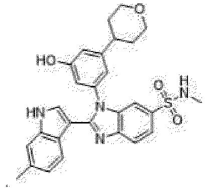 | 3-(3-hydroxy-5-tetrahydropyran-4-yl-phenyl)-N-methyl-2-(6-methyl-1H-indol-3-yl)benzimidazole-5-sulfonamide | ¹H-NMR (Chloroform-d) δ 8.41 (s, 1H), 8.32 (d, J = 8.2 Hz, 1H), 7.92 (d, J = 8.5 Hz, 1H), 7.74 (d, J = 10.1 Hz, 1H), 7.48 (s, 1H), 7.09 (d, J = 8.2 Hz, 1H), 7.03 (s, 1H), 6.85 (s, 1H), 6.57 (s, 1H), 6.43 (s, 1H), 6.23-6.15 (1H), 4.44 (q, J = 5.3 Hz, 1H), 3.98 (d, J = 10.7 Hz, 2H), 3.42 (td, J = 11.1, 3.3 Hz, 2H), 2.62 (d, J = 5.5 Hz, 4H), 2.39 (s, 3H), 1.69-1.60 (m, 4H); m/z N.D. |
| 101 | 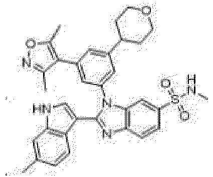 | 1-(3-(3,5-dimethylisoxazol-4-yl)-5-(tetrahydro-2H-pyran-4-yl)phenyl)-N-methyl-2-(6-methyl-1H-indol-3-yl)-1H-benzo[d]imidazole-6-sulfonamide | N.D; m/z 596 |
| 102 | 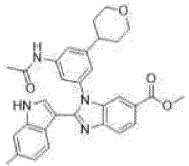 | methyl 3-(3-acetamido-5-tetrahydropyran-4-yl-phenyl)-2-(6-methyl-1H-indol-3-yl)benzimidazole-5-carboxylate | ¹H-NMR (500 MHz, Chloroform-d) δ 8.96 (s, 1H), 8.88 (s, 1H), 8.23 (d, J = 8.2 Hz, 1H), 7.98 (d, J = 8.2 Hz, 1H), 7.84 (s, 1H), 7.78 (d, J = 8.2 Hz, 1H), 7.63 (s, 1H), 7.01 (d, J = 7.9 Hz, 1H), 6.93 (s, 1H), 6.83 (s, 1H), 6.74 (s, 1H), 6.24 (s, 1H), 3.93 (d, J = 11.3 Hz, 2H), 3.88 (s, 3H), 3.44-3.29 (m, 2H), 2.72-2.58 (m, 1H), 2.37 (s, 3H), 2.30 (s, 3H), 1.62-1.52 (m, 4H); m/z 523 |
| 103 | 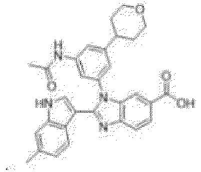 | 3-(3-acetamido-5-tetrahydropyran-4-yl-phenyl)-2-(6-methyl-1H-indol-3-yl)benzimidazole-5-carboxylic acid | N.D.; m/z 509 |
| 104 | 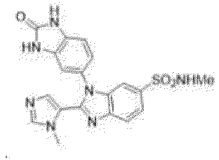 | N-methyl-2-(3-methylimidazol-4-yl)-3-(2-oxo-1,3-dihydrobenzimidazol -5-yl)benzimidazole-5-sulfonamide | ¹H-NMR (500MHz, Methanol-d₄) δ 7.93 (d, J = 8.5 Hz, 1H), 7.88 (s, 1H), 7.83-7.78 (1H), 7.63 (s, 1H), 7.28 (d, J = 7.9 Hz, 1H), 7.15 (d, J = 7.9 Hz, 2H), 6.62-6.55 (1H), 4.10 (s, 3H), 2.50-2.45 (3H); m/z N.D. |
| 105 | 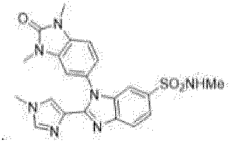 | 3-(1,3-dimethyl-2-oxo-benzimidazol-5-yl)-N-methyl-2-(1-methylimidazol-4-yl)benzimidazole-5-sulfonamide | ¹H-NMR (500 MHz, Chloroform-d) δ 7.85 (d, J = 8.2 Hz, 1H), 7.73 (d, J = 8.2 Hz, 1H), 7.63 (s, 1H), 7.43 (s, 1H), 7.17-7.09 (2H), 7.09-6.96 (2H), 4.99 (s, 1H), 3.66 (s, 3H), 3.51 (s, 3H), 3.41 (s, 3H), 2.61 (s, 3H); m/z 452 |
| 106 | 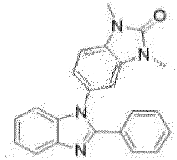 | 1',3'-dimethyl-2-phenyl-1',3'-dihydro-2'H-[1,5'-bibenzo[d]imidazol]-2'-one | ¹H-NMR (500 MHz, Chloroform-d) δ 7.90 (d, J = 8.0 Hz, 1H), 7.61 (d, J = 7.0 Hz, 2H), 7.37 - 7.32 (m, 2H), 7.31 (dd, J = 6.2, 1.4 Hz, 2H), 7.28 (dd, J = 8.3, 1.1 Hz, 1H), 7.20 (d, J = 8.1 Hz, 1H), 7.05 (s, 2H), 6.93 (s, 1H), 3.49 (s, 3H), 3.37 (s, 3H); m/z N.D. |
| 107 | 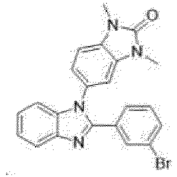 | 2-(3-bromophenyl)-1',3'-dimethyl-1',3'-dihydro-2'H-[1,5'-bibenzo[d]imidazol]-2'-one | ¹H-NMR (500 MHz, Chloroform-d) δ 7.96 (s, 1H), 7.90 (d, J = 8.0 Hz, 1H), 7.48 (d, J = 8.0 Hz, 1H), 7.37 (d, J = 6.3 Hz, 2H), 7.29 (t, J = 7.6 Hz, 1H), 7.20 (d, J = 8.0 Hz, 1H), 7.12 (t, J = 7.9 Hz, 1H), 7.10 - 7.03 (m, 2H), 6.93 (d, J = 1.4 Hz, 1H), 3.51 (s, 3H), 3.40 (s, 3H); m/z N.D. |
| 108 | 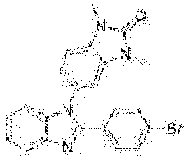 | 2-(4-bromophenyl)-1',3'-dimethyl-1',3'-dihydro-2'H-[1,5'-bibenzo[d]imidazol]-2'-one | ¹H-NMR (500 MHz, Chloroform-d) δ 7.88 (d, J = 7.9 Hz, 1H), 7.45 (dd, J = 23.3, 8.4 Hz, 4H), 7.34 (t, J = 7.5 Hz, 1H), 7.27 (dd, J = 8.3, 4.0 Hz, 1H), 7.18 (d, J = 8.0 Hz, 1H), 7.04 (dd, J = 18.1, 8.1 Hz, 2H), 6.93 (s, 1H), 3.50 (s, 3H), 3.39 (s, 3H); m/z N.D. |
| 109 | 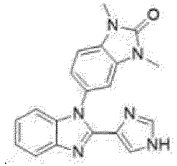 | 2-(1H-imidazol-5-yl)-1',3'-dimethyl-1',3'-dihydro-2'H-[1,5'-bibenzo[d]imidazol]-2'-one | ¹H-NMR (500 MHz, Chloroform-d) δ 7.76 (d, J = 8.0 Hz, 1H), 7.72 (s, 1H), 7.34 (t, J = 7.1 Hz, 1H), 7.28 (d, J = 7.1 Hz, 1H), 7.24 - 7.17 (m, 2H), 7.12 (d, J = 8.0 Hz, 1H), 7.05 (d, J = 1.7 Hz, 1H), 6.54 (s, 1H), 3.55 (s, 3H), 3.44 (s, 3H); m/z N.D. |
| 110 | 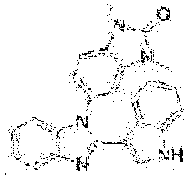 | 2-(1H-indol-3-yl)-1',3'-dimethyl-1',3'-dihydro-2'H-[1,5'-bibenzo[d]imidazol]-2'-one | ¹H-NMR (500 MHz, Chloroform-d) δ 8.62 (s, 1H), 8.56 (s, 1H), 7.90 (d, J = 8.0 Hz, 1H), 7.31 (dd, J = 12.1, 4.2 Hz, 2H), 7.25 - 7.20 (m, 3H), 7.17 (dd, J = 8.1, 1.8 Hz, 1H), 7.12 - 7.06 (m, 2H), 6.98 (d, J = 1.7 Hz, 1H), 6.60 (d, J = 2.4 Hz, 1H), 3.51 (s, 3H), 3.36 (s, 3H); m/z N.D. |
| 111 | 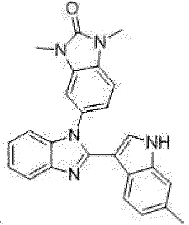 | 1',3'-dimethyl-2-(6-methyl-1H-indol-3-yl)-1',3'-dihydro-2'H-[1,5'-bibenzo[d]imidazol]-2'-one | ¹H-NMR (500 MHz, Chloroform-d) δ 8.42 (d, J = 7.8 Hz, 1H), 8.35 (s, 1H), 7.89 (d, J = 8.0 Hz, 1H), 7.30 (t, J = 7.6 Hz, 1H), 7.21 (t, J = 7.5 Hz, 1H), 7.17 (dd, J = 8.1, 1.6 Hz, 1H), 7.08 (dd, J = 12.1, 8.1 Hz, 4H), 6.98 (s, 1H), 6.55 (s, 1H), 3.51 (s, 3H), 3.36 (s, 3H), 2.45 (s, 3H); m/z N.D. |
| 112 | 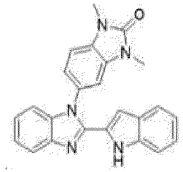 | 2-(1H-indol-2-yl)-1',3'-dimethyl-1',3'-dihydro-2'H-[1,5'-bibenzo[d]imidazol]-2'-one | ¹H-NMR (500 MHz, Chloroform-d) δ 10.40 (s, 1H), 7.81 (d, J = 8.0 Hz, 1H), 7.43 (d, J = 8.0 Hz, 1H), 7.38 - 7.30 (m, 2H), 7.28 - 7.24 (m, 2H), 7.23 - 7.16 (m, 2H), 7.12 - 7.07 (m, J = 4.8, 3.0 Hz, 2H), 7.04 (t, J = 7.1 Hz, 1H), 5.89 (d, J = 1.3 Hz, 1H), 3.59 (s, 3H), 3.44 (s, 3H); m/z N.D. |
| 113 | 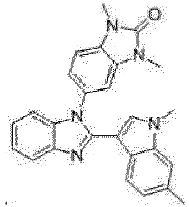 | 2-(1,6-dimethyl-1*H-*indol-3-yl)-1',3'-dimethyl-1',3'-dihydro-2'*H*-[1,5'-bibenzo[*d*]imidazol]-2'-one | ¹H-NMR (500 MHz, Chloroform-d) δ 8.43 (d, J = 8.2 Hz, 1H), 7.88 (d, J = 8.0 Hz, 1H), 7.31 - 7.27 (m, 1H), 7.21 - 7.16 (m, 2H), 7.10 (dd, J = 15.5, 7.7 Hz, 3H), 7.04 (d, J = 7.9 Hz, 1H), 7.00 (d, J = 1.8 Hz, 1H), 6.44 (s, 1H), 3.59 (s, 3H), 3.52 (s, 3H), 3.38 (s, 3H), 2.50 (s, 3H); m/z N.D. |
| 114 | 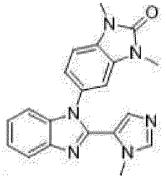 | 1',3'-dimethyl-2-(1-methyl-1H-imidazol-5-yl)-1',3'-dihydro-2'H-[1,5'-bibenzo[d]imidazol]-2'-one | ¹H-NMR (500 MHz, Chloroform-d) δ 7.83 (dt, 1H), 7.51 (s, 1H), 7.33 (ddd, J = 8.1, 7.2, 1.2 Hz, 1H), 7.27 - 7.23 (m, 1H), 7.13 - 7.07 (m, 3H), 6.94 (dd, J = 1.5, 0.6 Hz, 1H), 6.46 (s, 1H), 4.16 (s, 3H), 3.52 (s, 3H), 3.40 (s, 3H); m/z N.D. |
| 115 | 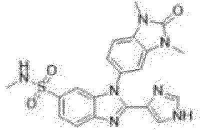 | 2-(1H-imidazol-5-yl)-N,1',3'-trimethyl-2'-oxo-2',3'-dihydro-1'H-[1,5'-bibenzo[d]imidazole ]-6-sulfonamide | ¹H-NMR (500 MHz, Acetone-d₆) δ 7.82 (d, J = 8.5 Hz, 1H), 7.77 (ddd, J = 7.4, 4.1, 0.8 Hz, 1H), 7.73 (dd, J = 8.5, 1.7 Hz, 1H), 7.64 - 7.58 (m, 1H), 7.54 (dd, J = 1.7, 0.6 Hz, 1H), 7.41 - 7.38 (m, 1H), 7.35 (s, 1H), 7.25 (s, 1H), 6.28 (dd, J = 10.1, 5.2 Hz, 1H), 3.51 (s, 3H), 3.41 (s, 3H), 2.52 (d, J = 5.2 Hz, 3H); m/z N.D. |
| 116 | 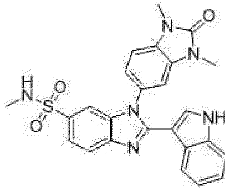 | 2-(1H-indol-3-yl)-N,1',3'-trimethyl-2'-oxo-2',3'-dihydro-1'H-[1,5'-bibenzo[d]imidazole ]-6-sulfonamide | ¹H-NMR (500 MHz, Acetone-d₆) δ 10.35 (s, 1H), 7.75 (d, J = 8.4 Hz, 1H), 7.68 (dd, J = 8.4, 1.7 Hz, 1H), 7.44 (d, J = 1.6 Hz, 1H), 7.38 (d, J = 8.1 Hz, 1H), 7.32 (d, J = 1.9 Hz, 1H), 7.23 (dd, J = 8.1, 2.0 Hz, 1H), 6.80 (dt, J = 3.1, 1.7 Hz, 1H), 6.77 (dd, J = 4.6, 2.6 Hz, 1H), 6.36 (dd, J = 4.2, 2.7 Hz, 1H), 6.17 (q, J = 5.3 Hz, 1H), 3.51 (s, 3H), 3.42 (s, 3H), 2.51 (d, J = 5.2 Hz, 3H); m/z N.D. |
| 117 | 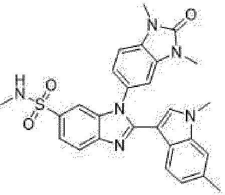 | 2-(1,6-dimethyl-1*H-*indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, DMSO-d₆) δ 8.53 (d, J = 8.3 Hz, 1H), 7.94 (d, J = 8.1 Hz, 2H), 7.60 (dd, J = 8.4, 1.8 Hz, 1H), 7.51 (d, J = 2.0 Hz, 1H), 7.45 (d, J = 8.2 Hz, 1H), 7.27 (dd, J = 12.8, 4.6 Hz, 2H), 7.22 (s, 1H), 7.11 (d, J = 6.9 Hz, 1H), 6.59 (s, 1H), 3.62 (s, 3H), 3.46 (s, 3H), 2.89 (s, 3H), 2.73 (s, 3H), 2.56 (s, 3H); m/z N.D. |
| 118 | 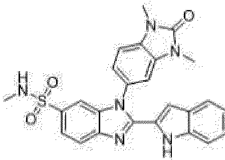 | 2-(1H-indol-2-yl)-N,1',3'-trimethyl-2'-oxo-2',3'-dihydro-1'H-[1,5'-bibenzo[d]imidazole ]-6-sulfonamide | ¹H-NMR (500 MHz, DMSO-d₆) δ 12.15 (s, 1H), 7.96 (d, J = 8.5 Hz, 1H), 7.72 (dd, J = 8.0, 1.7 Hz, 1H), 7.59 (s, 1H), 7.49 (dd, J = 13.3, 8.2 Hz, 2H), 7.43 - 7.39 (m, 3H), 7.37 (dd, J = 8.3, 1.9 Hz, 1H), 7.16 (t, 1H), 6.95 (t, J = 7.4 Hz, 1H), 5.87 (s, 1H), 3.49 (s, 3H), 2.89 (s, 3H), 2.73 (s, 3H); m/z N.D. |
| 119 | 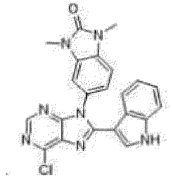 | 5-(6-chloro-8-(1H-indol-3-yl)-9H-purin-9-yl)-1,3-dimethyl-1,3-dihydro-2H-benzo[d]imidazol-2-one | ¹H-NMR (500 MHz, Acetone-d₆) δ 10.93 (s, 1H), 8.84 (dd, J = 7.7, 1.5 Hz, 1H), 8.16 (s, 1H), 7.63 (d, J = 2.9 Hz, 1H), 7.50 (d, J = 23.2 Hz, 2H), 7.27 - 7.24 (m, 2H), 7.21 (s, 1H), 6.93 (s, 1H), 3.38 (s, 3H), 3.33 (s, 3H); m/z N.D. |
| 120 | 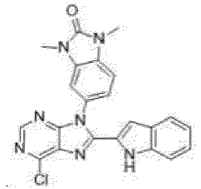 | 5-(6-chloro-8-(1H-indol-2-yl)-9H-purin-9-yl)-1,3-dimethyl-1,3-dihydro-2H-benzo[d]imidazol-2-one | ¹H-NMR (500 MHz, DMSO-d₆) δ 12.15 (s, 1H), 7.95 (s, 1H), 7.72 (dd, J = 8.0, 1.7 Hz, 1H), 7.59 (d, J = 1.9 Hz, 1H), 7.50 (d, J = 8.1 Hz, 1H), 7.47 (d, J = 8.3 Hz, 1H), 7.37 (dd, J = 7.9, 2.3 Hz, 1H), 7.18 - 7.14 (m, 1H), 6.95 (t, J = 7.0 Hz, 1H), 5.87 (s, 1H), 2.89 (s, 3H), 2.73 (s, 3H); m/z N.D. |
| 121 | 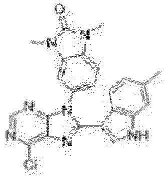 | 5-(6-chloro-8-(6-methyl-1H-indol-3-yl)-9H-purin-9-yl)-1,3-dimethyl-1,3-dihydro-2H-benzo[d]imidazol-2-one | ¹H-NMR (500 MHz, Methanol-d₄) δ 8.53 (s, 1H), 8.40 (d, J = 8.4 Hz, 1H), 7.41 (dd, J = 5.1, 3.1 Hz, 2H), 7.28 (dd, J = 8.3, 1.9 Hz, 1H), 7.21 (s, 1H), 7.10 (d, J = 8.1 Hz, 1H), 6.71 (s, 1H), 3.54 (s, 3H), 3.42 (s, 3H), 2.46 (s, 3H); m/z N.D. |
| 122 | 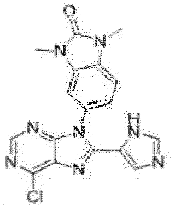 | 5-(6-chloro-8-(1H-imidazol-5-yl)-9H-purin-9-yl)-1,3-dimethyl-1,3-dihydro-2H-benzo[d]imidazol-2-one | ¹H-NMR (500 MHz, Acetone-d₆) δ 8.57 (s, 1H), 7.77 (s, 1H), 7.42 - 7.21 (m, 4H), 3.50 (s, 3H), 3.40 (s, 3H); m/z N.D. |
| 123 | 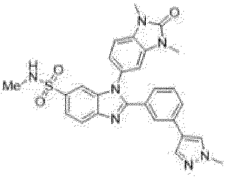 | N,1',3'-trimethyl-2-(3-(1-methyl-1H-pyrazol-4-yl)phenyl)-2'-oxo-2',3'-dihydro-1'H-[1,5'-bibenzo[d]imidazole ]-6-sulfonamide | ¹H-NMR (500 MHz, Acetone-d₆) δ 7.94 (dd, J = 8.5, 0.5 Hz, 1H), 7.91 (t, J = 1.6 Hz, 1H), 7.85 (s, 1H), 7.79 (dd, J = 8.5, 1.7 Hz, 1H), 7.67 (d, J = 1.2 Hz, 1H), 7.62 - 7.58 (m, 2H), 7.49 (ddd, J = 7.8, 1.7, 1.1 Hz, 1H), 7.38 (d, J = 1.9 Hz, 1H), 7.35 (d, J = 8.1 Hz, 1H), 7.32 (t, J = 8.0 Hz, 1H), 7.26 (dd, J = 8.1, 2.0 Hz, 1H), 6.26 (dd, J = 10.4, 5.2 Hz, 1H), 3.88 (s, 3H), 3.49 (s, 3H), 3.38 (s, 3H), 2.54 (d, J = 5.2 Hz, 3H); m/z N.D. |
| 124 | 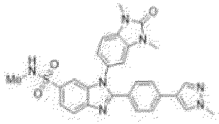 | N,1',3'-trimethyl-2-(4-(1-methyl-1H-pyrazol-4-yl)phenyl)-2'-oxo-2',3'-dihydro-1'H-[1,5'-bibenzo[d]imidazole ]-6-sulfonamide | ¹H-NMR (500 MHz, Acetone-d₆) δ 8.03 (s, 1H), 7.91 (d, J = 8.4 Hz, 1H), 7.83 (s, 1H), 7.77 (dd, J = 8.5, 1.7 Hz, 1H), 7.69 (d, J = 8.5 Hz, 2H), 7.63 (d, J = 1.6 Hz, 1H), 7.55 (d, J = 8.6 Hz, 2H), 7.37 (d, J = 2.1 Hz, 1H), 7.34 (d, J = 8.2 Hz, 1H), 7.23 (dd, J = 8.2, 2.0 Hz, 1H), 6.27 (s, 1H), 3.90 (s, 3H), 3.49 (s, 3H), 3.38 (s, 3H), 2.53 (d, J = 5.2 Hz, 3H); m/z N.D. |
| 125 | 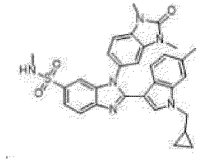 | 2-(1-(cyclopropylmethyl) -6-methyl-1*H*-indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 8.69 (d, J = 8.2 Hz, 1H), 7.87 (d, J = 8.4 Hz, 1H), 7.73 (dd, J = 8.4, 1.8 Hz, 1H), 7.51 (d, J = 1.4 Hz, 1H), 7.45 (d, J = 8.1 Hz, 1H), 7.38 (d, J = 1.9 Hz, 1H), 7.33 - 7.29 (m, 2H), 7.12 (d, J = 8.2 Hz, 1H), 6.72 (s, 1H), 6.20 (q, J = 5.2 Hz, 1H), 3.84 (d, J = 7.0 Hz, 2H), 3.53 (s, 3H), 3.42 (s, 3H), 2.53 (d, J = 5.2 Hz, 3H), 2.48 (s, 3H), 1.07 - 1.01 (m, 1H), 0.35 (dd, J = 8.0, 1.3 Hz, 2H), 0.12 (d, J = 5.2 Hz, 2H); m/z N.D. |
| 126 | 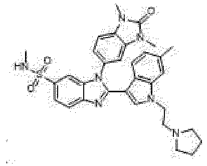 | *N*,1',3'-trimethyl-2-(6-methyl-1-(2-(pyrrolidin-1-yl)ethyl)-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 8.69 (d, J = 8.2 Hz, 1H), 7.88 (d, J = 8.4 Hz, 1H), 7.73 (dd, J = 8.4, 1.7 Hz, 1H), 7.48 (s, 1H), 7.42 (d, J = 8.1 Hz, 1H), 7.38 (d, J = 1.8 Hz, 1H), 7.29 (dd, J = 8.1, 2.0 Hz, 1H), 7.25 (s, 1H), 7.10 (d, J = 8.4 Hz, 1H), 6.71 (d, J = 2.7 Hz, 1H), 6.19 (d, J = 5.2 Hz, 1H), 4.03 (s, 3H), 3.52 (s, 3H), 3.41 (s, 3H), 3.30 (s, 6H), 3.09 (q, J = 5.4 Hz, 2H), 2.53 (d, J = 5.3 Hz, 4H), 2.44 (s, 3H); m/z N.D. |
| 127 | 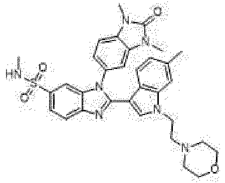 | *N*,1',3'-trimethyl-2-(6-methyl-1-(2-morpholinoethyl)-1*H*-indol-3-yl)-2'-oxo-2',3'-dihydro-1'*H*-[1,5'-bibenzo[*d*]imidazole]-6-sulfonamide | ¹H-NMR (500 MHz, Acetone-d₆) δ 8.70 (d, J = 8.2 Hz, 1H), 7.86 (d, J = 8.4 Hz, 1H), 7.72 (dd, J = 8.4, 1.8 Hz, 1H), 7.48 (d, J = 1.4 Hz, 1H), 7.44 (d, J = 8.1 Hz, 1H), 7.39 (d, J = 1.9 Hz, 1H), 7.34 - 7.27 (m, 2H), 7.11 (dd, J = 8.2, 0.9 Hz, 1H), 6.78 (s, 1H), 6.18 (dd, J = 10.4, 5.2 Hz, 1H), 4.10 (t, J = 6.0 Hz, 2H), 3.51 (s, 3H), 3.43 - 3.38 (m, 7H), 2.59 - 2.51 (m, 5H), 2.47 (s, 3H), 2.29 - 2.21 (m, 4H); m/z N.D. |
| 128 | 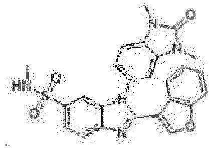 | 2-(benzofuran-3-yl)-*N*,1',3'-trimethyl-2'-oxo-2',3'-dihydro-1'*H*-[1,5'-bibenzo[*d*]imidazole ]-6-sulfonamide | ¹H-NMR (500 MHz, Acetone-d₆) δ 9.00 (d, J = 8.3 Hz, 1H), 8.03 - 7.98 (m, 2H), 7.82 (dd, J = 8.5, 1.7 Hz, 1H), 7.66 (d, J = 1.3 Hz, 1H), 7.55 (ddd, J = 8.3, 7.1, 1.2 Hz, 1H), 7.49 (dd, J = 8.0, 1.2 Hz, 1H), 7.46 (s, 1H), 7.44 (d, J = 1.9 Hz, 1H), 7.33 (d, J = 8.1 Hz, 1H), 7.27 (dd, J = 8.2, 2.0 Hz, 1H), 6.31 (q, J = 5.2 Hz, 1H), 3.47 (s, 3H), 3.37 (s, 3H), 2.55 (d, J = 5.2 Hz, 3H); m/z N.D. |
| 129 | 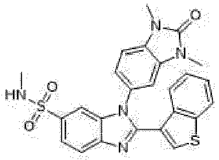 | 2-(benzo[*b*]thiophen-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 8.71 (dd, J = 6.3, 2.9 Hz, 1H), 8.00 (d, J = 8.5 Hz, 1H), 7.80 (dd, J = 8.5, 1.7 Hz, 1H), 7.60 (d, J = 1.2 Hz, 1H), 7.57 (dd, J = 6.2, 3.0 Hz, 1H), 7.49 - 7.41 (m, 4H), 7.35 (dd, J = 8.1, 2.0 Hz, 1H), 7.21 (s, 1H), 6.28 (d, J = 5.1 Hz, 1H), 3.53 (s, 3H), 3.42 (s, 3H), 2.55 (d, J = 5.2 Hz, 3H); m/z N.D. |
| 130 | 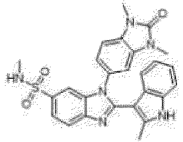 | *N*,1',3'-trimethyl-2-(2-methyl-1*H*-indol-3-yl)-2'-oxo-2',3'-dihydro-1'*H*-[1,5'-bibenzo[*d*]imidazole]-6-sulfonamide | ¹H-NMR (500 MHz, Methanol-d₄) δ 7.89 (dd, J = 8.8, 2.8 Hz, 2H), 7.80 (d, J = 8.5 Hz, 1H), 7.28 (dd, J = 20.0, 6.0 Hz, 2H), 7.18 (s, 1H), 7.11 (d, J = 1.8 Hz, 2H), 7.01 (td, J = 7.8, 2.2 Hz, 1H), 6.87 (td, J = 7.6, 2.4 Hz, 1H), 3.36 (s, 3H), 3.24 (s, 3H), 2.50 (s, 3H), 2.31 (s, 3H); m/z N.D. |
| 131 | 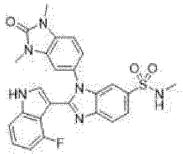 | 2-(4-fluoro-1*H-*indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 10.88 (s, 1H), 7.88 (d, J = 8.6 Hz, 1H), 7.76 (dd, J = 8.6, 1.7 Hz, 1H), 7.67 (d, J = 1.6 Hz, 1H), 7.41 (d, J = 2.3 Hz, 1H), 7.30 (d, J = 1.5 Hz, 1H), 7.28 (d, J = 8.0 Hz, 1H), 7.21 (d, J = 8.1 Hz, 1H), 7.19 (dd, J = 8.1, 1.7 Hz, 1H), 7.14 (td, J = 7.9, 4.9 Hz, 1H), 6.79 (dd, J = 11.3, 7.8 Hz, 1H), 6.24 (d, J = 5.3 Hz, 1H), 3.41 (s, 3H), 3.32 (s, 3H), 2.54 (d, J = 5.2 Hz, 3H) ; m/z N.D. |
| 132 | 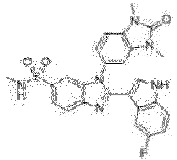 | 2-(5-fluoro-1*H-*indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 10.62 (s, 1H), 8.56 (dd, J = 10.4, 2.6 Hz, 1H), 7.91 (d, J = 8.4 Hz, 1H), 7.75 (dd, J = 8.4, 1.7 Hz, 1H), 7.50 (d, J = 1.7 Hz, 1H), 7.48 (dd, J = 8.8, 4.6 Hz, 1H), 7.43 (d, J = 8.1 Hz, 1H), 7.42 (d, J = 1.9 Hz, 1H), 7.32 (dd, J = 8.1, 2.0 Hz, 1H), 7.05 (td, J = 9.0, 2.7 Hz, 1H), 6.85 (d, J = 3.0 Hz, 1H), 6.22 (q, J = 4.9 Hz, 1H), 3.52 (s, 3H), 3.42 (s, 3H), 2.53 (d, J = 5.3 Hz, 3H) ; m/z N.D. |
| 133 | 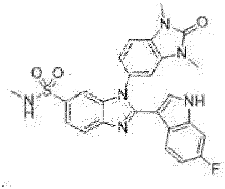 | 2-(6-fluoro-1*H-*indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 10.57 (s, 1H), 8.83 (dd, J = 8.9, 5.6 Hz, 1H), 7.90 (d, J = 8.4 Hz, 1H), 7.74 (d, J = 8.4 Hz, 1H), 7.49 (s, 1H), 7.43 (d, J = 8.2 Hz, 1H), 7.41 (s, 1H), 7.32 - 7.29 (m, 1H), 7.22 - 7.18 (m, 1H), 7.08 (t, J = 9.1 Hz, 1H), 6.80 (s, 1H), 6.21 (d, J = 4.8 Hz, 1H), 3.52 (s, 3H), 3.41 (s, 3H), 2.53 (d, J = 6.1 Hz, 3H) ; m/z N.D. |
| 134 | 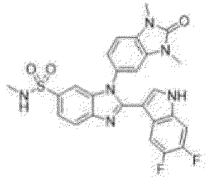 | 2-(5,6-difluoro-1*H-*indol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 10.68 (s, 1H), 8.72 (dd, J = 11.8, 8.4 Hz, 1H), 7.92 (d, J = 8.4 Hz, 1H), 7.75 (dd, J = 8.4, 1.7 Hz, 1H), 7.50 (d, J = 1.4 Hz, 1H), 7.42 (td, J = 8.7, 4.3 Hz, 3H), 7.32 (dd, J = 8.1, 1.9 Hz, 1H), 6.84 (d, J = 2.4 Hz, 1H), 6.23 (d, J = 5.1 Hz, 1H), 3.52 (s, 3H), 3.41 (s, 3H), 2.53 (d, J = 5.2 Hz, 3H) ; m/z N.D. |
| 135 | 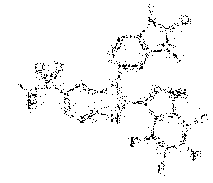 | *N*,1',3'-trimethyl-2'-oxo-2-(4,5,6,7-tetrafluoro-1*H*-indol-3-yl)-2',3'-dihydro-1'*H*-[1,5'-bibenzo[*d*]imidazole ]-6-sulfonamide | ¹H-NMR (500 MHz, Chloroform-d) δ 8.89 (s, 1H), 7.98 (d, J = 8.5 Hz, 1H), 7.81 (dd, J = 8.5, 1.7 Hz, 1H), 7.76 (d, J = 1.6 Hz, 1H), 7.18 (d, J = 2.4 Hz, 1H), 7.08 (dd, J = 8.2, 1.8 Hz, 1H), 7.03 (d, J = 8.2 Hz, 1H), 6.92 (d, J = 1.7 Hz, 1H), 4.34 (q, J = 5.4 Hz, 1H), 3.47 (s, 3H), 3.37 (s, 3H), 2.66 (d, J = 5.4 Hz, 3H) ; m/z N.D. |
| 136 | 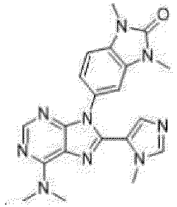 | 5-(6-(dimethylamino)-8-(1-methyl-1H-imidazol-5-yl)-9*H-*purin-9-yl)-1,3-dimethyl-1,3-dihydro-2*H-*benzo[*d*]imidazol-2-one | ¹H-NMR (500 MHz, Methanol-d₄) δ 8.14 (s, 1H), 7.75 (s, 1H), 7.34 (d, J = 8.2 Hz, 1H), 7.30 (d, J = 1.9 Hz, 1H), 7.15 (dd, J = 8.2, 1.9 Hz, 1H), 6.36 (d, J = 0.9 Hz, 1H), 4.10 (s, 3H), 3.60 (s, 6H), 3.51 (s, 3H), 3.42 (s, 3H) ; m/z N.D. |
| 137 | 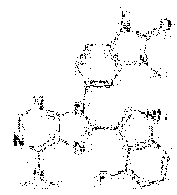 | 5-(6-(dimethylamino)-8-(4-fluoro-1H-indol-3-yl)-9*H*-purin-9-yl)-1,3-dimethyl-1,3-dihydro-2*H-*benzo[*d*]imidazol-2-one | ¹H-NMR (500 MHz, Chloroform-d) δ 8.51 (s, 1H), 8.34 (s, 1H), 7.14 - 7.11 (m, 2H), 7.07 - 7.05 (m, 1H), 7.05 (d, J = 2.8 Hz, 1H), 7.02 (d, J = 1.7 Hz, 1H), 6.93 (d, J = 8.2 Hz, 1H), 6.81 (ddd, J = 10.9, 6.2, 2.3 Hz, 1H), 3.63 (s, 6H), 3.39 (s, 3H), 3.33 (s, 3H) ; m/z N.D. |
| 138 | 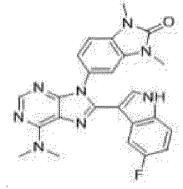 | 5-(6-(dimethylamino)-8-(5-fluoro-1*H*-indol-3-yl)-9*H*-purin-9-yl)-1,3-dimethyl-1,3-dihydro-2*H-*benzo[*d*]imidazol-2-one | ¹H-NMR (500 MHz, Acetone-d₆) δ 10.49 (s, 1H), 8.35 (dd, J = 10.4, 2.7 Hz, 1H), 8.12 (s, 1H), 7.45 (dd, J = 8.8, 4.5 Hz, 1H), 7.32 (d, J = 8.1 Hz, 1H), 7.28 (d, J = 1.9 Hz, 1H), 7.18 (dd, J = 8.1, 1.9 Hz, 1H), 7.03 (td, J = 9.1, 2.6 Hz, 1H), 6.74 - 6.73 (m, 1H), 3.65 (dd, J = 69.5, 42.5 Hz, 6H), 3.49 (s, 3H), 3.40 (s, 3H) ; m/z N.D. |
| 139 | 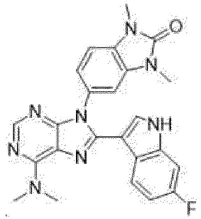 | 5-(6-(dimethylamino)-8-(6-fluoro-1*H*-indol-3-yl)-9*H*-purin-9-yl)-1,3-dimethyl-1,3-dihydro-2*H-*benzo[*d*]imidazol-2-one | ¹H-NMR (500 MHz, Methanol-d₄) δ 8.50 (dd, J = 8.9, 5.5 Hz, 1H), 8.10 (s, 1H), 7.38 (d, J = 8.2 Hz, 1H), 7.33 (d, J = 1.9 Hz, 1H), 7.20 (dd, J = 8.2, 2.0 Hz, 1H), 7.08 (dd, J = 9.5, 2.3 Hz, 1H), 7.00 - 6.95 (m, 1H), 6.54 (s, 1H), 3.69 (s, 6H), 3.53 (s, 3H), 3.43 (s, 3H) ; m/z N.D. |
| 140 | 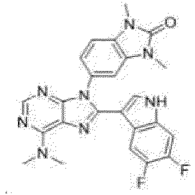 | 5-(8-(5,6-difluoro-1*H*-indol-3-yl)-6-(dimethylamino)-9*H*-purin-9-yl)-1,3-dimethyl-1,3-dihydro-2*H-*benzo[*d*]imidazol-2-one | ¹H-NMR (500 MHz, Methanol-d₄) δ 8.35 (dd, J = 11.6, 8.2 Hz, 1H), 8.11 (s, 1H), 7.39 (d, J = 8.2 Hz, 1H), 7.34 (d, J = 1.8 Hz, 1H), 7.26 (dd, J = 10.7, 6.9 Hz, 1H), 7.21 (dd, J = 8.2, 2.0 Hz, 1H), 6.56 (s, 1H), 3.69 (s, 6H), 3.54 (s, 3H), 3.43 (s, 3H) ; m/z N.D. |
| 141 | 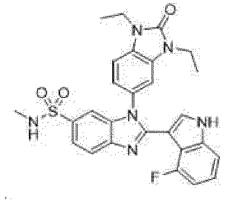 | 1',3'-diethyl-2-(4-fluoro-1*H*-indol-3-yl)-*N*-methyl-2'-oxo-2',3'-dihydro-1'*H-*[1,5'-bibenzo[*d*]imidazole ]-6-sulfonamide | ¹H-NMR (500 MHz, Acetone-d₆) δ 10.57 (s, 1H), 8.81 (dd, J = 8.9, 5.7 Hz, 1H), 7.89 (dd, J = 8.4, 0.4 Hz, 1H), 7.75 (dd, J = 8.4, 1.8 Hz, 1H), 7.53 (dd, 1H), 7.48 (dd, J = 7.7, 5.0 Hz, 2H), 7.30 (dd, J = 8.1, 2.0 Hz, 1H), 7.21 (dd, J = 9.6, 2.3 Hz, 1H), 7.07 (ddd, J = 9.7, 8.9, 2.4 Hz, 1H), 6.83 (d, J = 2.9 Hz, 1H), 6.20 (q, J = 5.2 Hz, 1H), 4.06 (dd, J = 14.2, 7.1 Hz, 2H), 3.96 (q, J = 7.2 Hz, 2H), 2.54 (d, J = 5.2 Hz, 3H), 1.39 (t, J = 7.2 Hz, 3H), 1.24 (t, J = 7.2 Hz, 3H) ; m/z N.D. |
| 142 | 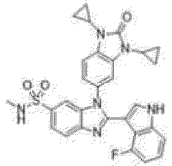 | 1',3'-dicyclopropyl-2-(4-fluoro-1*H-*indol-3-yl)-*N-*methyl-2'-oxo-2',3'-dihydro-1'*H*-[1,5'-bibenzo[*d*]imidazole ]-6-sulfonamide | ¹H-NMR (500 MHz, Acetone-d₆) δ 10.57 (s, 1H), 8.81 (dd, J = 8.9, 5.7 Hz, 1H), 7.90 (d, J = 8.4 Hz, 1H), 7.75 (dd, J = 8.4, 1.7 Hz, 1H), 7.53 (d, J = 1.6 Hz, 1H), 7.49 (d, J = 8.1 Hz, 1H), 7.46 (d, J = 2.0 Hz, 1H), 7.30 (dd, J = 8.1, 2.0 Hz, 1H), 7.20 (dd, J = 9.6, 2.4 Hz, 1H), 7.07 (td, 1H), 6.86 (d, J = 2.9 Hz, 1H), 6.21 (q, J = 5.1 Hz, 1H), 2.97 (tt, J = 6.9, 3.6 Hz, 1H), 2.85 (td, J = 6.8, 3.5 Hz, 1H), 2.55 (d, J = 5.2 Hz, 3H), 1.17 - 1.10 (m, 2H), 1.06 - 1.01 (m, 2H), 0.97 (d, J = 6.8 Hz, 2H), 0.91 (d, J = 3.4 Hz, 2H) ; m/z N.D. |
| 143 | 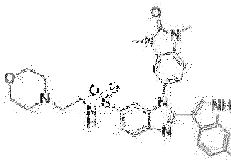 | 1',3'-dimethyl-2-(6-methyl-1*H*-indol-3-yl)-*N*-(2-morpholinoethyl)-2'-oxo-2',3'-dihydro-1'*H*-[1,5'-bibenzo[*d*]imidazole ]-6-sulfonamide | ¹H-NMR (500 MHz, Acetone-d₆) δ 10.39 (s, 1H), 8.69 (d, J = 8.2 Hz, 1H), 7.87 (d, J = 8.4 Hz, 1H), 7.76 (dd, J = 8.4, 1.8 Hz, 1H), 7.52 (d, J = 1.5 Hz, 1H), 7.42 (d, J = 8.1 Hz, 1H), 7.38 (d, J = 1.9 Hz, 1H), 7.29 (dd, J = 8.1, 2.0 Hz, 1H), 7.25 (s, 1H), 7.10 (dd, J = 8.3, 1.1 Hz, 1H), 6.71 (d, J = 2.9 Hz, 1H), 6.12 (s, 1H), 3.52 (s, 3H), 3.50 (t, 4H), 3.42 (s, 3H), 2.98 (dd, J = 11.8, 5.9 Hz, 2H), 2.44 (s, 3H), 2.35 (t, J = 6.1 Hz, 2H), 2.24 (s, 4H) ; m/z N.D. |
| 144 | 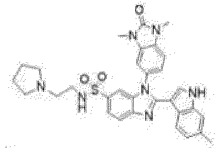 | 1',3'-dimethyl-2-(6-methyl-1*H*-indol-3-yl)-2'-oxo-*N*-(2-(pyrrolidin-1-yl)ethyl)-2',3'-dihydro-1'*H*-[1,5'-bibenzo[*d*]imidazole ]-6-sulfonamide | ¹H-NMR (500 MHz, Acetone-d₆) δ 10.36 (s, 1H), 8.69 (d, J = 8.2 Hz, 1H), 7.84 (dd, J = 8.4, 0.5 Hz, 1H), 7.80 (dd, J = 8.4, 1.7 Hz, 1H), 7.53 (d, J = 1.2 Hz, 1H), 7.42 (d, J = 8.1 Hz, 1H), 7.37 (d, J = 1.9 Hz, 1H), 7.28 (dd, J = 8.1, 2.0 Hz, 1H), 7.25 (s, 1H), 7.10 (d, J = 8.3 Hz, 1H), 6.69 (d, J = 2.9 Hz, 1H), 6.44 (s, 1H), 3.52 (s, 3H), 3.42 (s, 3H), 2.78 (s, 8H), 2.44 (s, 3H) ; m/z N.D. |
| 145 | 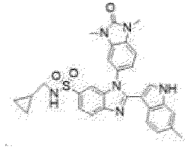 | *N-*(cyclopropylmethyl) -1',3'-dimethyl-2-(6-methyl-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 10.36 (s, 1H), 8.68 (d, J = 8.2 Hz, 1H), 7.86 (d, J = 8.4 Hz, 1H), 7.75 (dd, J = 8.4, 1.8 Hz, 1H), 7.50 (d, J = 1.4 Hz, 1H), 7.42 (d, J = 8.1 Hz, 1H), 7.38 (d, J = 1.9 Hz, 1H), 7.29 (dd, J = 8.1, 2.0 Hz, 1H), 7.25 (s, 1H), 7.10 (d, J = 8.3 Hz, 1H), 6.71 (d, J = 2.9 Hz, 1H), 6.44 (t, J = 6.0 Hz, 1H), 3.52 (s, 3H), 3.42 (s, 3H), 2.75 (t, J = 6.5 Hz, 2H), 2.44 (s, 3H), 0.91 - 0.81 (m, 1H), 0.37 (ddd, J = 8.0, 5.9, 4.5 Hz, 2H), 0.09 (dt, J = 5.9, 4.5 Hz, 2H) ; m/z N.D. |
| 146 | 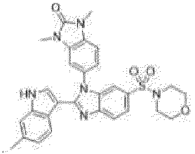 | 1',3'-dimethyl-2-(6-methyl-1*H*-indol-3-yl)-6-(morpholinosulfonyl )-1',3'-dihydro-2'*H-*[1,5'-bibenzo[*d*]imidazol]-2'-one | ¹H-NMR (500 MHz, Acetone-d₆) δ 10.40 (s, 1H), 8.69 (d, J = 8.2 Hz, 1H), 7.93 (d, J = 8.4 Hz, 1H), 7.65 (dd, J = 8.4, 1.7 Hz, 1H), 7.43 - 7.39 (m, 3H), 7.33 (dd, J = 8.1, 2.0 Hz, 1H), 7.25 (s, 1H), 7.11 (dd, J = 8.3, 1.2 Hz, 1H), 6.73 - 6.70 (m, 1H), 3.67 - 3.63 (m, 4H), 3.52 (s, J = 0.6 Hz, 3H), 3.41 (s, J = 0.4 Hz, 3H), 2.93 - 2.88 (m, 4H), 2.44 (s, 3H) ; m/z N.D. |
| 147 | 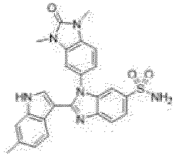 | 1',3'-dimethyl-2-(6-methyl-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 10.35 (s, 1H), 8.69 (d, J = 7.9 Hz, 1H), 7.85 (d, J = 8.8 Hz, 1H), 7.82 - 7.78 (m, 1H), 7.53 (s, 1H), 7.42 (d, J = 8.2 Hz, 1H), 7.37 (d, J = 1.9 Hz, 1H), 7.28 (dd, J = 8.1, 2.0 Hz, 1H), 7.24 (s, 1H), 7.10 (d, J = 9.1 Hz, 1H), 6.69 (d, J = 2.9 Hz, 1H), 6.44 (s, 2H), 3.52 (s, 3H), 3.42 (s, 3H), 2.44 (s, 3H) ; m/z N.D. |
| 148 | 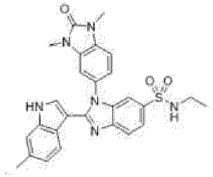 | *N*-ethyl-1',3'-dimethyl-2-(6-methyl-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 10.37 (s, 1H), 8.69 (d, J = 8.2 Hz, 1H), 7.87 (d, J = 8.4 Hz, 1H), 7.74 (dd, J = 8.4, 1.8 Hz, 1H), 7.49 (d, J = 1.4 Hz, 1H), 7.42 (d, J = 8.1 Hz, 1H), 7.38 (d, J = 1.9 Hz, 1H), 7.29 (dd, J = 8.1, 2.0 Hz, 1H), 7.25 (s, 1H), 7.10 (dd, J = 8.3, 1.1 Hz, 1H), 6.71 - 6.70 (m, 1H), 6.28 (t, J = 6.0 Hz, 1H), 3.52 (s, 3H), 3.47 (dd, J = 19.2, 7.3 Hz, 2H), 3.41 (s, 3H), 2.44 (s, 3H), 1.04 (t, J = 7.2 Hz, 3H) ; m/z N.D. |
| 149 | 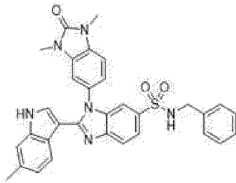 | *N*-benzyl-1',3'-dimethyl-2-(6-methyl-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 10.36 (s, 1H), 8.70 (d, J = 8.2 Hz, 1H), 7.87 (d, J = 8.4 Hz, 1H), 7.79 (dd, J = 8.4, 1.8 Hz, 1H), 7.51 - 7.50 (m, 1H), 7.42 (d, J = 8.1 Hz, 1H), 7.37 (d, J = 1.9 Hz, 1H), 7.28 (dd, J = 8.1, 2.0 Hz, 1H), 7.26 - 7.23 (m, 5H), 7.20 (dq, J = 5.9, 3.8 Hz, 1H), 7.11 (dd, J = 8.2, 1.0 Hz, 1H), 6.80 (t, J = 6.3 Hz, 1H), 6.73 - 6.69 (m, 1H), 4.08 (d, J = 6.6 Hz, 2H), 3.52 (s, 3H), 3.41 (s, 3H), 2.45 (s, 3H) ; m/z N.D. |
| 150 | 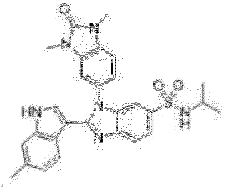 | *N*-isopropyl-1',3'-dimethyl-2-(6-methyl-1*H*-indol-3-yl)-2'-oxo-2',3'-dihydro-1'*H*-[1,5'-bibenzo[*d*]imidazole ]-6-sulfonamide | ¹H-NMR (500 MHz, Acetone-d₆) δ 10.37 (s, 1H), 8.68 (d, J = 8.2 Hz, 1H), 7.86 (d, J = 8.4 Hz, 1H), 7.76 (dd, J = 8.4, 1.8 Hz, 1H), 7.51 (d, J = 1.5 Hz, 1H), 7.42 (d, J = 8.1 Hz, 1H), 7.38 (d, J = 1.9 Hz, 1H), 7.29 (dd, J = 8.1, 2.0 Hz, 1H), 7.25 (s, 1H), 7.10 (dd, J = 8.3, 1.1 Hz, 1H), 6.71 (d, J = 2.9 Hz, 1H), 6.26 (d, J = 7.3 Hz, 1H), 3.52 (s, 3H), 3.41 (s, 3H), 3.39 - 3.32 (m, 1H), 2.44 (s, 3H), 1.02 (d, J = 6.5 Hz, 6H) ; m/z N.D. |
| 151 | 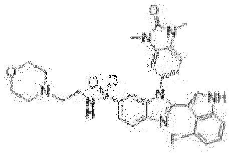 | 2-(5-fluoro-1*H-*indol-3-yl)-1',3'-dimethyl-*N*-(2-morpholinoethyl)-2'-oxo-2',3'-dihydro-1'*H*-[1,5'-bibenzo[*d*]imidazole ]-6-sulfonamide | ¹H-NMR (500 MHz, Acetone-d₆) δ 10.93 (s, 1H), 7.88 (dd, J = 8.5, 0.5 Hz, 1H), 7.80 (dd, J = 8.5, 1.8 Hz, 1H), 7.70 (d, J = 1.6 Hz, 1H), 7.41 (d, J = 2.8 Hz, 1H), 7.29 (dd, J = 6.9, 5.0 Hz, 2H), 7.21 (d, J = 8.2 Hz, 1H), 7.18 (dd, J = 8.2, 1.8 Hz, 1H), 7.13 (td, J = 8.0, 4.9 Hz, 1H), 6.78 (ddd, J = 11.1, 7.8, 0.7 Hz, 1H), 6.20 (s, 1H), 3.51 - 3.46 (m, 4H), 3.41 (s, 3H), 3.32 (s, 3H), 2.99 (dd, J = 11.6, 6.0 Hz, 2H), 2.37 (t, J = 6.3 Hz, 2H), 2.24 (s, 4H) ; m/z N.D. |
| 152 | 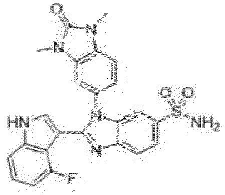 | 2-(4-fluoro-1*H-*indol-3-yl)-1',3'-dimethyl-2'-oxo-2',3'-dihydro-1'*H-*[1,5'-bibenzo[*d*]imidazole ]-6-sulfonamide | ¹H-NMR (500 MHz, Acetone-d₆) δ 10.89 (s, 1H), 7.84 (dd, J = 2.8, 1.1 Hz, 2H), 7.72 (s, 1H), 7.41 (d, J = 2.7 Hz, 1H), 7.28 (dd, J = 5.0, 3.1 Hz, 2H), 7.21 (d, J = 8.2 Hz, 1H), 7.17 (dd, J = 8.2, 1.9 Hz, 1H), 7.13 (td, J = 8.0, 4.8 Hz, 1H), 6.78 (dd, J = 11.1, 7.8 Hz, 1H), 6.48 (s, 2H), 3.41 (s, 3H), 3.32 (s, 3H) ; m/z N.D. |
| 153 | 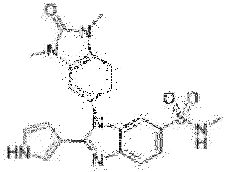 | *N*,1',3'-trimethyl-2'-oxo-2-(1*H*-pyrrol-3-yl)-2',3'-dihydro-1'*H*-[1,5'-bibenzo[*d*]imidazole ]-6-sulfonamide | ¹H-NMR (500 MHz, Acetone-d₆) δ 10.35 (s, 1H), 7.75 (d, J = 8.4 Hz, 1H), 7.68 (dd, J = 8.4, 1.7 Hz, 1H), 7.44 (d, J = 1.6 Hz, 1H), 7.38 (d, J = 8.1 Hz, 1H), 7.32 (d, J = 1.9 Hz, 1H), 7.23 (dd, J = 8.1, 2.0 Hz, 1H), 6.80 (dt, J = 3.1, 1.7 Hz, 1H), 6.77 (dd, J = 4.6, 2.6 Hz, 1H), 6.36 (dd, J = 4.2, 2.7 Hz, 1H), 6.17 (q, J = 5.3 Hz, 1H), 3.51 (s, 3H), 3.42 (s, 3H), 2.51 (d, J = 5.2 Hz, 3H) ; m/z N.D. |
| 154 | 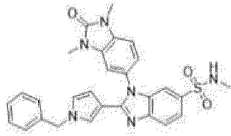 | 2-(1-benzyl-1*H-*pyrrol-3-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.73 (d, J = 8.6 Hz, 1H), 7.67 (dd, J = 8.4, 1.8 Hz, 1H), 7.43 (d, J = 1.4 Hz, 1H), 7.30 (dd, J = 12.6, 7.1 Hz, 5H), 7.19 (dd, J = 11.2, 1.9 Hz, 2H), 7.16 - 7.14 (m, J = 8.4, 1.8 Hz, 2H), 6.81 - 6.79 (m, 1H), 6.56 (t, J = 2.0 Hz, 1H), 6.35 (dd, J = 2.9, 1.8 Hz, 1H), 5.04 (s, 2H), 3.51 (s, 3H), 3.36 (s, 3H), 2.50 (d, J = 5.2 Hz, 3H) ; m/z N.D. |
| 155 | 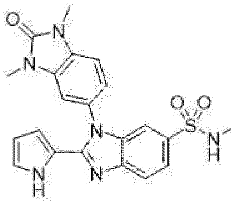 | *N*,1',3'-trimethyl-2'-oxo-2-(1*H*-pyrrol-2-yl)-2',3'-dihydro-1'*H*-[1,5'-bibenzo[*d*]imidazole ]-6-sulfonamide | ¹H-NMR (500 MHz, Acetone-d₆) δ 11.35 (s, 1H), 7.81 (d, J = 7.6 Hz, 1H), 7.75 (d, J = 7.9 Hz, 1H), 7.50 (s, 1H), 7.42 (d, J = 8.2 Hz, 1H), 7.39 (d, J = 1.8 Hz, 1H), 7.30 (dd, J = 8.2, 1.9 Hz, 1H), 7.10 (s, 1H), 6.24 (q, J = 4.8 Hz, 1H), 6.05 (s, 1H), 5.68 (s, 1H), 3.53 (s, 3H), 3.43 (s, 3H), 2.53 - 2.51 (m, 3H) ; m/z N.D. |
| 156 | 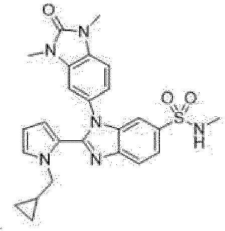 | 2-(1-(cyclopropylmethyl) -1*H*-pyrrol-2-yl)-*N*,1',3'-trimethyl-2'-oxo-2',3'-dihydro-1'*H*-[1,5'-bibenzo[*d*]imidazole ]-6-sulfonamide | ¹H-NMR (500 MHz, Acetone-d₆) δ 7.85 (d, J = 8.4 Hz, 1H), 7.74 (dd, J = 8.5, 1.8 Hz, 1H), 7.52 (d, J = 1.6 Hz, 1H), 7.33 (d, J = 8.2 Hz, 1H), 7.29 (d, J = 1.9 Hz, 1H), 7.19 (dd, J = 8.2, 2.0 Hz, 1H), 7.09 - 7.06 (m, 1H), 6.25 - 6.20 (m, 1H), 5.93 (dd, J = 3.9, 2.6 Hz, 1H), 5.83 (dd, J = 3.9, 1.7 Hz, 1H), 4.53 (d, J = 7.1 Hz, 2H), 3.49 (s, 3H), 3.40 (s, 3H), 2.53 (d, J = 5.2 Hz, 3H), 1.45 - 1.36 (m, 1H), 0.53 - 0.48 (m, 2H), 0.40 - 0.36 (m, 2H) ; m/z N.D. |
| 157 | 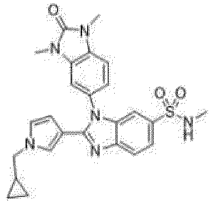 | 2-(1-(cyclopropylmethyl) -1*H*-pyrrol-3-yl)-*N*,1',3'-trimethyl-2'-oxo-2',3'-dihydro-1'*H*-[1,5'-bibenzo[*d*]imidazole ]-6-sulfonamide | ¹H-NMR (500 MHz, Acetone-d₆) δ 7.74 (d, J = 8.5 Hz, 1H), 7.68 (dd, J = 8.4, 1.8 Hz, 1H), 7.45 (s, 1H), 7.39 (d, J = 8.2 Hz, 1H), 7.32 (d, J = 2.0 Hz, 1H), 7.23 (dd, J = 8.1, 2.0 Hz, 1H), 6.87 (s, 1H), 6.76 - 6.70 (m, 1H), 6.22 - 6.18 (m, 1H), 6.18 - 6.14 (m, 1H), 3.71 (d, J = 7.1 Hz, 2H), 3.51 (s, 3H), 3.42 (s, 3H), 2.51 (d, J = 5.2 Hz, 3H), 1.11 - 1.08 (m, 1H), 0.48 (d, J = 7.8 Hz, 2H), 0.25 (d, J = 4.9 Hz, 2H) ; m/z N.D. |
| 158 | 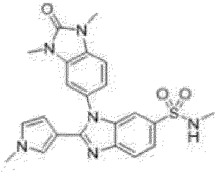 | *N*,1',3'-trimethyl-2-(1-methyl-1*H-*pyrrol-3-yl)-2'-oxo-2',3'-dihydro-1'*H-*[1,5'-bibenzo[*d*]imidazole ]-6-sulfonamide | ¹H-NMR (500 MHz, Acetone-d₆) δ 7.73 (d, J = 8.4 Hz, 1H), 7.67 (d, J = 1.7 Hz, 1H), 7.43 (d, J = 1.7 Hz, 1H), 7.37 (d, J = 8.1 Hz, 1H), 7.31 (d, J = 1.9 Hz, 1H), 7.21 (dd, J = 8.1, 2.0 Hz, 1H), 6.80 (t, J = 1.9 Hz, 1H), 6.62 (t, 1H), 6.19 - 6.14 (m, 2H), 3.59 (s, 3H), 3.51 (s, 3H), 3.42 (s, 3H), 2.51 (d, J = 5.2 Hz, 3H) ; m/z N.D. |
| 159 | 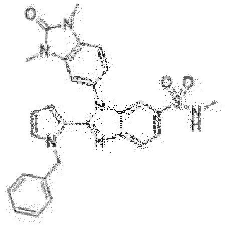 | 2-(1-benzyl-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.87 (d, J = 8.0 Hz, 1H), 7.74 (dd, J = 8.5, 1.8 Hz, 1H), 7.50 (d, J = 1.3 Hz, 1H), 7.30 (d, J = 8.1 Hz, 1H), 7.27 (dd, J = 8.0, 6.4 Hz, 2H), 7.23 (d, J = 7.2 Hz, 1H), 7.13 (d, J = 1.7 Hz, 2H), 7.12 (s, 1H), 7.02 (dd, J = 8.1, 2.0 Hz, 1H), 6.97 (d, J = 1.9 Hz, 1H), 6.23 (q, J = 4.6 Hz, 1H), 6.02 (dd, J = 3.9, 2.7 Hz, 1H), 5.96 (s, 2H), 5.91 (dd, J = 3.9, 1.7 Hz, 1H), 3.49 (s, 3H), 3.37 (s, 3H), 2.53 (d, J = 5.2 Hz, 3H) ; m/z N.D. |
| 160 | 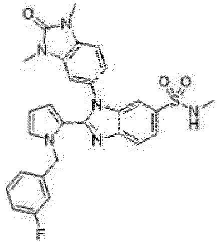 | 2-(1-(3-fluorobenzyl)-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.84 (dd, 1H), 7.72 (dd, J = 8.5, 1.8 Hz, 1H), 7.48 (d, J = 1.3 Hz, 1H), 7.35 - 7.27 (m, 2H), 7.14 (dd, J = 2.6, 1.7 Hz, 1H), 7.06 (dd, J = 7.0, 1.9 Hz, 2H), 6.99 - 6.94 (m, 2H), 6.85 (dt, 1H), 6.22 (dd, J = 10.3, 5.1 Hz, 1H), 6.03 (dd, J = 3.9, 2.7 Hz, 1H), 6.00 (s, 2H), 5.91 (dd, J = 3.9, 1.7 Hz, 1H), 3.48 (s, 3H), 3.37 (s, 3H), 2.50 (d, J = 5.2 Hz, 3H) ; m/z N.D. |
| 161 | 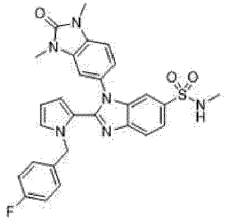 | 2-(1-(4-fluorobenzyl)-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.85 (d, J = 8.5 Hz, 1H), 7.73 (dd, J = 8.5, 1.7 Hz, 1H), 7.49 (d, J = 1.4 Hz, 1H), 7.31 (d, J = 8.1 Hz, 1H), 7.21 (dd, J = 8.7, 5.4 Hz, 2H), 7.12 (dd, J = 2.6, 1.7 Hz, 1H), 7.05 - 7.01 (m, 4H), 6.23 (d, J = 5.4 Hz, 1H), 6.01 (dd, J = 3.9, 2.7 Hz, 1H), 5.94 (s, 2H), 5.89 (dd, J = 3.9, 1.7 Hz, 1H), 3.48 (s, 3H), 3.37 (s, 3H), 2.51 (d, J = 5.2 Hz, 3H) ; m/z N.D. |
| 162 | 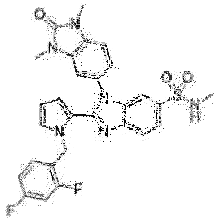 | 2-(1-(2,4-difluorobenzyl)-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.82 (d, J = 8.5 Hz, 1H), 7.72 (dd, J = 8.5, 1.7 Hz, 1H), 7.49 (d, J = 1.4 Hz, 1H), 7.33 (d, J = 8.1 Hz, 1H), 7.16 (d, J = 1.9 Hz, 1H), 7.09 (dd, J = 8.1, 1.9 Hz, 2H), 7.06 - 6.87 (m, 3H), 6.23 (d, J = 5.2 Hz, 1H), 6.03 (dd, J = 3.9, 2.7 Hz, 1H), 6.01 (s, 2H), 5.90 (dd, J = 4.0, 1.7 Hz, 1H), 3.49 (s, 3H), 3.39 (s, 3H), 2.51 (d, J = 5.2 Hz, 3H) ; m/z N.D. |
| 163 | 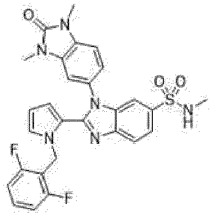 | 2-(1-(2,6-difluorobenzyl)-1*H-*pyrrol-2-yl)-*N*,1',3'-trimethyl-2'-oxo-2',3'-dihydro-1'*H-*[1,5'-bibenzo[*d*]imidazole]-6-sulfonamide | ¹H-NMR (500 MHz, Acetone-d₆) δ 7.91 (d, J = 8.5 Hz, 1H), 7.77 (dd, J = 8.5, 1.7 Hz, 1H), 7.58 (d, J = 1.4 Hz, 1H), 7.43 (tt, J = 8.4, 6.5 Hz, 1H), 7.29 (d, J = 8.1 Hz, 1H), 7.15 (d, J = 1.8 Hz, 1H), 7.12 (dd, J = 8.1, 2.0 Hz, 1H), 7.07 - 7.00 (m, 2H), 6.91 (d, J = 0.9 Hz, 1H), 6.26 (q, J = 10.2, 5.0 Hz, 1H), 6.02 (s, 2H), 5.96 (dd, J = 3.9, 2.8 Hz, 1H), 5.89 (dd, J = 3.9, 1.6 Hz, 1H), 3.46 (s, 3H), 3.35 (s, 3H), 2.55 - 2.53 (m, 3H) ; m/z N.D. |
| 164 | 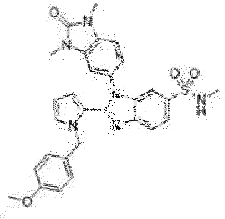 | 2-(1-(4-methoxybenzyl)-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.87 (dd, J = 8.5, 0.4 Hz, 1H), 7.74 (dd, J = 8.5, 1.8 Hz, 1H), 7.51 (dd, J = 1.7, 0.4 Hz, 1H), 7.27 (d, J = 8.1 Hz, 1H), 7.11 - 7.04 (m, 3H), 7.02 (dd, J = 8.2, 2.0 Hz, 1H), 6.93 (d, J = 1.9 Hz, 1H), 6.83 - 6.77 (m, 2H), 6.24 (dd, J = 10.5, 5.3 Hz, 1H), 5.97 (dd, J = 3.9, 2.7 Hz, 1H), 5.87 (dd, J = 3.9, 1.7 Hz, 1H), 5.84 (s, 2H), 3.71 (s, 3H), 3.46 (s, 3H), 3.33 (s, 3H), 2.52 (d, J = 5.2 Hz, 3H) ; m/z N.D. |
| 165 | 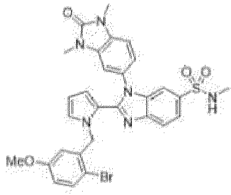 | 2-(1-(2-bromo-5-methoxybenzyl)-1*H-*pyrrol-2-yl)-*N*,1',3'-trimethyl-2'-oxo-2',3'-dihydro-1'*H-*[1,5'-bibenzo[*d*]imidazole ]-6-sulfonamide | ¹H-NMR (500 MHz, Acetone-d₆) δ 7.78 (dd, J = 8.5, 0.6 Hz, 1H), 7.69 (dd, J = 8.5, 1.8 Hz, 1H), 7.47 (dd, J = 1.7, 0.6 Hz, 1H), 7.46 (d, J = 8.7 Hz, 1H), 7.34 (dd, J = 7.8, 0.8 Hz, 1H), 7.16 - 7.12 (m, 3H), 6.75 (dd, J = 8.7, 3.1 Hz, 1H), 6.21 (dt, J = 10.4, 5.0 Hz, 1H), 6.09 (dd, J = 4.0, 2.7 Hz, 1H), 6.03 (d, J = 3.1 Hz, 1H), 5.99 (d, J = 3.8 Hz, 2H), 5.96 (dd, J = 3.9, 1.7 Hz, 1H), 3.63 (s, 3H), 3.50 (s, 3H), 3.40 (s, 3H), 2.49 (d, J = 5.2 Hz, 3H) ; m/z N.D. |

### Experimental Example 1. CBP Inhibitory Activity

A time resolved fluorescence resonance energy transfer (TR-FRET) experiment was used to verify the activity of CBP bromodomain.

The assay was performed in a final volume of 14 µl, optimized based on a CREBBP time resolved fluorescence resonance energy transfer (TR-FRET) assay kit (BPS Bioscience, cat. 32619) to maximize an assay window, and performed in a final volume of 14 µl, while maximizing the assay window. A TR-FRET fluorescence acceptor and a Tb donor were diluted in an assay buffer containing 2-[4-(2-hydroxyethyl)piperazin-1-yl]ethanesulfonic acid (hereafter, referred to as an assay buffer, BPS Bioscience, cat. 33012), and 3 µl of each solution was transferred to all wells of a white 384-low-volume well plate (Corning, cat. 4513). 2 µl of 4X Ac-H4 peptide (final concentration 85 nM) was transferred to high control wells and test wells. Each compound was prepared at 10 concentration points by serially diluting 3-fold from the highest concentration in a dimethyl sulfoxide solvent. The compound at each concentration was then diluted in the assay buffer to prepare a diluted compound solution that was 4-fold more concentrated than the final concentration. 2 µl of each compound or dimethyl sulfoxide prepared in this way was distributed into an assay plate so that the final test concentration was a minimum of 0.5 µM to a maximum of 10 µM (final dimethyl sulfoxide concentration 1%). 6 µL of 1X assay buffer was added to low control wells and 2 µl was added to high control and test wells. Then, 2 µl of 4X CBP BRD protein (final concentration 7.5 nM, BPS bioscience, cat 31128) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias). For data analysis, the values of each well were obtained using a TR-FRET ratio ((Signal 665 nm/Signal 615 nm) × 20000). Data analysis was performed using the TR-FRET ratio ((Signal 665 nm/Signal 615 nm) × 20000). Data were reported as percent inhibition compared to control wells based on the following equation: %Activity = ((FRETcompound - FRETLow av)/(FRETHigh av - FRETLow av)) × 100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

### Experimental Example 2. BRD4 Inhibitory Activity

BRD4 inhibitory activity was measured using a time resolved fluorescence resonance energy transfer (TR-FRET) experiment to verify the activity of BRD4 bromodomain.

The assay was performed in a final volume of 14 µl, optimized based on a CREBBP time resolved fluorescence resonance energy transfer (TR-FRET) assay kit (BPS Bioscience, cat. 32613) to maximize an assay window, and performed in a final volume of 14 µl, while maximizing the assay window. A TR-FRET fluorescence acceptor and a Tb donor were diluted in an assay buffer containing 2-[4-(2-hydroxyethyl)piperazin-1-yl]ethanesulfonic acid (hereafter, referred to as an assay buffer, BPS Bioscience, cat. 33012), and 3 µl of each solution was transferred to all wells of a white 384-low-volume well plate (Corning, cat. 4513). 2 µl of 4X Ac-H4 peptide (final concentration 15 nM) was transferred to high control wells and test wells. Each compound was prepared at 10 concentration points by serially diluting 3-fold from the highest concentration in a dimethyl sulfoxide solvent. The compound at each concentration was then diluted in the assay buffer to prepare a diluted compound solution that was 4-fold more concentrated than the final concentration. 2 µl of each compound or dimethyl sulfoxide prepared in this way was distributed into an assay plate so that the final test concentration was a minimum of 0.5 µM to a maximum of 10 µM (final DMSO concentration 1%). 6 µL of 1X assay buffer was added to low control wells and 2 µl was added to high control and test wells. Then, 2 µl of 4X BRD4 (BD1) protein (final concentration 7.5 nM, BPS bioscience, cat 31040) was added to initiate a PPI reaction. The plate was reacted at room temperature for 2 hours before measuring the signal. The TR-FRET signal was measured using an EnVision Multilabel plate reader (PerkinElmer; Mirror LNACE/DELFIA Dual/Bias). For data analysis, the values of each well were obtained using the TR-FRET ratio ((Signal 665 nm/Signal 615 nm) × 20000). Data analysis was performed using the TR-FRET ratio ((Signal 665 nm/Signal 615 nm) × 20000). Data were reported as percent inhibition compared to control wells based on the following equation: %Activity = ((FRETcompound - FRETLow av)/(FRETHigh av - FRETLow av)) × 100. IC50 analysis was determined by standard 4 parameter curve fitting according to an algorithm using GraphPad Prism 7.0 (GraphPad Software Inc., La Jolla, CA, USA). All data are expressed as mean ± standard error of the mean (SEM).

The results according to Experimental Examples 1 and 2 are shown in Tables 3 and 4 below.

In Tables 3 and 4 below, a case where the activity value is 0 to 500 nM is indicated as "+++", a case with greater than 500 and 5 µM or less is indicated as "++", and a case with greater than 5 µM is indicated as "+."

**[Table 3]**

| **Example** | **CBP BRD IC₅₀** | **Example** | **CBP BRD IC₅₀** |
|---|---|---|---|
| 1 | ++ | 84 | + |
| 2 | ++ | 85 | + |
| 3 | ++ | 86 | + |
| 4 | + | 87 | + |
| 5 | ++ | 88 | ++ |
| 6 | + | 89 | + |
| 7 | ++ | 90 | + |
| 8 | ++ | 91 | + |
| 9 | + | 92 | + |
| 10 | +++ | 93 | + |
| 11 | ++ | 94 | + |
| 12 | + | 95 | + |
| 13 | ++ | 96 | +++ |
| 14 | + | 97 | + |
| 15 | ++ | 98 | + |
| 16 | +++ | 99 | + |
| 17 | +++ | 100 | + |
| 18 | ++ | 101 | + |
| 19 | +++ | 102 | + |
| 20 | ++ | 103 | + |
| 21 | + | 104 | + |
| 22 | ++ | 105 | + |
| 23 | + | 106 | + |
| 24 | + | 107 | + |
| 25 | + | 108 | + |
| 26 | + | 109 | + |
| 27 | + | 110 | + |
| 28 | + | 111 | + |
| 29 | ++ | 112 | + |
| 30 | + | 113 | + |
| 31 | + | 114 | + |
| 32 | +++ | 115 | + |
| 33 | +++ | 116 | ++ |
| 34 | + | 117 | + |
| 35 | + | 118 | + |
| 36 | + | 119 | + |
| 37 | +++ | 120 | + |
| 38 | +++ | 121 | + |
| 39 | ++ | 122 | + |
| 40 | ++ | 123 | ++ |
| 41 | + | 124 | + |
| 42 | ++ | 125 | + |
| 43 | + | 126 | ++ |
| 44 | + | 127 | N.D |
| 45 | + | 128 | ++ |
| 46 | + | 129 | ++ |
| 47 | ++ | 130 | N.D |
| 48 | +++ | 131 | +++ |
| 49 | + | 132 | ++ |
| 50 | ++ | 133 | ++ |
| 51 | ++ | 134 | ++ |
| 52 | ++ | 135 | N.D |
| 53 | + | 136 | + |
| 54 | + | 137 | ++ |
| 55 | + | 138 | + |
| 56 | ++ | 139 | ++ |
| 57 | + | 140 | + |
| 58 | + | 141 | + |
| 59 | + | 142 | + |
| 60 | ++ | 143 | ++ |
| 61 | ++ | 144 | ++ |
| 62 | + | 145 | ++ |
| 63 | ++ | 146 | ++ |
| 64 | ++ | 147 | +++ |
| 65 | ++ | 148 | ++ |
| 66 | ++ | 149 | ++ |
| 67 | ++ | 150 | ++ |
| 68 | ++ | 151 | ++ |
| 69 | + | 152 | +++ |
| 70 | ++ | 153 | ++ |
| 71 | + | 154 | + |
| 72 | ++ | 155 | ++ |
| 73 | ++ | 156 | ++ |
| 74 | + | 157 | ++ |
| 75 | + | 158 | + |
| 76 | + | 159 | + |
| 77 | + | 160 | + |
| 78 | ++ | 161 | + |
| 79 | + | 162 | + |
| 80 | ++ | 163 | + |
| 81 | + | 164 | ++ |
| 82 | + | 165 | + |
| 83 | + | | |

**[Table 4]**

| **Example** | **BRD4 (BD1) IC₅₀** | **Example** | **BRD4 (BD1) IC₅₀** |
|---|---|---|---|
| 1 | N.D | 84 | N.D |
| 2 | ++ | 85 | + |
| 3 | ++ | 86 | + |
| 4 | N.D | 87 | + |
| 5 | + | 88 | N.D |
| 6 | N.D | 89 | + |
| 7 | ++ | 90 | + |
| 8 | ++ | 91 | + |
| 9 | N.D | 92 | + |
| 10 | ++ | 93 | + |
| 11 | + | 94 | + |
| 12 | N.D | 95 | + |
| 13 | N.D | 96 | + |
| 14 | N.D | 97 | + |
| 15 | N.D | 98 | + |
| 16 | ++ | 99 | + |
| 17 | + | 100 | + |
| 18 | + | 101 | + |
| 19 | ++ | 102 | + |
| 20 | + | 103 | + |
| 21 | N.D | 104 | + |
| 22 | + | 105 | + |
| 23 | N.D | 106 | N.D |
| 24 | N.D | 107 | N.D |
| 25 | N.D | 108 | N.D |
| 26 | N.D | 109 | N.D |
| 27 | N.D | 110 | N.D |
| 28 | N.D | 111 | N.D |
| 29 | + | 112 | N.D |
| 30 | N.D | 113 | N.D |
| 31 | N.D | 114 | N.D |
| 32 | ++ | 115 | N.D |
| 33 | ++ | 116 | N.D |
| 34 | N.D | 117 | N.D |
| 35 | N.D | 118 | N.D |
| 36 | N.D | 119 | N.D |
| 37 | ++ | 120 | N.D |
| 38 | + | 121 | N.D |
| 39 | + | 122 | N.D |
| 40 | + | 123 | N.D |
| 41 | N.D | 124 | N.D |
| 42 | N.D | 125 | N.D |
| 43 | N.D | 126 | N.D |
| 44 | N.D | 127 | N.D |
| 45 | N.D | 128 | N.D |
| 46 | N.D | 129 | + |
| 47 | N.D | 130 | N.D |
| 48 | ++ | 131 | ++ |
| 49 | N.D | 132 | N.D |
| 50 | N.D | 133 | N.D |
| 51 | ++ | 134 | N.D |
| 52 | + | 135 | N.D |
| 53 | N.D | 136 | N.D |
| 54 | + | 137 | N.D |
| 55 | N.D | 138 | N.D |
| 56 | N.D | 139 | N.D |
| 57 | N.D | 140 | N.D |
| 58 | + | 141 | N.D |
| 59 | N.D | 142 | N.D |
| 60 | N.D | 143 | + |
| 61 | N.D | 144 | N.D |
| 62 | N.D | 145 | N.D |
| 63 | N.D | 146 | + |
| 64 | + | 147 | ++ |
| 65 | + | 148 | N.D |
| 66 | ++ | 149 | N.D |
| 67 | N.D | 150 | N.D |
| 68 | N.D | 151 | ++ |
| 69 | N.D | 152 | ++ |
| 70 | ++ | 153 | + |
| 71 | N.D | 154 | + |
| 72 | N.D | 155 | + |
| 73 | + | 156 | ++ |
| 74 | + | 157 | + |
| 75 | + | 158 | + |
| 76 | N.D | 159 | ++ |
| 77 | N.D | 160 | ++ |
| 78 | + | 161 | ++ |
| 79 | N.D | 162 | + |
| 80 | + | 163 | + |
| 81 | N.D | 164 | +++ |
| 82 | N.D | 165 | ++ |
| 83 | N.D | | |

## Claims

1. A compound represented by the following Formula I: 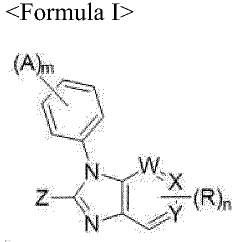 where
W, X and Y are each independently C or N,
n is an integer from 0 to 3, where if n is an integer of 2 or more, Rs are the same or different, and Rs are each independently NR^{a}R^{b}, SO₂NHR^{c}, CONHR^{d}, SO₂R^{e}, CO₂R^{f}, halogen, C₁₋₄ alkyl, C₁₋₄ haloalkyl, or 3- to 9-membered heteroaryl, and R^{a} to R^{f} are each independently selected from the group consisting of hydrogen, C₁₋₆ alkyl, hydroxy, C₁₋₅ alkoxy, and 3- to 10-membered heterocycloalkyl,
m is an integer from 0 to 3, where if m is an integer of 2 or more, As are the same or different, and As are each independently amide, hydroxy, C₁₋₅ alkoxy, 3- to 10-membered heterocycloalkyl, 3- to 9-membered heteroaryl or halogen, or two As are fused together to form with an atom bonded thereto, a 3- to 10-membered heterocycloalkyl, or 3- to 9-membered heteroaryl, and
Z is hydrogen, amide, hydroxy, C₁₋₅ alkoxy, C₁₋₄ alkyl, C₆₋₁₄ aryl, 3- to 10-membered heterocycloalkyl, or 3- to 14-membered heteroaryl,
where R, R^{a} to R^{f}, A and Z are each substituted or unsubstituted with Substituent Group I:
Substituent Group I: substituted or unsubstituted C₁₋₁₀ alkyl, substituted or unsubstituted C₂₋₁₀ alkenyl, substituted or unsubstituted C₃₋₂₀ carbocycle, substituted or unsubstituted C₃₋₂₀ cycloalkyl, substituted or unsubstituted C₆₋₃₀ aryl, substituted or unsubstituted 3- to 30-membered heterocycle, substituted or unsubstituted C₁₋₁₀ alkoxy, substituted or unsubstituted C₂₋₁₀ alkenyloxy, substituted or unsubstituted C₃₋₁₀ cycloalkyloxy, substituted or unsubstituted C₆₋₃₀ aryloxy, substituted or unsubstituted 3-to 30-membered heterocycleoxy, substituted or unsubstituted C₁₋₁₀ alkylketone, substituted or unsubstituted C₂₋₁₀ alkenylketone, substituted or unsubstituted C₃₋₁₀ cycloalkylketone, substituted or unsubstituted C₆₋₃₀ arylketone, substituted or unsubstituted 3- to 30-membered heterocycleketone, substituted or unsubstituted C₁₋₁₀ alkylthio, substituted or unsubstituted C₂₋₁₀ alkenylthio, substituted or unsubstituted C₃₋₁₀ cycloalkylthio, substituted or unsubstituted C₆₋₃₀ arylthio, substituted or unsubstituted 3- to 30-membered heterocyclethio, aldehyde, carboxy, halogen, substituted or unsubstituted C₁₋₁₀ haloalkyl, hydroxy, substituted or unsubstituted amino, substituted or unsubstituted imine, cyano, substituted or unsubstituted nitro, substituted or unsubstituted amide, thiol, sulfone and phosphoric acid,
where a C₁₋₁₀ alkyl moiety is interposed by a heteroatom group selected from - O-, -S-, -SO-, -SO₂-, -NR^{g}- (R^{g} is hydrogen or substituted or unsubstituted C₁₋₁₀ alkyl), - N=, =N-, -POR^{g}- and -PO₄R^{g}-, and a heterocycle moiety comprises at least one of S, N, P and O as a ring-forming atom.

2. The compound according to claim 1, wherein R, R^{a} to R^{f}, A and Z are each substituted or unsubstituted with Substituent Group I-1:
Substituent Group I-1: substituted or unsubstituted C₁₋₅ alkyl, substituted or unsubstituted C₃₋₅ cycloalkyl, substituted or unsubstituted amino, substituted or unsubstituted C₆₋₈ aryl, substituted or unsubstituted 3- to 9-membered heterocycloalkyl, substituted or unsubstituted 3- to 11-membered heteroaryl, substituted or unsubstituted C₁₋₃ haloalkyl, substituted or unsubstituted C₁₋₃ alkoxy, hydroxy, halogen, aldehyde, cyano, and substituted or unsubstituted amide.

3. The compound according to claim 1, wherein the compound of Formula I is represented by any one of the following Formulas I-1 to I-5: 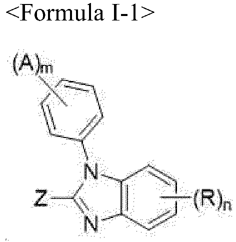 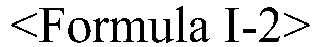 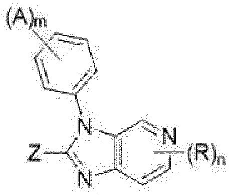 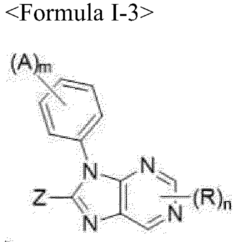 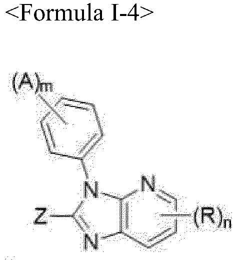 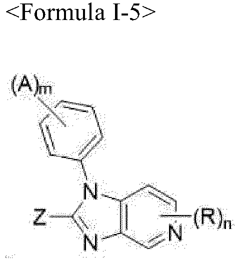 where
R, A, Z, n and m are the same as defined in claim 1.

4. The compound according to claim 3, wherein Formula I-1 is represented by any one of the following Formulas I-1-1 to 1-1-6: 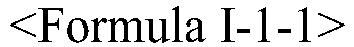 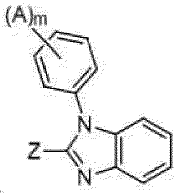 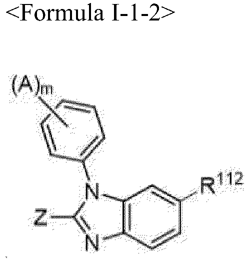 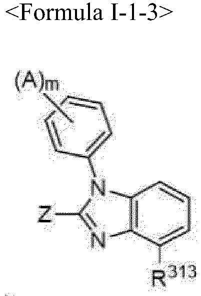 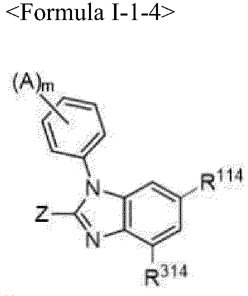 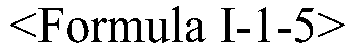 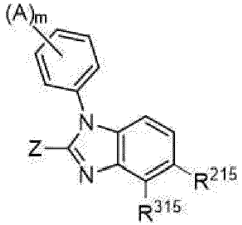 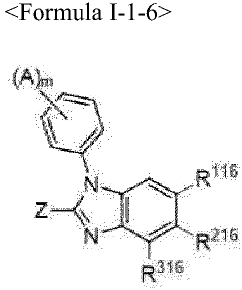 where
R¹¹², R³¹³, R¹¹⁴, R³¹⁴, R²¹⁵, R³¹⁵, R¹¹⁶, R²¹⁶ and R³¹⁶ are the same or different, are each independently SO₂NHR^{c}, CONHR^{d}, SO₂R^{e}, CO₂R^{f}, C₁₋₄ haloalkyl, or 3- to 9-membered heteroaryl, and are each substituted or unsubstituted with Substituent Group I, and
R^{c}, R^{d}, R^{e}, R^{f}, A, Z and m are the same as defined in claim 1.

5. The compound according to claim 3, wherein Formula I-2 is represented by any one of the following Formulas I-2-1 to I-2-3: 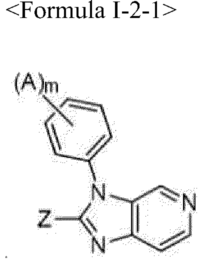 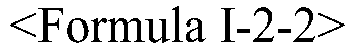 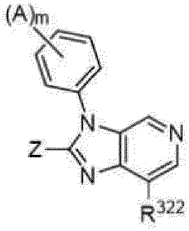 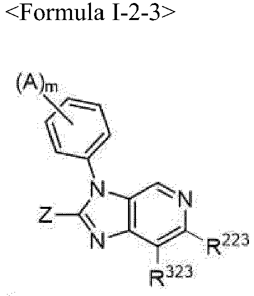 where
R³²², R²²³ and R³²³ are the same or different, are each independently NR^{a}R^{b}, SO₂NHR^{c}, CONHR^{d}, C₁₋₄ alkyl, or 3- to 9-membered heteroaryl, and are each substituted or unsubstituted with Substituent Group I, and
R^{a}, R^{b}, R^{c}, R^{d}, A, Z and m are the same as defined in claim 1.

6. The compound according to claim 3, wherein Formula I-3 is represented by any one of the following Formulas I-3-1 to I-3-4: 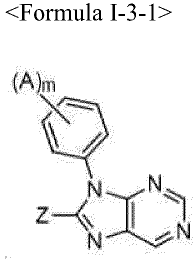 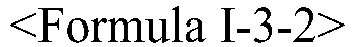 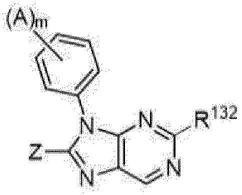 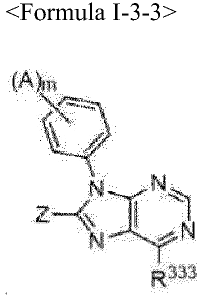 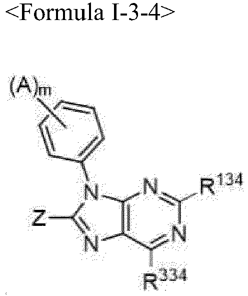 where
R¹³², R³³³, R¹³⁴ and R³³⁴ are the same or different, are each independently NR^{a}R^{b} or halogen, and are each substituted or unsubstituted with Substituent Group I, and
R^{a}, R^{b}, A, Z and m are the same as defined in claim 1.

7. The compound according to claim 3, wherein Formula I-4 is represented by any one of the following Formulas I-4-1 to I-4-6: 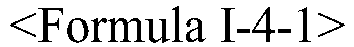 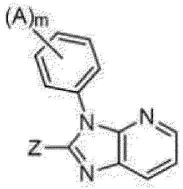 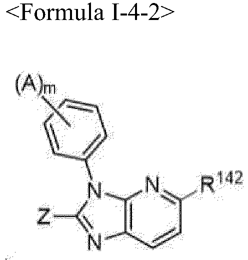 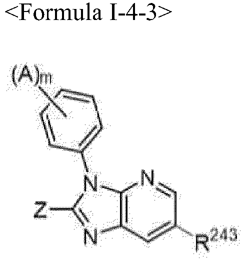 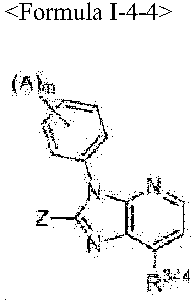 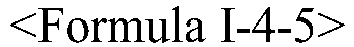 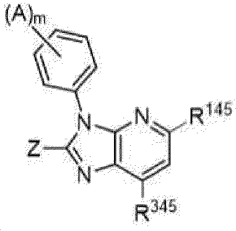 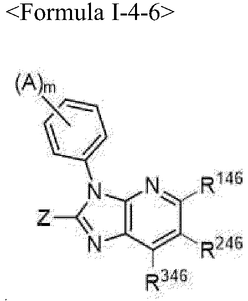 where
R¹⁴², R²⁴³, R³⁴⁴, R¹⁴⁵, R³⁴⁵, R¹⁴⁶, R²⁴⁶ and R³⁴⁶ are the same or different, are each independently NR^{a}R^{b} or CONHR^{d}, and are each substituted or unsubstituted with Substituent Group I, and
R^{a}, R^{b}, R^{d}, A, Z and m are the same as defined in claim 1.

8. The compound according to claim 3, wherein Formula I-5 is represented by any one of the following Formulas I-5-1 to I-5-3: 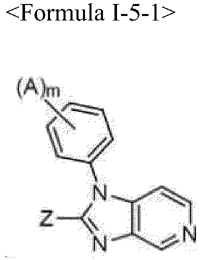 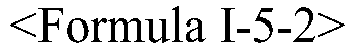 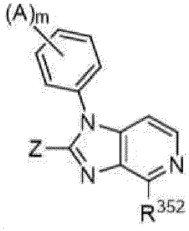 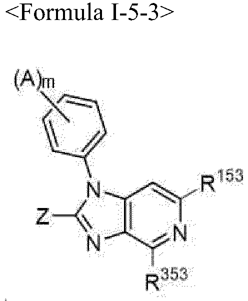 where
R³⁵², R¹⁵³ and R³⁵³ are the same or different, are each independently NR^{a}R^{b} or halogen, and are each substituted or unsubstituted with Substituent Group I, and
R^{a}, R^{b}, A, Z and m are the same as defined in claim 1.

9. The compound according to claim 1, wherein
R is each independently NR^{a}R^{b}, SO₂NHR^{c}, CONHR^{d}, SO₂R^{e}, CO₂R^{f}, chloro, C₁₋₂ haloalkyl, or pyrazolyl,
where R^{a} to R^{f} are each independently hydrogen, methyl, ethyl, propyl, pyrrolidyl or morpholinyl, and R^{a} to R^{f} are each substituted or unsubstituted with Substituent Group I-1-1, and
R is substituted or unsubstituted with Substituent Group I-1-2:
Substituent Group I-1-1: substituted or unsubstituted C₁₋₃ alkyl, substituted or unsubstituted C₃₋₅ cycloalkyl, substituted or unsubstituted amino, substituted or unsubstituted C₆₋₈ aryl, substituted or unsubstituted 3- to 9-membered heterocycloalkyl, and substituted or unsubstituted 3- to 11-membered heteroaryl;
Substituent Group I-1-2: substituted or unsubstituted C₁₋₃ alkyl.

10. The compound according to claim 1, wherein
A is acetamide, carbamide, hydroxy, isoxazolyl, hydropyranyl or tetrahydropyranyl, or imidazolidinonyl, lactamyl, piperazinedionyl or pyrrolyl, which is formed by fusing two As from each other together with an atom bonded thereto,
A is substituted or unsubstituted with Substituent Group I-1-3:
Substituent Group I-1-3: substituted or unsubstituted C₁₋₄ alkyl, substituted or unsubstituted C₃₋₅ cycloalkyl, and substituted or unsubstituted amide.

11. The compound according to claim 1, wherein Z is hydrogen, methyl, hydroxy, amide, phenyl, naphthalenyl, tetrahydrofuranyl, lactamyl, tetrahydropyranyl, pyrrolyl, thiazolyl, imidazolyl, thiophenyl, isoxazolyl, pyrazolyl, pyridinyl, benzimidazolyl, indolyl, azaindolyl, indazolyl, benzofuranyl, benzothiophenyl, pyrazolopyridinyl, pyrazolopyrimidinyl, pyrrolopyridinyl, quinolinyl or isoquinolinyl,
Z is substituted or unsubstituted with Substituent Group I-1-4:
Substituent Group I-1-4: substituted or unsubstituted C₁₋₅ alkyl, substituted or unsubstituted C₃₋₅ cycloalkyl, C₁₋₃ haloalkyl, substituted or unsubstituted C₆₋₈ aryl, substituted or unsubstituted 3- to 8-membered heteroaryl, substituted or unsubstituted 4-to 8-membered heterocycloalkyl, substituted or unsubstituted 3- to 8-membered heterocycloalkenyl, substituted or unsubstituted C₁₋₃ alkoxy, hydroxy, halogen, aldehyde, cyano, and substituted or unsubstituted amide.

12. The compound according to claim 1, being any one selected from the following group:
N,1',3'-trimethyl-2-(1-methyl-1H-pyrazol-4-yl)-2'-oxo-2',3'-dihydro-1'H-[1,5'-bibenzo[d]imidazole]-6-sulfonamide,
2-(5-fluoronaphthalen-1-yl)-N,1',3'-trimethyl-2'-oxo-2',3'-dihydro-1'H-[1,5'-bibenzo[d]imidazole]-6-sulfonamide,
2-(4-hydroxyphenyl)-N,1',3'-trimethyl-2'-oxo-2',3'-dihydro-1'H-[1,5'-bibenzo[d]imidazole]-6-sulfonamide,
N,1',3'-trimethyl-2'-oxo-2-(tetrahydrofuran-3-yl)-2',3'-dihydro-1'H-[1,5'-bibenzo[d]imidazole]-6-sulfonamide,
N,1',3'-trimethyl-2'-oxo-2-(quinolin-8-yl)-2',3'-dihydro-1'H-[1,5'-bibenzo[d]imidazole]-6-sulfonamide,
2-(1H-indazol-4-yl)-N,1',3'-trimethyl-2'-oxo-2',3'-dihydro-1'H-[1,5'-bibenzo[d]imidazole]-6-sulfonamide,
N,1',3'-trimethyl-2-(2-methyl-3-(trifluoromethyl)phenyl)-2'-oxo-2',3'-dihydro-1'H-[1,5'-bibenzo[d]imidazole]-6-sulfonamide,
2-(2,3-dimethylphenyl)-N,1',3'-trimethyl-2'-oxo-2',3'-dihydro-1'H-[1,5'-bibenzo[d]imidazole]-6-sulfonamide,
2-(3-cyclopropyl-1-methyl-1H-pyrazol-4-yl)-N,1',3'-trimethyl-2'-oxo-2',3'-dihydro-1'H-[1,5'-bibenzo[d]imidazole]-6-sulfonamide,
2-(4-chloro-1H-indol-3-yl)-N,1',3'-trimethyl-2'-oxo-2',3'-dihydro-1'H-[1,5'-bibenzo[d]imidazole]-6-sulfonamide,
N,1',3'-trimethyl-2-(2-methylthiophen-3-yl)-2'-oxo-2',3'-dihydro-1'H-[1,5'-bibenzo[d]imidazole]-6-sulfonamide,
2-(3-methoxynaphthalen-2-yl)-N,1',3'-trimethyl-2'-oxo-2',3'-dihydro-1'H-[1,5'-bibenzo[d]imidazole]-6-sulfonamide,
2-(isoquinolin-1-yl)-N,1',3'-trimethyl-2'-oxo-2',3'-dihydro-1'H-[1,5'-bibenzo[d]imidazole]-6-sulfonamide,
2-(2,2-dimethyltetrahydro-2H-pyran-4-yl)-N,1',3'-trimethyl-2'-oxo-2',3'-dihydro-1'H-[1,5'-bibenzo[d]imidazole]-6-sulfonamide,
N,1',3'-trimethyl-2'-oxo-2-(pyrazolo[1,5-a]pyrimidin-3-yl)-2',3'-dihydro-1'H-[1,5'-bibenzo[d]imidazole]-6-sulfonamide,
2-(4-hydroxynaphthalen-1-yl)-N,1',3'-trimethyl-2'-oxo-2',3'-dihydro-1'H-[1,5'-bibenzo[d]imidazole]-6-sulfonamide,
N,1',3'-trimethyl-2-(1-methyl-1H-indazol-7-yl)-2'-oxo-2',3'-dihydro-1'H-[1,5'-bibenzo[d]imidazole]-6-sulfonamide,
N,1',3'-trimethyl-2'-oxo-2-(thiazol-5-yl)-2',3'-dihydro-1'H-[1,5'-bibenzo[d]imidazole]-6-sulfonamide,
2-(4-chloro-1H-pyrrolo[2,3-b]pyridin-3-yl)-N,1',3'-trimethyl-2'-oxo-2',3'-dihydro-1'H-[1,5'-bibenzo[d]imidazole]-6-sulfonamide,
2-(isoquinolin-8-yl)-N,1',3'-trimethyl-2'-oxo-2',3'-dihydro-1'H-[1,5'-bibenzo[d]imidazole]-6-sulfonamide,
5-(6-((3-(azepan-1-yl)propyl)(methyl)amino)-8-(1H-pyrrolo[2,3-b]pyridin-2-yl)-9H-purin-9-yl)-1,3-dimethyl-1,3-dihydro-2H-benzo[d]imidazol-2-one,
5-(6-((3-azepan-1-yl)propyl)(methyl)amino)-8-(6-methyl-1H-indol-3-yl)-9H-purin-9-yl)-1,3-dimethyl-1,3-dihydro-2H-benzo[d]imidazol-2-one,
5-(6-((3-(azepan-1-yl)propyl)(methyl)amino)-8-(1H-pyrrolo[3,2-b]pyridin-3-yl)-9H-purin-9-yl)-1,3-dimethyl-1H-benzo[d]imidazol-2(3H)-one,
5-(6-((3-(azepan-1-yl)propyl)(methyl)amino)-8-(1-ethyl-1H-indol-4-yl)-9H-purin-9-yl)-1,3-dimethyl-1,3-dihydro-2H-benzo[d]imidazol-2-one,
5-(8-(2,5-dimethyl-1-(4-methylpyridin-2-yl)-1H-pyrrol-3-yl)-6-(methyl(2-(methyl(phenyl)amino)ethyl)amino)-9H-purin-9-yl)-1,3-dimethyl-1,3-dihydro-2H-benzo[d]imidazol-2-one,
5-(6-((1-benzylpyrrolidin-3-yl)(methyl)amino)-8-(thiazol-5-yl)-9H-purin-9-yl)-1,3-dimethyl-1,3-dihydro-2H-benzo[d]imidazol-2-one,
1,3-dimethyl-5-(6-(methyl(2-(thiophen-3-yl)ethyl)amino)-8-(6-methyl-1H-indol-3-yl)-9H-purin-9-yl)-1,3-dihydro-2H-benzo[d]imidazol-2-one,
2-(3,5-dimethylisoxazol-4-yl)-N,1',3'-trimethyl-2'-oxo-2',3'-dihydro-1'H-[1,5'-bibenzo[d]imidazole]-6-sulfonamide,
N,1',3'-trimethyl-2'-oxo-2-(1H-pyrazol-4-yl)-2',3'-dihydro-1'H-[1,5'-bibenzo[d]imidazole]-6-sulfonamide,
5-(8-(1-isopropyl-1H-pyrrol-3-yl)-6-((2-(6-methoxy-1H-indol-3-yl)ethyl)(methyl)amino)-9H-purin-9-yl)-1,3-dimethyl-1,3-dihydro-2H-benzo[d]imidazol-2-one,
5-(6-((3-(1H-pyrrol-1-yl)benzyl)(methyl)amino)-8-(1H-benzo[d]imidazol-4-yl)-9H-purin-9-yl)-1,3-dimethyl-1,3-dihydro-2H-benzo[d]imidazol-2-one,
5-(6-(((1-cyclohexylpyrrolidin-3-yl)methyl)(methyl)amino)-8-(6-methyl-1H-indol-3-yl)-9H-purin-9-yl)-1,3-dimethyl-1,3-dihydro-2H-benzo[d]imidazol-2-one,
5-(6-(((1-cyclohexylpyrrolidin-3-yl)methyl)(methyl)amino)-8-(7-ethyl-1H-indol-3-yl)-9H-purin-9-yl)-1,3-dimethyl-1,3-dihydro-2H-benzo[d]imidazol-2-one,
5-(8-(1H-indol-5-yl)-6-(methyl(2-(m-tolyloxy)ethyl)amino)-9H-purin-9-yl)-1,3-dimethyl-1,3-dihydro-2H-benzo[d]imidazol-2-one,
5-(8-(6-chloro-1H-indol-3-yl)-6-(methyl(2-(m-tolyloxy)ethyl)amino)-9H-purin-9-yl)-1,3-dimethyl-1,3-dihydro-2H-benzo[d]imidazol-2-one,
5-(8-(6bromo-1H-indol-3-yl)-6-((3-(4-chloro-1H-pyrazol-1-yl)propyl)(methyl)amino)-9H-purin-9-yl)-1,3-dimethyl-1H-benzo[d]imidazol-2(3H)-one,
5-(6-((2-(1-cyclopentylpiperidin-4-yl)ethyl)(methyl)amino)-8-(6-methoxy-1H-indol-3-yl)-9H-purin-9-yl)-1,3-dimethyl-1,3-dihydro-2H-benzo[d]imidazol-2-one,
N,1',3'-trimethyl-2-(1-methyl-1H-imidazol-5-yl)-2'-oxo-2',3'-dihydro-1'H-[1,5'-bibenzo[d]imidazole]-6-sulfonamide,
5-(6-(dimethylamino)-8-(6-methyl-1H-indol-3-yl)-9H-purin-9-yl)-1,3-dimethyl-1,3-dihydro-2H-benzo[d]imidazol-2-one,
3-(1,3-dimethyl-2-oxo-benzimidazol-5-yl)-N-methyl-2-(6-methyl-1H-indol-3-yl)benzimidazole-5-sulfonamide,
2-[3-(3,4-difluorophenyl)imidazol-4-yl]-3-(1,3-dimethyl-2-oxo-benzimidazol-5-yl)-N-methyl-benzimidazole-5-sulfonamide,
2-(6-bromo-1H-indol-3-yl)-3-(1,3-dimethyl-2-oxo-benzimidazol-5-yl)-N-methylbenzimidazole-5-sulfonamide,
3-(1,3-dimethyl-2-oxo-benzimidazol-5-yl)-N-methyl-2-(6-oxo-2-piperidyl)benzimidazole-5-sulfonamide,
(S)-2-(1-(3,4-difluorophenyl)-6-oxopiperidin-2-yl)-N,1',3'-trimethyl-2'-oxo-2',3'-dihydro-1'H-[1,5'-bibenzo[d]imidazole]-6-sulfonamide,
2-(5-fluoro-1-phenyl-1H-pyrazol-4-yl)-N,1',3'-trimethyl-2'-oxo-2',3'-dihydro-1'H-[1,5'-bibenzo[d]imidazole]-6-sulfonamide,
3-(1,3-dimethyl-2-oxo-benzimidazol-5-yl)-2-(4-isoquinolyl)-N-methyl-benzimidazole-5-sulfonamide,
tert-butyl 4-[3-[1-(1,3-dimethyl-2-oxo-benzimidazol-5-yl)-6-(methylsulfamoyl)benzimidazol-2-yl]-1H-indol-6-yl]-3,6-dihydro-2H-pyridine-1-carboxylate,
3-(1,3-dimethyl-2-oxo-benzimidazol-5-yl)-N-methyl-2-[6-(1-methylpyrazol-4-yl)-1H-indol-3-yl]benzimidazole-5-sulfonamide,
tert-butyl 4-(3-(1',3'-dimethyl-6-(N-methylsulfamoyl)-2'-oxo-2',3'-dihydro-1'H-[1,5'-bibenzo[d]imidazol]-2-yl)-1H-indol-6-yl)piperidine-1-carboxylate,
N,1',3'-trimethyl-2'-oxo-2-(6-(piperidin-4-yl)-1H-indol-3-yl)-2',3'-dihydro-1'H-[1,5'-bibenzo[d]imidazole]-6-sulfonamide,
3-(1,3-dimethyl-2-oxo-benzimidazol-5-yl)-2-(1H-indazol-5-yl)-N-methylbenzimidazole-5-sulfonamide,
N,1',3'-trimethyl-2-(6-methyl-1H-indol-3-yl)-2'-oxo-2',3'-dihydro-1'H-[1,5'-bibenzo[d]imidazole]-6-carboxamide,
3-(1,3-dimethyl-2-oxo-2,3-dihydro-1H-benzo[d]imidazol-5-yl)-2-(3-methoxynaphthalen-2-yl)-N-methyl-3H-imidazo[4,5-b]pyridine-5-carboxamide,
5-[6-(difluoromethyl)-2-(6-methyl-1H-indol-3-yl)benzimidazol-1-yl]-1,3-dimethyl-benzimidazol-2-one,
5-(4-chloro-2-(6-methyl-1H-indol-3-yl)-1H-imidazo[4,5-c]pyridin-1-yl)-1,3-dimethyl-1,3-dihydro-2H-benzo[d]imidazol-2-one,
5-[4-(dimethylamino)-2-(6-methyl-1H-indol-3-yl)imidazo[4,5-c]pyridin-1-yl]-1,3-dimethyl-benzimidazol-2-one,
5-[4-(dimethylamino)imidazo[4,5-c]pyridin-1-yl]-1,3-dimethyl-benzimidazol-2-one
1,3-dimethyl-5-[2-(6-methyl-1H-indol-3-yl)-6-(1-methylpyrazol-4-yl)benzimidazol-1-yl]benzimidazol-2-one,
5-[7-(dimethylamino)-2-(6-methyl-1H-indol-3-yl)imidazo[4,5-b]pyridin-3-yl]-1,3-dimethyl-benzimidazol-2-one,
2-(7-bromo-1H-indol-3-yl)-3-(1,3-dimethyl-2-oxo-benzimidazol-5-yl)-N-methylbenzimidazole-5-sulfonamide,
2-(7-chloro-1H-indol-3-yl)-3-(1,3-dimethyl-2-oxo-benzimidazol-5-yl)-N-methylbenzimidazole-5-sulfonamide,
2-(5-chloro-1H-indol-3-yl)-3-(1,3-dimethyl-2-oxo-benzimidazol-5-yl)-N-methylbenzimidazole-5-sulfonamide,
3-(1,3-dimethyl-2-oxo-benzimidazol-5-yl)-N-methyl-2-(5-methyl-1H-indazol-3-yl)benzimidazole-5-sulfonamide,
3-(1,3-dimethyl-2-oxo-benzimidazol-5-yl)-N-methyl-2-(5-methyl-1H-indol-3-yl)benzimidazole-5-sulfonamide,
1,3-dimethyl-5-[2-(6-methyl-1H-indol-3-yl)-6-methylsulfonyl-benzimidazol-1-yl]benzimidazol-2-one,
1,3-dimethyl-5-[2-(6-methyl-1H-indol-3-yl)imidazo[4,5-c]pyridin-1-yl]benzimidazol-2-one,
N-methyl-2-(6-methyl-1H-indol-3-yl)-1-(1,3,3-trimethyl-2-oxoindolin-6-yl)-1H-benzo[d]imidazole-6-sulfonamide,
2-(5-cyano-1H-indol-3-yl)-3-(1,3-dimethyl-2-oxo-benzimidazol-5-yl)-N-methylbenzimidazole-5-sulfonamide,
2-(5-bromo-1H-indol-3-yl)-3-(1,3-dimethyl-2-oxo-benzimidazol-5-yl)-N-methylbenzimidazole-5-sulfonamide,
2-(4-bromo-1H-indol-3-yl)-3-(1,3-dimethyl-2-oxo-benzimidazol-5-yl)-N-methylbenzimidazole-5-sulfonamide,
3-(1,3-dimethyl-2-oxo-benzimidazol-5-yl)-N-methyl-2-[6-(trifluoromethyl)-1H-indol-3-yl]benzimidazole-5-sulfonamide,
2-(7-cyclopropyl-1H-indol-3-yl)-3-(1,3-dimethyl-2-oxo-benzimidazol-5-yl)-N-methylbenzimidazole-5-sulfonamide,
2-(4-cyclopropyl-1H-indol-3-yl)-3-(1,3-dimethyl-2-oxo-benzimidazol-5-yl)-N-methylbenzimidazole-5-sulfonamide,
N,1',3'-trimethyl-2,2'-dioxo-2,2',3,3'-tetrahydro-1'H-[1,5'-bibenzo[d]imidazole]-6-sulfonamide,
N-[4-[1-(1,3-dimethyl-2-oxo-benzimidazol-5-yl)-6-(methylsulfamoyl)benzimidazol-2-yl]-3-methyl-phenyl]acetamide,
3-(1,3-dimethyl-2-oxo-benzimidazol-5-yl)-N-methyl-2-[5-(trifluoromethyl)-1H-indol-3-yl]benzimidazole-5-sulfonamide,
2-(6-bromo-1H-pyrrolo[3,2-c]pyridin-3-yl)-N,1',3'-trimethyl-2'-oxo-2',3'-dihydro-1'H-[1,5'-bibenzo[d]imidazole]-6-sulfonamide,
3-(1,3-dimethyl-2-oxo-benzimidazol-5-yl)-N-methyl-2-(1H-pyrrolo[3,2-c]pyridin-3-yl)benzimidazole-5-sulfonamide,
3-(1,3-dimethyl-2-oxo-benzimidazol-5-yl)-2-(4-formyl-1-naphthyl)-N-methylbenzimidazole-5-sulfonamide,
1,3-dimethyl-5-[2-(6-methyl-1H-indol-3-yl)imidazo[4,5-c]pyridin-3-yl]benzimidazol-2-one,
2-(4-(difluoromethyl)naphthalen-1-yl)-N,1',3'-trimethyl-2'-oxo-2',3'-dihydro-1'H-[1,5'-bibenzo[d]imidazole]-6-sulfonamide,
N-methyl-2-(6-methyl-1H-indol-3-yl)-3-(1,3,3-trimethyl-2-oxo-indolin-5-yl)benzimidazole-5-sulfonamide,
1',3'-dimethyl-6-(N-methylsulfamoyl)-2'-oxo-N-(pyridin-3-yl)-2',3'-dihydro-1'H-[1,5'-bibenzo[d]imidazole]-2-carboxamide,
1-(1,3-dimethyl-2-oxo-benzimidazol-5-yl)-6-(methylsulfamoyl)-N-(4-pyridyl)benzimidazole-2-carboxamide,
3-(1,3-dimethyl-2-oxo-benzimidazol-4-yl)-N-methyl-2-(6-methyl-1H-indol-3-yl)benzimidazole-5-sulfonamide,
3-(1,3-dimethyl-2-oxo-benzimidazol-4-yl)-N-methyl-2-(3-methylimidazol-4-yl)benzimidazole-5-sulfonamide,
N-methyl-2-(6-methyl-1H-indol-3-yl)-3-(2-oxo-3,4-dihydro-1H-quinolin-7-yl)benzimidazole-5-sulfonamide,
3-(1,3-dimethyl-2-oxo-benzimidazol-5-yl)-2-(1H-indazol-3-yl)-N-methylbenzimidazole-5-sulfonamide,
methyl 3-(1,3-dimethyl-2-oxo-benzimidazol-5-yl)-2-(6-methyl-1H-indol-3-yl)benzimidazole-5-carboxylate,
3-(1,3-dimethyl-2-oxo-benzimidazol-5-yl)-2-(6-methyl-1H-indol-3-yl)benzimidazole-5-carboxylic acid,
3-[3-(3,5-dimethylisoxazol-4-yl)phenyl]-N-methyl-2-(6-methyl-1H-indol-3-yl)benzimidazole-5-sulfonamide,
1,1,3-trimethyl-3-[3-[2-(6-methyl-1H-indol-3-yl)-6-(methylsulfamoyl)benzimidazol-1-yl]phenyl]urea,
N-methyl-5-[2-(6-methyl-1H-indol-3-yl)-6-(methylsulfamoyl)benzimidazol-1-yl]-1H-indole-3-carboxamide,
3-(1,3-dimethyl-2-oxo-benzimidazol-5-yl)-N-methyl-2-(2-pyridyl)benzimidazole-5-sulfonamide,
3-(1,4-dimethyl-2,3-dioxo-quinoxalin-5-yl)-N-methyl-2-(6-methyl-1H-indol-3-yl)benzimidazole-5-sulfonamide,
N-[3-[2-(6-methyl-1H-indol-3-yl)-6-(methylsulfamoyl)benzimidazol-1-yl]-5-tetrahydropyran-4-yl-phenyl]acetamide,
3-[3-(3,5-dimethylisoxazol-4-yl)phenyl]-N-methyl-2-(3-methylimidazol-4-yl)benzimidazole-5-sulfonamide,
N-[3-[2-(6-methyl-1H-indol-3-yl)-6-(methylsulfamoyl)benzimidazol-1-yl]phenyl]acetamide,
N-methyl-5-[2-(6-methyl-1H-indol-3-yl)-6-(methylsulfamoyl)benzimidazol-1-yl]-7-tetrahydropyran-4-yl-1H-indole-3-carboxamide,
3-(3-hydroxy-5-tetrahydropyran-4-yl-phenyl)-N-methyl-2-(6-methyl-1H-indol-3-yl)benzimidazole-5-sulfonamide,
1-(3-(3,5-dimethylisoxazol-4-yl)-5-(tetrahydro-2H-pyran-4-yl)phenyl)-N-methyl-2-(6-methyl-1H-indol-3-yl)-1H-benzo[d]imidazole-6-sulfonamide,
methyl 3-(3-acetamido-5-tetrahydropyran-4-yl-phenyl)-2-(6-methyl-1H-indol-3-yl)benzimidazole-5-carboxylate,
3-(3-acetamido-5-tetrahydropyran-4-yl-phenyl)-2-(6-methyl-1H-indol-3-yl)benzimidazole-5-carboxylic acid,
N-methyl-2-(3-methylimidazol-4-yl)-3-(2-oxo-1,3-dihydrobenzimidazol-5-yl)benzimidazole-5-sulfonamide,
3-(1,3-dimethyl-2-oxo-benzimidazol-5-yl)-N-methyl-2-(1-methylimidazol-4-yl)benzimidazole-5-sulfonamide,
1',3'-dimethyl-2-phenyl-1',3'-dihydro-2'H-[1,5'-bibenzo[d]imidazol]-2'-one,
2-(3-bromophenyl)-1',3'-dimethyl-1',3'-dihydro-2'H-[1,5'-bibenzo[d]imidazol]-2'-one,
2-(4-bromophenyl)-1',3'-dimethyl-1',3'-dihydro-2'H-[1,5'-bibenzo[d]imidazol]-2'-one,
2-(1H-imidazol-5-yl)-1',3'-dimethyl-1',3'-dihydro-2'H-[1,5'-bibenzo[d]imidazol]-2'-one,
2-(1H-indol-3-yl)-1',3'-dimethyl-1',3'-dihydro-2'H-[1,5'-bibenzo[d]imidazol]-2'-one,
1',3'-dimethyl-2-(6-methyl-1H-indol-3-yl)-1',3'-dihydro-2'H-[1,5'-bibenzo[d]imidazol]-2'-one,
2-(1H-indol-2-yl)-1',3'-dimethyl-1',3'-dihydro-2'H-[1,5'-bibenzo[d]imidazol]-2'-one,
2-(1,6-dimethyl-1*H*-indol-3-yl)-1',3'-dimethyl-1',3'-dihydro-2'*H*-[1,5'-bibenzo[*d*]imidazol]-2'-one,
1',3'-dimethyl-2-(1-methyl-1H-imidazol-5-yl)-1',3'-dihydro-2'H-[1,5'-bibenzo[d]imidazol]-2'-one,
2-(1H-imidazol-5-yl)-N,1',3'-trimethyl-2'-oxo-2',3'-dihydro-1'H-[1,5'-bibenzo[d]imidazole]-6-sulfonamide,
2-(1H-indol-3-yl)-N,1',3'-trimethyl-2'-oxo-2',3'-dihydro-1'H-[1,5'-bibenzo[d]imidazole]-6-sulfonamide,
2-(1,6-dimethyl-1*H*-indol-3-yl)-*N*,1',3'-trimethyl-2'-oxo-2',3'-dihydro-1'*H*-[1,5'-bibenzo[*d*]imidazole]-6-sulfonamide,
2-(1H-indol-2-yl)-N,1',3'-trimethyl-2'-oxo-2',3'-dihydro-1'H-[1,5'-bibenzo[d]imidazole]-6-sulfonamide,
5-(6-chloro-8-(1H-indol-3-yl)-9H-purin-9-yl)-1,3-dimethyl-1,3-dihydro-2H-benzo[d]imidazol-2-one,
5-(6-chloro-8-(1H-indol-2-yl)-9H-purin-9-yl)-1,3-dimethyl-1,3-dihydro-2H-benzo[d]imidazol-2-one,
5-(6-chloro-8-(6-methyl-1H-indol-3-yl)-9H-purin-9-yl)-1,3-dimethyl-1,3-dihydro-2H-benzo[d]imidazol-2-one,
5-(6-chloro-8-(1H-imidazol-5-yl)-9H-purin-9-yl)-1,3-dimethyl-1,3-dihydro-2H-benzo[d]imidazol-2-one,
N,1',3'-trimethyl-2-(3-(1-methyl-1H-pyrazol-4-yl)phenyl)-2'-oxo-2',3'-dihydro-1'H-[1,5'-bibenzo[d]imidazole]-6-sulfonamide,
N,1',3'-trimethyl-2-(4-(1-methyl-1H-pyrazol-4-yl)phenyl)-2'-oxo-2',3'-dihydro-1'H-[1,5'-bibenzo[d]imidazole]-6-sulfonamide,
2-(1-(cyclopropylmethyl)-6-methyl-1*H*-indol-3-yl)-*N*,1',3'-trimethyl-2'-oxo-2',3'-dihydro-1'*H*-[1,5'-bibenzo[*d*]imidazole]-6-sulfonamide,
*N*,1',3'-trimethyl-2-(6-methyl-1-(2-(pyrrolidin-1-yl)ethyl)-1*H*-indol-3-yl)-2'-oxo-2',3'-dihydro-1'*H*-[1,5'-bibenzo[*d*]imidazole]-6-sulfonamide,
*N*,1',3'-trimethyl-2-(6-methyl-1-(2-morpholinoethyl)-1*H*-indol-3-yl)-2'-oxo-2',3'-dihydro-1'*H*-[1,5'-bibenzo[*d*]imidazole]-6-sulfonamide,
2-(benzofuran-3-yl)-*N*,1',3'-trimethyl-2'-oxo-2',3'-dihydro-1'*H*-[1,5'-bibenzo[*d*]imidazole]-6-sulfonamide,
2-(benzo[*b*]thiophen-3-yl)-*N*,1',3'-trimethyl-2'-oxo-2',3'-dihydro-1'*H*-[1,5'-bibenzo[*d*]imidazole]-6-sulfonamide,
*N*,1',3'-trimethyl-2-(2-methyl-1*H*-indol-3-yl)-2'-oxo-2',3'-dihydro-1'*H*-[1,5'-bibenzo[*d*]imidazole]-6-sulfonamide,
2-(4-fluoro-1*H*-indol-3-yl)-*N*,1',3'-trimethyl-2'-oxo-2',3'-dihydro-1'*H*-[1,5'-bibenzo[*d*]imidazole]-6-sulfonamide,
2-(5-fluoro-1*H*-indol-3-yl)-*N*,1',3'-trimethyl-2'-oxo-2',3'-dihydro-1'*H*-[1,5'-bibenzo[*d*]imidazole]-6-sulfonamide,
2-(6-fluoro-1*H*-indol-3-yl)-*N*,1',3'-trimethyl-2'-oxo-2',3'-dihydro-1'*H*-[1,5'-bibenzo[*d*]imidazole]-6-sulfonamide,
2-(5,6-difluoro-1*H*-indol-3-yl)-*N*,1',3'-trimethyl-2'-oxo-2',3'-dihydro-1'*H*-[1,5'-bibenzo[*d*]imidazole]-6-sulfonamide,
*N*,1',3'-trimethyl-2'-oxo-2-(4,5,6,7-tetrafluoro-1*H*-indol-3-yl)-2',3'-dihydro-1'*H*-[1,5'-bibenzo[*d*]imidazole]-6-sulfonamide,
5-(6-(dimethylamino)-8-(1-methyl-1*H*-imidazol-5-yl)-9*H*-purin-9-yl)-1,3-dimethyl-1,3-dihydro-2*H*-benzo[*d*]imidazol-2-one,
5-(6-(dimethylamino)-8-(4-fluoro-1*H*-indol-3-yl)-9*H*-purin-9-yl)-1,3-dimethyl-1,3-dihydro-2*H*-benzo[*d*]imidazol-2-one,
5-(6-(dimethylamino)-8-(5-fluoro-1*H*-indol-3-yl)-9*H*-purin-9-yl)-1,3-dimethyl-1,3-dihydro-2*H*-benzo[*d*]imidazol-2-one,
5-(6-(dimethylamino)-8-(6-fluoro-1*H*-indol-3-yl)-9*H*-purin-9-yl)-1,3-dimethyl-1,3-dihydro-2*H*-benzo[*d*]imidazol-2-one,
5-(8-(5,6-difluoro-1*H*-indol-3-yl)-6-(dimethylamino)-9*H*-purin-9-yl)-1,3-dimethyl-1,3-dihydro-2*H*-benzo[*d*]imidazol-2-one,
1',3'-diethyl-2-(4-fluoro-1*H*-indol-3-yl)-*N*-methyl-2'-oxo-2',3'-dihydro-1'*H*-[1,5'-bibenzo[*d*]imidazole]-6-sulfonamide,
1',3'-dicyclopropyl-2-(4-fluoro-1*H*-indol-3-yl)-*N*-methyl-2'-oxo-2',3'-dihydro-1'*H*-[1,5'-bibenzo[*d*]imidazole]-6-sulfonamide,
1',3'-dimethyl-2-(6-methyl-1*H*-indol-3-yl)-*N*-(2-morpholinoethyl)-2'-oxo-2',3'-dihydro-1'*H*-[1,5'-bibenzo[*d*]imidazole]-6-sulfonamide,
1',3'-dimethyl-2-(6-methyl-1*H*-indol-3-yl)-2'-oxo-*N*-(2-(pyrrolidin-1-yl)ethyl)-2',3'-dihydro-1'*H*-[1,5'-bibenzo[*d*]imidazole]-6-sulfonamide,
*N*-(cyclopropylmethyl)-1',3'-dimethyl-2-(6-methyl-1*H*-indol-3-yl)-2'-oxo-2',3'-dihydro-1'*H*-[1,5'-bibenzo[*d*]imidazole]-6-sulfonamide,
1',3'-dimethyl-2-(6-methyl-1*H*-indol-3-yl)-6-(morpholinosulfonyl)-1',3'-dihydro-2'*H-*[1,5'-bibenzo[*d*]imidazol]-2'-one,
1',3'-dimethyl-2-(6-methyl-1*H*-indol-3-yl)-2'-oxo-2',3'-dihydro-1'*H*-[1,5'-bibenzo[*d*]imidazole]-6-sulfonamide,
*N*-ethyl-1',3'-dimethyl-2-(6-methyl-1*H*-indol-3-yl)-2'-oxo-2',3'-dihydro-1'*H*-[1,5'-bibenzo[*d*]imidazole]-6-sulfonamide,
*N*-benzyl-1',3'-dimethyl-2-(6-methyl-1*H*-indol-3-yl)-2'-oxo-2',3'-dihydro-1'*H*-[1,5'-bibenzo[*d*]imidazole]-6-sulfonamide,
*N*-isopropyl-1',3'-dimethyl-2-(6-methyl-1*H*-indol-3-yl)-2'-oxo-2',3'-dihydro-1'*H*-[1,5'-bibenzo[*d*]imidazole]-6-sulfonamide,
2-(5-fluoro-1*H*-indol-3-yl)-1',3'-dimethyl-*N*-(2-morpholinoethyl)-2'-oxo-2',3'-dihydro-1'*H*-[1,5'-bibenzo[*d*]imidazole]-6-sulfonamide,
2-(4-fluoro-1*H*-indol-3-yl)-1',3'-dimethyl-2'-oxo-2',3'-dihydro-1'*H*-[1,5'-bibenzo[*d*]imidazole]-6-sulfonamide,
*N*,1',3'-trimethyl-2'-oxo-2-(1*H*-pyrrol-3-yl)-2',3'-dihydro-1'*H*-[1,5'-bibenzo[*d*]imidazole]-6-sulfonamide,
2-(1-benzyl-1*H*-pyrrol-3-yl)-*N*,1',3'-trimethyl-2'-oxo-2',3'-dihydro-1'*H*-[1,5'-bibenzo[*d*]imidazole]-6-sulfonamide,
*N*,1',3'-trimethyl-2'-oxo-2-(1*H*-pyrrol-2-yl)-2',3'-dihydro-1'*H*-[1,5'-bibenzo[*d*]imidazole]-6-sulfonamide,
2-(1-(cyclopropylmethyl)-1*H*-pyrrol-2-yl)-*N*,1',3'-trimethyl-2'-oxo-2',3'-dihydro-1'*H-*[1,5'-bibenzo[*d*]imidazole]-6-sulfonamide,
2-(1-(cyclopropylmethyl)-1*H*-pyrrol-3-yl)-*N*,1',3'-trimethyl-2'-oxo-2',3'-dihydro-1'*H-*[1,5'-bibenzo[*d*]imidazole]-6-sulfonamide,
*N*,1',3'-trimethyl-2-(1-methyl-1*H*-pyrrol-3-yl)-2'-oxo-2',3'-dihydro-1'*H*-[1,5'-bibenzo[*d*]imidazole]-6-sulfonamide,
2-(1-benzyl-1*H*-pyrrol-2-yl)-*N*,1',3'-trimethyl-2'-oxo-2',3'-dihydro-1'*H*-[1,5'-bibenzo[*d*]imidazole]-6-sulfonamide,
2-(1-(3-fluorobenzyl)-1*H*-pyrrol-2-yl)-*N*,1',3'-trimethyl-2'-oxo-2',3'-dihydro-1'*H*-[1,5'-bibenzo[*d*]imidazole]-6-sulfonamide,
2-(1-(4-fluorobenzyl)-1*H*-pyrrol-2-yl)-*N*,1',3'-trimethyl-2'-oxo-2',3'-dihydro-1'*H*-[1,5'-bibenzo[*d*]imidazole]-6-sulfonamide,
2-(1-(2,4-difluorobenzyl)-1*H*-pyrrol-2-yl)-*N*,1',3'-trimethyl-2'-oxo-2',3'-dihydro-1'*H-*[1,5'-bibenzo[*d*]imidazole]-6-sulfonamide,
2-(1-(2,6-difluorobenzyl)-1*H*-pyrrol-2-yl)-*N*,1',3'-trimethyl-2'-oxo-2',3'-dihydro-1'*H-*[1,5'-bibenzo[*d*]imidazole]-6-sulfonamide,
2-(1-(4-methoxybenzyl)-1*H*-pyrrol-2-yl)-*N*,1',3'-trimethyl-2'-oxo-2',3'-dihydro-1'*H-*[1,5'-bibenzo[*d*]imidazole]-6-sulfonamide,
2-(1-(2-bromo-5-methoxybenzyl)-1*H*-pyrrol-2-yl)-*N*,1',3'-trimethyl-2'-oxo-2',3'-dihydro-1'*H*-[1,5'-bibenzo[*d*]imidazole]-6-sulfonamide.

13. The compound according to claim 1, being used as a CBP/p300 inhibitor.

14. An isomer of the compound according to claim 1, a pharmaceutically acceptable salt thereof, a hydrate thereof or a solvate thereof.

15. A pharmaceutical composition for preventing or treating cancer, inflammatory disorders or autoimmune diseases, the composition comprising at least one among the compound according to claim 1, an isomer thereof, a pharmaceutically acceptable salt thereof, a hydrate thereof and a solvate thereof, as an active ingredient.

16. The pharmaceutical composition according to claim 15, wherein the cancer is cancer dependent on oncogene MYC, tumor harboring a loss of function mutation of CBP or p300, or cancer with activation of CBP and/or p300 function.

17. The pharmaceutical composition according to claim 15, wherein the cancer is one or more selected from the group consisting of blood cancer, prostate cancer, breast cancer, lung cancer, pancreatic cancer, colorectal cancer and melanoma..

18. The pharmaceutical composition according to claim 15, wherein the cancer is one or more selected from the group consisting of acoustic neuroma, acute leukemia, acute lymphoblastic leukemia, acute myeloid leukemia, acute T-cell leukemia, basal cell carcinoma, cholangiocarcinoma, bladder cancer, brain cancer, breast cancer, bronchial carcinoma, cervical cancer, chondrosarcoma, chordoma, choriocarcinoma, chronic leukemia, chronic lymphocytic leukemia, chronic myeloid leukemia, chronic myeloid leukemia, colon cancer, colon cancer, craniopharyngioma, cystadenocarcinoma, diffuse large B-cell lymphoma, dysproliferative changes, embryonal carcinoma, endometrial cancer, endotheliosarcoma, ependymoma, epithelial carcinoma, erythroleukemia, esophageal cancer, estrogen receptor-positive breast cancer, essential thrombocythemia, Ewing's tumor, fibrosarcoma, follicular lymphoma, germ cell testicular cancer, glioma, glioblastoma, pedunculated tumor, heavy chain disease head and neck cancer, hemangioblastoma, hepatoma, hepatocellular carcinoma, hormone-insensitive prostate cancer, leiomyosarcoma, leukemia, liposarcoma, lung cancer, lymphoendotheliosarcoma, lymphangiosarcoma, lymphocytic leukemia, lymphoma, lymphoid malignancy of T-cell or B-cell origin, medullary carcinoma, medulloblastoma, melanoma, meningioma, mesothelioma, multiple myeloma, myeloid leukemia, myeloma, myxosarcoma, neuroblastoma, NUT midline carcinoma (NMC), non-small cell lung cancer, oligodendroglioma, oral cancer, osteosarcoma, ovarian cancer, pancreatic cancer, papillary adenocarcinoma, papillary carcinoma, pinealoma, polycythemia vera, prostate cancer, rectal cancer, renal cell carcinoma, retinoblastoma, rhabdomyosarcoma, sarcoma, sebaceous carcinoma, seminoma, skin cancer, small cell lung carcinoma, solid tumors (carcinomas and sarcomas), small cell lung cancer, stomach cancer, squamous cell carcinoma, synovioma, sweat gland carcinoma, thyroid cancer, Waldenstrom macroglobulinemia, testicular tumor, uterine cancer and Wilms tumor.

19. The pharmaceutical composition according to claim 15, wherein the inflammatory disorder or autoimmune disease is one or more selected from the group consisting of Addison's disease, acute gout, ankylosing spondylitis, asthma, atherosclerosis, Behcet's disease, bullous skin disease, chronic obstructive pulmonary disease, Crohn's disease, dermatitis, eczema, giant cell arteritis, fibrosis, glomerulonephritis, hepatic vascular occlusion, hepatitis, hypophysitis, immunodeficiency syndrome, inflammatory bowel disease, Kawasaki disease, lupus nephritis, multiple sclerosis, myocarditis, myositis, nephritis, organ transplant rejection, osteoarthritis, pancreatitis, pericarditis, polyarteritis nodosa pneumonia, primary biliary cirrhosis, psoriasis, psoriatic arthritis, rheumatoid arthritis, scleritis, sclerosing cholangitis, sepsis, systemic lupus erythematosus, Takayasu's arteritis, toxic shock, thyroiditis, type 1 diabetes, ulcerative colitis, uveitis, vitiligo, vasculitis, and Wegener's granulomatosis.
